# FASCICULE DE BREVET EUROPEEN

(11) **EP 3 463 294 B1**
(45) Date de publication et mention de la délivrance du brevet: **05.08.2020**
(21) Numéro de dépôt: 17728541.8
(22) Date de dépôt: 07.06.2017
(51) Int. Cl.: A61K 9/08, A61K 47/34, A61K 38/26

(54) **COMPOSITIONS SOUS FORME D'UNE SOLUTION AQUEUSE INJECTABLE COMPRENANT DU GLUCAGON HUMAIN ET UN CO-POLYAMINOACIDE STATISTIQUE**
ZUSAMMENSETZUNGEN IN FORM EINER INJIZIERBAREN WÄSSRIGEN LÖSUNG MIT MENSCHLICHEM GLUCAGON UND EINER STATISTISCHEN COPOLYAMINOSÄURE
COMPOSITIONS IN THE FORM OF AN INJECTABLE AQUEOUS SOLUTION, COMPRISING HUMAN GLUCAGON AND A STATISTICAL COPOLYAMINO ACID

(30) Priorité: 07.06.2016 FR 1655221; 10.01.2017 FR 1750221
(43) Date de publication de la demande: 10.04.2019
(73) Titulaire: Adocia, 69003 Lyon (FR)
(72) Inventeur: GEISSLER, Alexandre, 69007 Lyon (FR); LAAGE, Ségolène, 69003 Lyon (FR); SOULA, Olivier, 69330 Meyzieu (FR); DURACHER, David, 69004 Lyon (FR); MEIFFREN, Grégory, 69330 Meyzieu (FR)
(74) Mandataire: Tripoz, Inès
(86) Numéro de dépôt international: PCT/EP2017/063887
(87) Numéro de publication internationale: WO 2017/211917

(56) Documents cités:
- GB-A- 1 202 607
- US-A1- 2013 178 415

## Description

Le glucagon humain est une hormone hyperglycémiante d'action brève qui permet d'augmenter la glycémie, corrigeant ainsi un niveau hypoglycémique pouvant résulter d'un excès d'insuline. Il permet la libération de glucose par stimulation de la glycogénolyse hépatique, et possède des propriétés antagonistes de l'insuline (hypoglycémiante). Le glucagon humain est normalement sécrété par les cellules alpha des ilots de Langerhans dans le pancréas lorsqu'une hypoglycémie est détectée.

Le glucagon humain est utilisé à des fins thérapeutiques, comme le traitement d'urgence d'hypoglycémies sévères, encore appelé « rescue », mais également dans un cadre diagnostique lors de la réalisation d'examens médicaux, par exemple pour inhiber la motilité gastro-intestinale. D'autres applications sont également envisagées pour le glucagon humain, en particulier son utilisation dans un système de régulation bi-hormonal de la glycémie aussi appelé pancréas artificiel et dans l'hyperinsulinisme congénital qui est une maladie rare caractérisée par des niveaux très élevés d'insuline.

L'utilisation clinique du glucagon humain a été limitée à cause de certaines de ses propriétés peu favorables pour développer un produit pharmaceutique stable à visée thérapeutique. En effet, le glucagon humain présente une très faible solubilité à pH physiologique, une forte instabilité physique, à cause de sa propension à former des fibrilles sur une large gamme de pH. C'est pour cette raison que les seuls produits commerciaux à base de glucagon humain (Glucagen®, NOVO NORDISK et Glucagon pour injection, ELI LILLY) sont des formes lyophilisées à reconstituer extemporanément.

Les travaux d'Onoue et al. (Pharm. Res. 2004, 21(7), 1274-83) ont montré le caractère potentiellement dangereux de ces fibrilles : le glucagon humain fibrillé étant cytotoxique dans des cellules de mammifères en culture.

Outre son instabilité physique, le glucagon humain subit divers types de dégradation chimique. En solution aqueuse, il se dégrade rapidement pour former plusieurs produits de dégradation. Au moins 16 produits de dégradation du glucagon humain ont été identifiés par Kirsh et al. (International Journal of Pharmaceutics, 2000, 203, 115-125). La dégradation chimique de ce glucagon humain est donc rapide et complexe.

La mauvaise stabilité chimique et physique du glucagon humain en solution a conduit des sociétés pharmaceutiques comme NOVO NORDISK, ELI LILLY et plus récemment FRESENIUS KABI à commercialiser ce glucagon humain sous la forme d'un lyophilisat à reconstituer à pH acide (pH<3) juste avant injection. Le glucagon humain sous forme de lyophilisat est plus stable, et la préparation de la formulation à pH acide juste avant utilisation permet d'obtenir une solution limpide. Cependant, une fois le produit reconstitué celui-ci doit être utilisé rapidement car il subit une dégradation chimique et physique extrêmement rapide dans le tampon acide de reconstitution, avec apparition de fibrilles de glucagon humain dans les 24 heures suivant la reconstitution, et/ou une gélification de la composition. Cette présentation du produit est cependant insatisfaisante car elle oblige à une utilisation très rapide de la formulation. Cette instabilité rend non seulement l'utilisation en pompe impossible, mais elle présente également l'inconvénient de conduire à des pertes de produit importantes dans l'utilisation diagnostique. En effet, une composition de ce type n'étant plus utilisable quelques heures après préparation cela cause du gaspillage.

Enfin, même dans l'application de traitement d'urgence des réactions hypoglycémiques sévères, pouvant survenir lors d'une insulinothérapie chez les patients diabétiques, la formulation à reconstituer n'est pas non plus idéale, car elle implique une préparation longue et compliquée, par exemple la notice de GlucaGen® décrit un procédé en 5 étapes pour procéder à l'injection de la dose préconisée. D'ailleurs, une étude de la société LOCEMIA démontre que très peu de personnes (environ 10% des participants) devant réaliser la reconstitution dans l'urgence étaient capables de délivrer la dose adéquate. Enfin, le pH acide des solutions de glucagon humain peut générer des douleurs à l'injection chez le patient.

Il y a donc un besoin d'une solution de glucagon humain prête à l'emploi. Aujourd'hui, les solutions les plus avancées d'un point de vue clinique pour permettre la délivrance de glucagon humain contournent le problème de stabilité du glucagon humain en solution aqueuse de différentes manières.

La société LOCEMIA a mis au point un spray de glucagon humain lyophilisé, actuellement testé en étude clinique de phase 3, qui est destiné à être administré par voie intranasale. Ce spray est adapté à une utilisation dite « rescue », c'est-à-dire dans le cas d'une hypoglycémie sévère, car il est prêt à l'emploi et donc d'utilisation facile, contrairement aux solutions à reconstituer. Cependant, ce produit n'est pas adapté à une utilisation en pompe ou à une utilisation nécessitant un contrôle précis de la quantité de glucagon humain délivrée.

Pour sa part, XERIS a mis au point une formulation liquide du glucagon humain basée sur un solvant aprotique polaire, comme le DMSO, actuellement testée en études cliniques. Cependant, si l'injection de solution de solvants organiques pour une utilisation de type « rescue » est envisageable, il est largement préférable d'avoir une solution aqueuse de glucagon humain pour une utilisation chronique. Des compositions comprenant une association avec d'autres peptides sont envisagées notamment l'amyline ou un GLP-1 RA (Glucagon like peptide-1 receptor agonist).

Enfin, face aux difficultés de formulation du glucagon humain, des analogues du glucagon humain sont en cours de développement par des grandes sociétés pharmaceutiques, comme NOVO NORDISK, SANOFI OU ELI LILLY, afin d'obtenir des formulations ayant une stabilité compatible avec une utilisation pharmaceutique. Cependant, ces peptides dont la séquence primaire a été modifiée par rapport au peptide d'origine humaine peuvent présenter un risque de sécurité pour les patients.

Il y a donc un intérêt majeur pour une solution permettant d'améliorer la solubilisation et la stabilité, à la fois chimique et physique, du glucagon humain en solution aqueuse à un pH proche du pH physiologique, c'est-à-dire compris entre 6,0 et 8,0. Ceci pourrait permettre d'obtenir un produit pharmaceutique plus facilement utilisable par le patient en cas d'urgence, mais également d'ouvrir le champ à de nouvelles applications thérapeutiques du glucagon humain, comme par exemple son utilisation dans un pancréas artificiel bihormonal.

L'art antérieur propose des solutions pour tenter de résoudre ce problème.

Certains documents proposent de se placer à pH basique. Par exemple US2015291680 enseigne la solubilisation de glucagon humain à 1 mg/ml en se plaçant à un pH compris entre 8,8 et 9,4 et en utilisant de l'acide férulique ou le tétrahydrocurcumin. Cependant, outre le fait de se placer à pH basique, cette solution présente l'inconvénient de conduire à une stabilité du glucagon humain assez limitée dans le temps. L'article de Jackson et al (Curr. Diab. Rep., 2012, 12, 705-710) propose de formuler le glucagon humain à pH basique (environ 10) afin de limiter la formation de fibrilles. Cependant cette solution n'empêche pas une dégradation chimique rapide du glucagon humain.

La demande WO2014096440 (NOVOZYME) envisage au contraire de se placer à pH légèrement acide (environ 5,5) en présence d'albumine et de polysorbate, afin d'améliorer la stabilité en réduisant la vitesse de fibrillation. Cependant, cette solution présente une amélioration limitée de la stabilité. La plupart des solutions décrites dans l'art antérieur permettant d'obtenir une solution limpide de glucagon humain et de prévenir l'agrégation, la gélification ou la précipitation du glucagon humain impliquent l'utilisation de tensioactifs, de détergents ou d'agents solubilisant connus.

Par exemple, Matilainen et al (J. Pharm. Sci, 2008, 97, 2720-2729 et Eur. J. Pharm. Sci., 2009, 36, 412-420) a décrit l'utilisation de la cyclodextrine afin de limiter la vitesse de formation des fibrilles de glucagon humain. Cependant, l'amélioration apportée paraît insuffisante pour envisager une utilisation en pompe.

Parmi les solutions proposées figurent les tensioactifs hydrophiles :
- GB1202607 (NOVO NORDISK) décrit l'utilisation de détergents anioniques ou cationiques.
- US6384016 (NOVO NORDISK) et US2011097386 (BIODEL) utilisent des lysophospholipides (ou lysolécithines).
- WO2015095389 (AEGIS) décrit des tensioactifs non-ioniques, comme le dodécyl maltoside, pour améliorer la biodisponibilité d'agents thérapeutiques, dans le cas de délivrance par application sur les muqueuses ou l'épiderme, et en particulier dans le cas de délivrance oculaire, nasale, orale ou nasolacrymale. Ce document décrit que la présence d'alkyles glycosides conduit à une amélioration de l'absorption du glucagon humain au niveau oculaire,
- la demande WO2012059764 (ARECOR) décrit des tensioactifs cationiques, et plus précisément des chlorures d'ammonium aromatiques.

Les tensioactifs indiqués dans les documents ci-dessus peuvent être trop toxiques ou irritants pour une utilisation chronique par voie sous cutanée. Par exemple les lysophospholipides (ou lysolécithines) sont connus pour lyser les globules rouges du fait de leur propriétés hémolytiques. Lors d'une injection sous cutanée, cela peut provoquer des dommages locaux aux tissus et des douleurs au site d'injection. Dans le cas d'une injection en continue par une pompe, cela peut conduire à des douleurs et/ou à de l'irritation au niveau du site d'insertion de l'aiguille. La demande internationale WO2011138802 (Sun Pharma) décrit une solution prête à l'emploi de glucagon humain en solution aqueuse micellaire à un pH compris entre 5 et 7,5 en présence d'un lipide pegylé (pegylated distearoyl-phosphotidylethanolamine). Cependant, Garay et al. (Expert Opin Drug Deliv (2012) 9, 1319-1323) enseignent que le Poly Ethylène Glycol est à la fois immunogénique et antigénique. Ceci peut être préjudiciable aux patients présentant des anticorps anti-PEG. D'ailleurs, Ganson et al. (J. Allergy Clin. Immunol. (2015) doi:10.1016/j.jaci.2015.10.034) décrivent qu'une étude clinique portant sur de la pegnivacogin couplée à du méthoxypolyéthylène glycol (mPEG) de 40 kDa a conduit à des réponses inflammatoires dès la première dose de pegnivacogin sur 3 des 640 patients. Parmi ces trois patients deux remplissaient les critères d'anaphylaxie et un présentait une réaction dermale isolée, chaque évènement a été estimé sérieux, et l'un a même été estimé mettre la vie du patient en danger. Ces événements adverses ont causé l'arrêt de l'essai clinique et posent le problème des effets indésirables de composés pegylés.

Le document WO2013101749 (LATITUDE) décrit des nano-émulsions de glucagon humain. Cependant il revendique des performances assez modestes en termes de stabilité chimique, c'est-à-dire que la composition comprend au moins 75% de la concentration initiale après 3-7 jours à 37°C.

En outre, il est à noter, qu'à ce jour, à la connaissance de la demanderesse, aucune formulation pharmaceutique comprenant du glucagon humain sous forme de solution aqueuse n'est testée en étude clinique.

Il subsiste donc un besoin pour une formulation aqueuse liquide à un pH proche du pH physiologique compris entre 6,0 et 8,0 permettant de solubiliser et d'obtenir une bonne stabilité du glucagon humain, tant en termes de stabilité physique que de stabilité chimique. Plus particulièrement il existe un besoin pour une telle formulation qui puisse être utilisée dans une pompe bihormonale (insuline/glucagon humain).

Ce besoin est d'autant plus clair que Tan et al. (Diabetes, 2013, 62, 1131-138) montre que combiner le glucagon humain avec un GLP-1 RA est une proposition attractive de traitement de l'obésité et du diabète. Or, pouvoir formuler le glucagon humain de manière stable en solution aqueuse à un pH proche du pH physiologique compris entre 6,0 et 8,0 permet d'être dans des conditions plus favorables pour pouvoir améliorer la stabilité des GLP-1 RA sensibles aux conditions acides ou basiques.

Les co-polyaminoacides porteur de charges carboxylates et de radicaux hydrophobes Hy selon l'invention présentent une excellente résistance à l'hydrolyse. Ceci peut notamment être mis en «évidence en conditions accélérées, par exemple par des tests d'hydrolyse à pH basique (pH 12).

En outre des tests d'oxydation forcée, par exemple du type oxydation de fenton, montrent que les co-polyaminoacides porteur de charges carboxylates et de radicaux hydrophobes Hy présentent une bonne résistance à l'oxydation.

L'invention concerne ainsi des compositions stables physiquement sous forme d'une solution aqueuse injectable, dont le pH est compris entre 6,0 et 8,0, comprenant au moins :
a) du glucagon humain et
b) un co-polyaminoacide porteur de charges carboxylates et de radicaux hydrophobes Hy, ledit co-polyaminoacide étant constitué d'unités glutamiques ou aspartiques et lesdits radicaux hydrophobes Hy étant de formule I suivante : 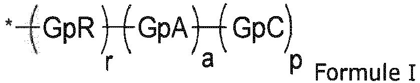 dans laquelle
   - GpR est un radical de formules II: 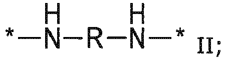
   - GpA est un radical de formules III ou III' : 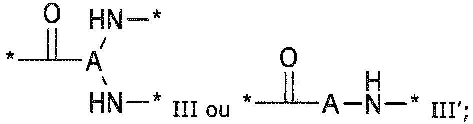
   - GpC est un radical de formule IV : 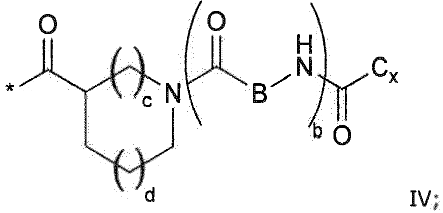
   - les * indiquent les sites de rattachement des différents groupes;
   - a est un entier égal à 0 ou à 1 ;
   - b est un entier égal à 0 ou à 1;
   - p est un entier égal à 1 ou à 2 et
      ∘ si p est égal à 1 alors a est égal à 0 ou à 1 et GpA est un radical de formule III' et,
      ∘ si p est égal à 2 alors a est égal à 1, et GpA est un radical de formule III;
   - c est un entier égal à 0 ou à 1, et si c est égal à 0 alors d est égal à 1 ou à 2;
   - d est un entier égal à 0, à 1 ou à 2;
   - r est un entier égal à 0 ou à 1, et
      ∘ si r est égal à 0 alors le radical hydrophobe de formule I est lié au co-polyaminoacide via une liaison covalente entre un carbonyl du radical hydrophobe et un atome d'azote en position N terminale du co-polyaminoacide, formant ainsi une fonction amide issue de la réaction d'une fonction amine en position N terminale du précurseur du co-polyaminoacide et une fonction acide portée par le précurseur du radical hydrophobe , et ∘ si r est égal à 1 alors le radical hydrophobe de formule I est lié au co-polyaminoacide :
      ▪ via une liaison covalente entre un atome d'azote du radical hydrophobe et un carbonyl du co-polyaminoacide, formant ainsi une fonction amide issue de la réaction d'une fonction amine du précurseur du radical hydrophobe et une fonction acide portée par le précurseur du co-polyaminoacide ou
      ▪ via une liaison covalente entre un carbonyl du radical hydrophobe et un atome d'azote en position N terminal du co-polyaminoacide, formant ainsi une fonction amide issue de la réaction d'une fonction acide du précurseur du radical hydrophobe et une fonction amine en position N terminale portée par le précurseur du co-polyaminoacide;
   - R est un radical choisi dans le groupe constitué par :
      ∘ un radical alkyle divalent, linéaire ou ramifié, comprenant si GpR est un radical de formule II de 2 à 12 atomes de carbone;
      ∘ un radical alkyle divalent, linéaire ou ramifié, comprenant si GpR est un radical de formule II de 2 à 11 atomes de carbone, ledit radical alkyle portant une ou plusieurs fonctions -CONH₂, et
      ∘ un radical éther ou polyéther non substitué comprenant de 4 à 14 atomes de carbone et de 1 à 5 atomes d'oxygène ;
   - A est un radical alkyle linéaire ou ramifié comprenant de 1 à 6 atomes de carbone;
   - B est un radical alkyle linéaire ou ramifié, éventuellement comprenant un noyau aromatique, comprenant de 1 à 9 atomes de carbone;
   - Cₓ est un radical alkyl monovalent linéaire ou ramifié, dans lequel x indique le nombre d'atomes de carbone et :
      ∘ si p est égal à 1, x est compris entre 11 et 25 (11 ≤ x ≤ 25) :
      ∘ si p est égal à 2, x est compris entre 9 et 15 (9 ≤ x ≤ 15),
   - le ratio i entre le nombre de radicaux hydrophobes et le nombre d'unités glutamiques ou aspartiques étant compris entre entre 0 < i ≤ 0,5 ;
   - lorsque plusieurs radicaux hydrophobes sont portés par un co-polyaminoacide alors ils sont identiques ou différents,
   - le degré de polymérisation DP en unités glutamiques ou aspartiques est compris entre 5 et 250 ;
   - les fonctions acides libres étant sous forme de sel de cation alkalin choisi dans le groupe constitué par Na⁺ et K⁺.

La présente invention concerne également le copolyaminoacide tel que revendiqué à la revendication 15.

Dans un mode de réalisation, la composition est caractérisée en ce que le pH est compris entre 6,6 et 7,8.

Dans un mode de réalisation, la composition est caractérisée en ce que le pH est compris entre 7,0 et 7,8.

Dans un mode de réalisation, la composition est caractérisée en ce que le pH est compris entre 6,8 et 7,4.

Dans un mode de réalisation, la composition est caractérisée en ce que lesdits radicaux hydrophobes sont choisis parmi les radicaux hydrophobes de formule I dans laquelle si p est égal à 1 et si x est inférieur ou égal à 14 (x ≤ 14) alors r = 0 ou r = 1.

Dans un mode de réalisation, la composition est caractérisée en ce que lesdits radicaux hydrophobes sont choisis parmi les radicaux hydrophobes de formule I dans laquelle si p est égal à 1 et si x est compris entre 15 et 16 (15 ≤ x ≤ 16), alors r = 1.

Dans un mode de réalisation, la composition est caractérisée en ce que lesdits radicaux hydrophobes sont choisis parmi les radicaux hydrophobes de formule I dans laquelle si p est égal à 1 et si x est supérieur à 17 (17 ≤ x) alors r = 1 et R est un radical éther ou polyéther.

Dans un mode de réalisation, la composition est caractérisée en ce que lesdits radicaux hydrophobes sont choisis parmi les radicaux hydrophobes de formule I dans laquelle si p est égal à 1 alors x est compris entre 17 et 25 (17 ≤ x ≤ 25).

Dans un mode de réalisation, la composition est caractérisée en ce que lesdits radicaux hydrophobes sont choisis parmi les radicaux hydrophobes de formule I dans laquelle p = 1, représentée par la formule V suivante : 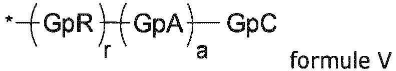 GpR, GpA, GpC, r et a ont les définitions données précédemment.

Dans un mode de réalisation, la composition est caractérisée en ce que le radical hydrophobe est un radical de formule V dans laquelle : r est égal à 1 (r=1) et a est égal à 0 (a = 0).

Dans un mode de réalisation, la composition est caractérisée en ce que le radical hydrophobe est un radical de formule V dans laquelle r est égal à 1 (r=1) et a est égal à 1 (a=1).

Dans un mode de réalisation, la composition est caractérisée en ce que le radical hydrophobe est un radical de formule V dans laquelle GpR est un radical de formule II.

Dans un mode de réalisation, la composition est caractérisée en ce que le radical hydrophobe est un radical de formule V dans laquelle GpR est un radical de formule II dans laquelle R est un radical alkyle linéaire divalent comprenant de 2 à 12 atomes de carbone.

Dans un mode de réalisation, la composition est caractérisée en ce que le radical hydrophobe est un radical de formule V dans laquelle GpR est un radical de formule II dans laquelle R est un radical alkyle divalent comprenant de 2 à 6 atomes de carbone.

Dans un mode de réalisation, la composition est caractérisée en ce que le radical hydrophobe est un radical de formule V dans laquelle GpR est un radical de formule II dans laquelle R est un radical alkyle linéaire divalent comprenant de 2 à 6 atomes de carbone.

Dans un mode de réalisation, la composition est caractérisée en ce que le radical hydrophobe est un radical de formule V dans laquelle GpR est un radical de formule II dans laquelle R est un radical alkyle divalent comprenant de 2 à 4 atomes de carbone.

Dans un mode de réalisation, la composition est caractérisée en ce que le radical hydrophobe est un radical de formule V dans laquelle GpR est un radical de formule II dans laquelle R est un radical alkyle linéaire divalent comprenant de 2 à 4 atomes de carbone.

Dans un mode de réalisation, la composition est caractérisée en ce que le radical hydrophobe est un radical de formule V dans laquelle GpR est un radical de formule II dans laquelle R est un radical alkyle divalent comprenant 2 atomes de carbone.

Dans un mode de réalisation, la composition est caractérisée en ce que le radical hydrophobe est un radical de formule V dans laquelle GpR est un radical de formule II'.

Dans un mode de réalisation, la composition est caractérisée en ce que le radical hydrophobe est un radical de formule V dans laquelle GpR est un radical de formule II, dans laquelle R est un radical alkyle divalent, comprenant de 2 à 5 atomes de carbone et portant une ou plusieurs fonctions amide (-CONH₂).

Dans un mode de réalisation, la composition est caractérisée en ce que le radical hydrophobe est un radical de formule V dans laquelle GpR est un radical de formule II, dans laquelle R est un radical alkyle linéaire divalent, comprenant de 2 à 5 atomes de carbone et portant une ou plusieurs fonctions amide (-CONH₂).

Dans un mode de réalisation, la composition est caractérisée en ce que le radical hydrophobe est un radical de formule V dans laquelle GpR est un radical de formule II dans laquelle R est un radical choisi dans le groupe constitué par les radicaux représentés par les formules ci-dessous :

| | |
|---|---|
| 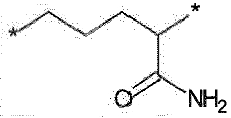 | Formule X1 |
| 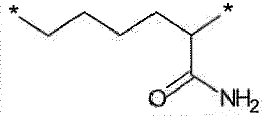 | Formule X2 |

Dans un mode de réalisation, la composition est caractérisée en ce que le radical R est lié au co-polyaminoacide via une fonction amide portée par le carbone en position delta ou epsilon (ou en position 4 ou 5) par rapport à la fonction amide (-CONH₂).

Dans un mode de réalisation, la composition est caractérisée en ce que le radical hydrophobe est un radical de formule V dans laquelle GpR est un radical de formule II ou II', dans laquelle R est un radical linéaire éther ou polyéther non substitué comprenant de 4 à 14 atomes de carbone et de 1 à 5 atomes d'oxygène.

Dans un mode de réalisation, la composition est caractérisée en ce que le radical hydrophobe est un radical de formule V dans laquelle GpR est un radical de formule II ou II', dans laquelle R est un radical éther.

Dans un mode de réalisation, la composition est caractérisée en ce que le radical hydrophobe est un radical de formule V dans laquelle GpR est un radical de formule II ou II', dans laquelle R est un radical éther comprenant de 4 à 6 atomes de carbone.

Dans un mode de réalisation, la composition est caractérisée en ce que le radical hydrophobe est un radical de formule V dans laquelle GpR est un radical de formule II dans laquelle R est un radical éther représenté par la formule 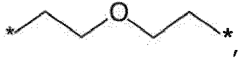 de formule X7.

Dans un mode de réalisation, la composition est caractérisée en ce que le radical hydrophobe est un radical de formule V dans laquelle GpR est un radical de formule II ou II', dans laquelle R est un radical polyéther.

Dans un mode de réalisation, la composition est caractérisée en ce que le radical hydrophobe est un radical de formule V dans laquelle GpR est un radical de formule II ou II', dans laquelle R est un radical linéaire polyéther comprenant de 6 à 10 atomes de carbone et de 2 à 3 atomes d'oxygène.

Dans un mode de réalisation, la composition est caractérisée en ce que le radical hydrophobe de formule V dans laquelle GpR est un radical de formule II ou II', dans laquelle R est un radical polyéther choisi dans le groupe constitué par les radicaux représentés par les formules ci-dessous :

| | |
|---|---|
| 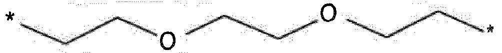 | Formule X3 |
| 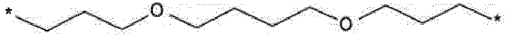 | Formule X4 |
| 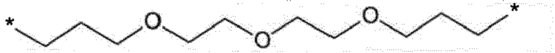 | Formule X5 |
| 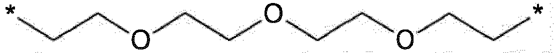 | Formule X6 |

Dans un mode de réalisation, la composition est caractérisée en ce que le radical hydrophobe de formule V dans laquelle GpR est un radical de formule II ou II', dans laquelle R est un radical de formule X3.

Dans un mode de réalisation, la composition est caractérisée en ce que le radical hydrophobe de formule V dans laquelle GpR est un radical de formule II ou II', dans laquelle R est un radical de formule X4.

Dans un mode de réalisation, la composition est caractérisée en ce que le radical hydrophobe de formule V dans laquelle GpR est un radical de formule II ou II', dans laquelle R est un radical de formule X5.

Dans un mode de réalisation, la composition est caractérisée en ce que le radical hydrophobe de formule V dans laquelle GpR est un radical de formule II ou II', dans laquelle R est un radical de formule X6.

Dans un mode de réalisation, la composition est caractérisée en ce que le radical hydrophobe de formule V dans laquelle GpR est un radical de formule II dans laquelle R est un radical polyéther choisi dans le groupe constitué par les radicaux représentés par les formules ci-dessous :

| | |
|---|---|
| 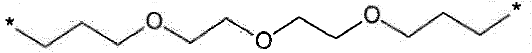 | Formule X5 |
| 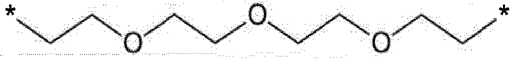 | Formule X6 |

Dans un mode de réalisation, la composition est caractérisée en ce que le radical hydrophobe de formule V dans laquelle GpR est un radical de formule II dans laquelle R est un radical polyéther de formule X5.

Dans un mode de réalisation, la composition est caractérisée en ce que le radical hydrophobe de formule V dans laquelle GpR est un radical de formule II dans laquelle R est un radical polyéther de formule X6.

Dans un mode de réalisation, la composition est caractérisée en ce que le radical hydrophobe est un radical de formule V dans laquelle a est égal à 0 (a=0) et r est égal à 0 (r=0).

Dans un mode de réalisation, la composition est caractérisée en ce que le radical hydrophobe est un radical de formule V dans laquelle a est égal à 1 (a = 1) et le radical GpA de formule III' est choisi dans le groupe constitué des radicaux représentés par les formules ci-dessous :

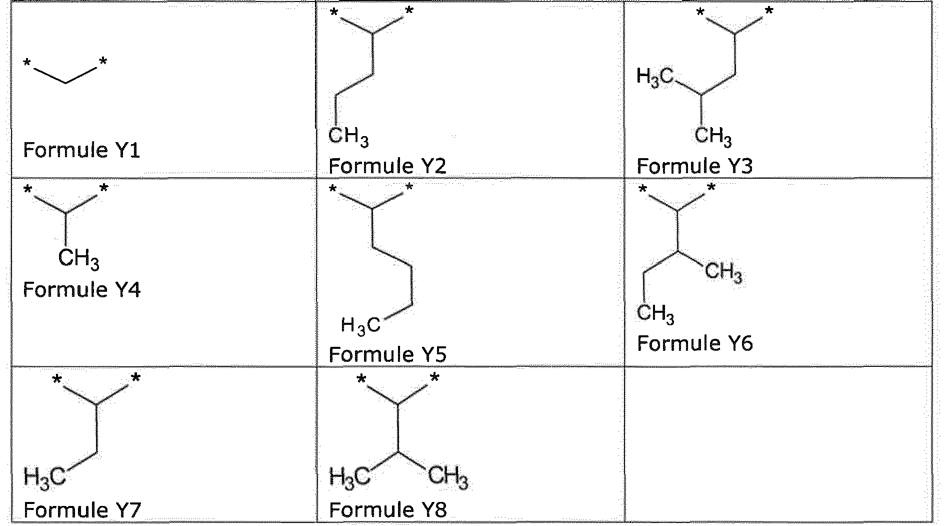

Dans un mode de réalisation, la composition est caractérisée en ce que le radical hydrophobe est un radical de formule V dans laquelle a est égal à 1 (a = 1) et le radical GpA de formule III' est un radical de formule Y1.

Dans un mode de réalisation, la composition est caractérisée en ce que le radical hydrophobe est un radical de formule V dans laquelle a est égal à 1 (a = 1) et le radical GpA de formule III' est un radical de formule Y2.

Dans un mode de réalisation, la composition est caractérisée en ce que le radical hydrophobe est un radical de formule V dans laquelle a est égal à 1 (a = 1) et le radical GpA de formule III' est un radical de formule Y3.

Dans un mode de réalisation, la composition est caractérisée en ce que le radical hydrophobe est un radical de formule V dans laquelle a est égal à 1 (a = 1) et le radical GpA de formule III' est un radical de formule Y4.

Dans un mode de réalisation, la composition est caractérisée en ce que le radical hydrophobe est un radical de formule V dans laquelle a est égal à 1 (a = 1) et le radical GpA de formule III' est un radical de formule Y5.

Dans un mode de réalisation, la composition est caractérisée en ce que le radical hydrophobe est un radical de formule V dans laquelle a est égal à 1 (a = 1) et le radical GpA de formule III' est un radical de formule Y6.

Dans un mode de réalisation, la composition est caractérisée en ce que le radical hydrophobe est un radical de formule V dans laquelle a est égal à 1 (a = 1) et le radical GpA de formule III' est un radical de formule Y7.

Dans un mode de réalisation, la composition est caractérisée en ce que le radical hydrophobe est un radical de formule V dans laquelle a est égal à 1 (a = 1) et le radical GpA de formule III' est un radical de formule Y8.

Dans un mode de réalisation, la composition est caractérisée en ce que le radical hydrophobe est un radical de formule V dans laquelle le radical GpC de formule IV est choisi dans le groupe constitué des radicaux de formules IVa, IVb ou IVc ci-après représentées :

| | |
|---|---|
| 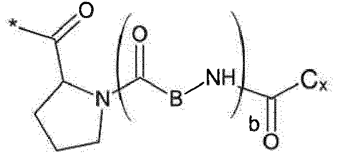 | Formule IVa |
| 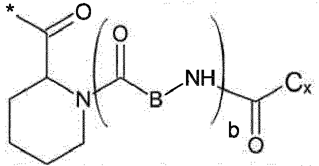 | Formule IVb |
| 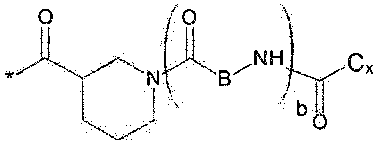 | Formule IVc |

Dans un mode de réalisation, la composition est caractérisée en ce que le radical hydrophobe est un radical de formule V dans laquelle le radical GpC est de formule IVa.

Dans un mode de réalisation, la composition est caractérisée en ce que le radical hydrophobe est un radical de formule V dans laquelle le radical GpC de formule IV est choisi dans le groupe constitué des radicaux de formules IVa, IVb ou IVc dans lesquels b est égal à 0, répondant respectivement aux formules IVd, IVe, et IVf ci-après représentées :

| | |
|---|---|
| 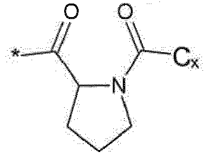 | Formule IVd |
| 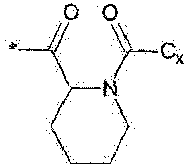 | Formule IVe |
| 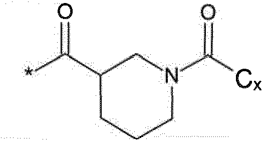 | Formule IVf |

Dans un mode de réalisation, la composition est caractérisée en ce que le radical hydrophobe est un radical de formule V dans laquelle le radical GpC répond à la formule IV ou IVa dans lesquelles b = 0, et répond à la formule IVd.

Dans un mode de réalisation, la composition est caractérisée en ce que le radical hydrophobe est un radical de formule V dans laquelle le radical GpC de formule IV dans laquelle b = 1 est choisi dans le groupe constitué des radicaux dans lesquels B est un résidu d'acide aminé choisi dans le groupe constitué par les radicaux représentés par les formules ci-dessous :

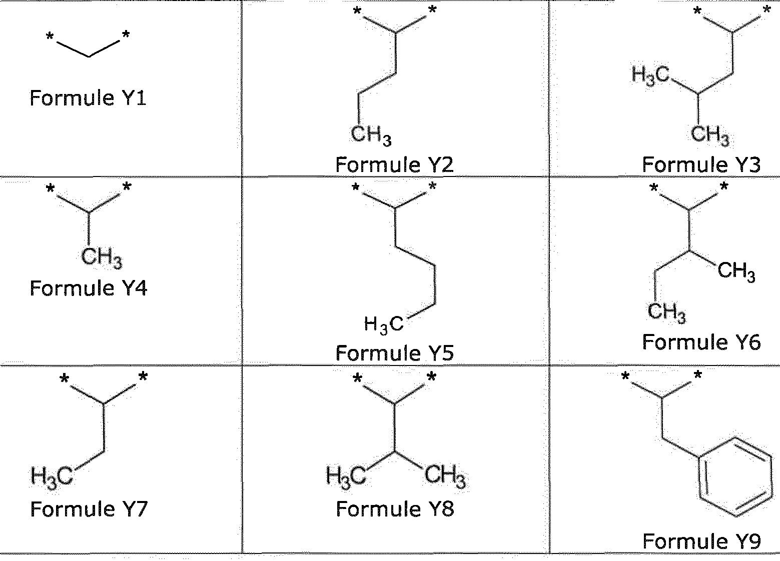

Dans un mode de réalisation, la composition est caractérisée en ce que le radical hydrophobe est un radical de formule V dans laquelle le radical GpC de formule IV ou IVa dans lesquelles b = 1, est choisi dans le groupe constitué des radicaux dans lesquels B est un résidu d'acide aminé choisi dans le groupe constitué par les radicaux représentés par les formules ci-dessous :

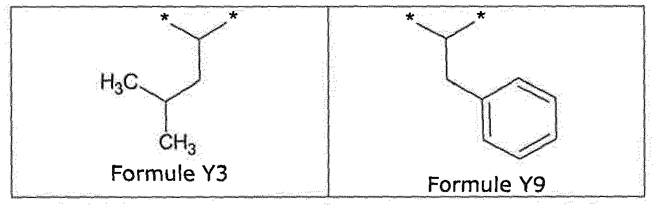

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles linéaires.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles ramifiés.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 11 et 14 atomes de carbone.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 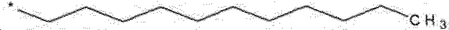 | x=11 |
| 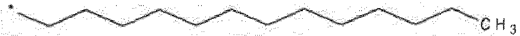 | x=13 |

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 15 et 16 atomes de carbone.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 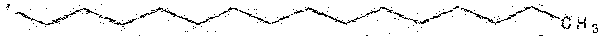 | x=15 |

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 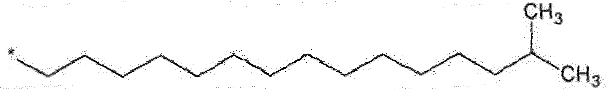 | x=16 |

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 17 et 25 atomes de carbone.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 17 et 18 atomes de carbone.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles représentés par les formules ci-dessous :

| | |
|---|---|
| 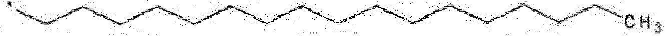 | x=17 |

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 18 et 25 atomes de carbone.

Dans un mode de réalisation, la composition est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles représentés par les formules ci-dessous :

| | |
|---|---|
| 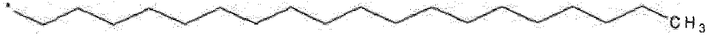 | x=19 |
| 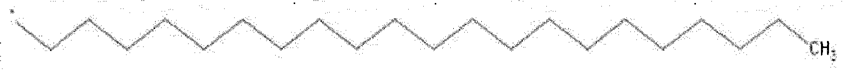 | x = 21 |

Dans un mode de réalisation, la composition est caractérisée en ce que lesdits radicaux hydrophobes sont choisis parmi les radicaux hydrophobes de formule I dans laquelle a = 1 et p = 2, représentée par la formule VI suivante : 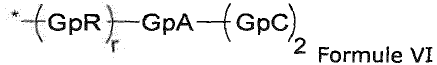 dans laquelle
GpR, GpA, GpC, r et a ont les définitions données précédemment.

Dans un mode de réalisation, la composition est caractérisée en ce que le radical hydrophobe est un radical de formule VI dans laquelle r=1.

Dans un mode de réalisation, la composition est caractérisée en ce que le radical hydrophobe est un radical de formule VI dans laquelle GpR est un radical de formule II.

Dans un mode de réalisation, la composition est caractérisée en ce que le radical hydrophobe est un radical de formule VI dans laquelle GpR est un radical de formule II dans lequel R est un radical alkyle linéaire divalent comprenant de 2 à 12 atomes de carbone.

Dans un mode de réalisation, la composition est caractérisée en ce que le radical hydrophobe est un radical de formule VI dans laquelle GpR est un radical de formule II dans laquelle R est un radical alkyle divalent comprenant de 2 à 6 atomes de carbone.

Dans un mode de réalisation, la composition est caractérisée en ce que le radical hydrophobe est un radical de formule VI dans laquelle GpR est un radical de formule II dans laquelle R est un radical alkyle linéaire divalent comprenant de 2 à 6 atomes de carbone.

Dans un mode de réalisation, la composition est caractérisée en ce que le radical hydrophobe est un radical de formule VI dans laquelle GpR est un radical de formule II dans laquelle R est un radical alkyle comprenant de 2 à 4 atomes de carbone.

Dans un mode de réalisation, la composition est caractérisée en ce que le radical hydrophobe est un radical de formule VI dans laquelle GpR est un radical de formule II dans laquelle R est un radical alkyle linéaire divalent comprenant de 2 à 4 atomes de carbone.

Dans un mode de réalisation, la composition est caractérisée en ce que le radical hydrophobe est un radical de formule VI dans laquelle GpR est un radical de formule II dans laquelle R est un radical alkyle linéaire divalent comprenant 2 atomes de carbone.

Dans un mode de réalisation, la composition est caractérisée en ce que le radical hydrophobe est un radical de formule VI dans laquelle GpR est un radical de formule II'.

Dans un mode de réalisation, la composition est caractérisée en ce que le radical hydrophobe est un radical de formule VI dans laquelle GpR est un radical de formule II, dans laquelle R est un radical alkyle divalent, comprenant de 2 à 5 atomes de carbone, et portant une ou plusieurs fonctions amide (-CONH2).

Dans un mode de réalisation, la composition est caractérisée en ce que le radical hydrophobe est un radical de formule VI dans laquelle GpR est un radical de formule II, dans laquelle R est un radical alkyle linéaire divalent, comprenant de 2 à 5 atomes de carbone et portant une ou plusieurs fonctions amide (-CONH2).

Dans un mode de réalisation, la composition est caractérisée en ce que le radical hydrophobe est un radical de formule VI dans laquelle GpR est un radical de formule II dans laquelle R est un radical choisi dans le groupe constitué par les radicaux représentés par les formules ci-dessous :

| | |
|---|---|
| 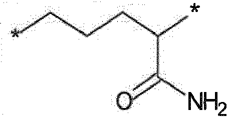 | Formule X1 |
| 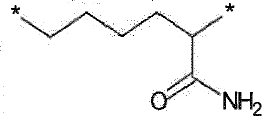 | Formule X2 |

Dans un mode de réalisation, la composition est caractérisée en ce que le radical hydrophobe est un radical de formule VI dans laquelle la fonction amine du radical GpR engagée dans la formation de la fonction amide qui lie ledit radical GpR au co-polyaminoacide est portée par un carbone en position delta ou epsilon (ou en position 4 ou 5) par rapport à la fonction amide (-CONH2).

Dans un mode de réalisation, la composition est caractérisée en ce que le radical hydrophobe est un radical de formule VI dans laquelle GpR est un radical de formule II, dans laquelle R est un radical linéaire éther ou polyéther non substitué comprenant de 4 à 14 atomes de carbone et de 1 à 5 atomes d'oxygène.

Dans un mode de réalisation, la composition est caractérisée en ce que le radical hydrophobe est un radical de formule VI dans laquelle GpR est un radical de formule II dans laquelle R est un radical éther.

Dans un mode de réalisation, la composition est caractérisée en ce que le radical éther R est un radical comprenant de 4 à 6 atomes de carbone.

Dans un mode de réalisation, la composition est caractérisée en ce que le radical éther est 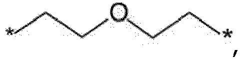 de formule X7.

Dans un mode de réalisation, la composition est caractérisée en ce que le radical hydrophobe est un radical de formule VI dans laquelle GpR est un radical de formule II, dans laquelle R est un radical polyéther.

Dans un mode de réalisation, la composition est caractérisée en ce que le radical hydrophobe est un radical de formule VI dans laquelle GpR est un radical de formule II, dans laquelle R est un radical linéaire polyéther comprenant de 6 à 10 atomes de carbone et de 2 à 3 atomes d'oxygène.

Dans un mode de réalisation, la composition est caractérisée en ce que le radical hydrophobe est un radical de formule VI dans laquelle GpR est un radical de formule II dans laquelle R est un radical linéaire polyéther choisi dans le groupe constitué par les radicaux représentés par les formules ci-dessous :

| | |
|---|---|
| 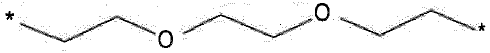 | Formule X3 |
| 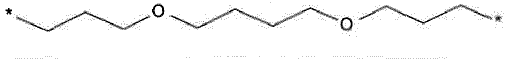 | Formule X4 |
| 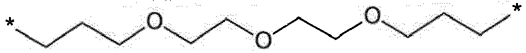 | Formule X5 |
| 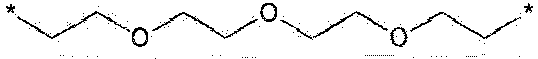 | Formule X6 |

Dans un mode de réalisation, la composition est caractérisée en ce que le radical hydrophobe est un radical de formule VI dans laquelle GpR est un radical de formule II dans laquelle R est un radical linéaire polyéther de formule X3.

Dans un mode de réalisation, la composition est caractérisée en ce que le radical hydrophobe est un radical de formule VI dans laquelle GpR est un radical de formule II dans laquelle R est un radical linéaire polyéther de formule X4.

Dans un mode de réalisation, la composition est caractérisée en ce que le radical hydrophobe est un radical de formule VI dans laquelle GpR est un radical de formule II dans laquelle R est un radical linéaire polyéther de formule X5.

Dans un mode de réalisation, la composition est caractérisée en ce que le radical hydrophobe est un radical de formule VI dans laquelle GpR est un radical de formule II dans laquelle R est un radical linéaire polyéther de formule X6.

Dans un mode de réalisation, la composition est caractérisée en ce que le radical hydrophobe est un radical de formule VI dans laquelle le radical GpA de formule III est choisi dans le groupe constitué des radicaux de formules IIIa, IIIb et IIIc ci-après représentées :

| | |
|---|---|
| 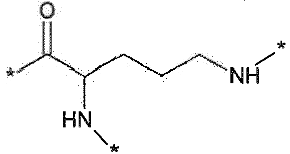 | Formule IIIa |
| 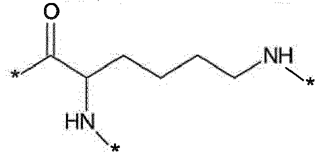 | Formule IIIb |
| 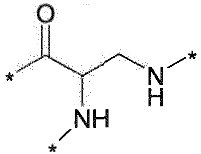 | Formule IIIc |

Dans un mode de réalisation, la composition est caractérisée en ce que le radical hydrophobe est un radical de formule VI dans laquelle le radical GpA de formule III est un radical de formule IIIb ci-après représentée :

| | |
|---|---|
| 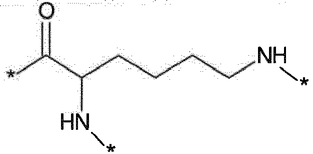 | Formule IIIb |

Dans un mode de réalisation, la composition est caractérisée en ce que le radical hydrophobe est un radical de formule VI dans laquelle le radical GpA de formule III est un radical de formule IIIc.

Dans un mode de réalisation, la composition est caractérisée en ce que le radical hydrophobe est un radical de formule VI dans laquelle le radical GpC de formule IV est choisi dans le groupe constitué des radicaux de formules IVa, IVb et IVc ci-après représentées :

| | |
|---|---|
| 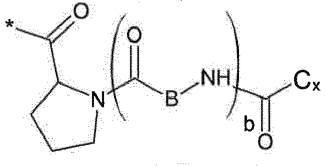 | Formule IVa |
| 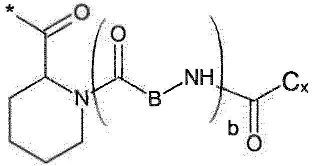 | Formule IVb |
| 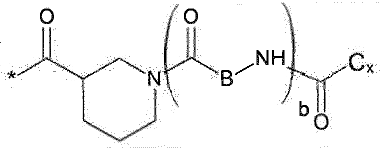 | Formule IVc |

Dans un mode de réalisation, la composition est caractérisée en ce que le radical hydrophobe est un radical de formule VI dans laquelle le radical GpC est de formule IVa.

Dans un mode de réalisation, la composition est caractérisée en ce que le radical hydrophobe est un radical de formule VI dans laquelle le radical GpC de formule IV est choisi dans le groupe constitué des radicaux de formules IVa, IVb ou IVc dans lesquels b est égal à 0, répondant respectivement aux formules IVd, IVe, et IVf ci-après représentées :

| | |
|---|---|
| 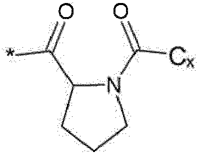 | Formule IVd |
| 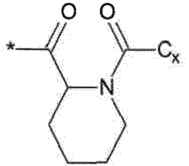 | Formule IVe |
| 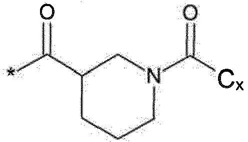 | Formule IVf |

Dans un mode de réalisation, la composition est caractérisée en ce que le radical hydrophobe est un radical de formule VI dans laquelle le radical GpC répond à la formule IV ou IVa dans lesquelles b = 0, et répond à la formule IVd.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles linéaires comprenant entre 9 et 15 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles ramifiés comprenant entre 9 et 15 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant 9 ou 10 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant entre 11 et 15 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant entre 11 et 13 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué car les radicaux représentés car les formules ci-dessous :

| | |
|---|---|
| 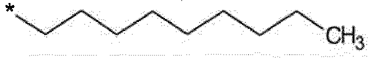 | x = 9 |
| 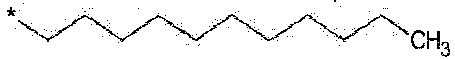 | x = 11 |
| 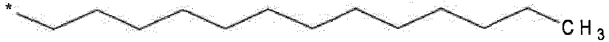 | x = 13 |

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant 14 ou 15 atomes de carbone.

Dans un mode de réalisation, la composition est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 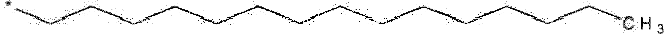 | x=15 |

Le co-polyaminoacide porteur de charges carboxylates et d'au moins un radical hydrophobe de formule I peut également être appelé « co-polyaminoacide » dans la présente description.

Dans un mode de réalisation, la composition est caractérisée en ce que le co-polyaminoacide porteur de charges carboxylates et de radicaux hydrophobes est choisi parmi les co-polyaminoacides de formule VIIa suivante : 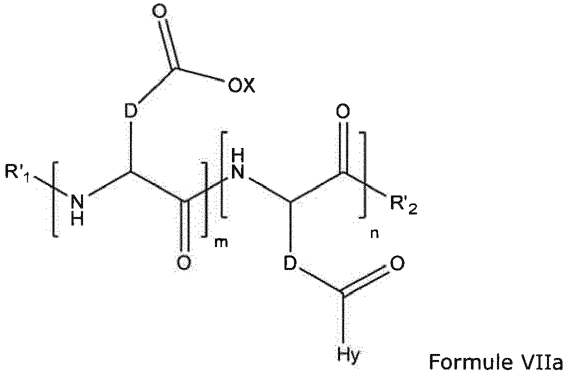 dans laquelle,
- D représente, indépendamment, soit un groupe -CH₂- (unité aspartique) soit un groupe -CH₂-CH₂- (unité glutamique),
- Hy est un radical hydrophobe choisi parmi les radicaux hydrophobes de formules I, V ou VI, dans lesquelles r = 1 et GpR est un radical de Formule II,
- X représente un H ou une entité cationique choisie dans le groupe comprenant les cations métalliques ;
- n + m représente le degré de polymérisation DP du co-polyaminoacide, c'est-à-dire le nombre moyen d'unités monomériques par chaîne de co-polyaminoacide et 5 ≤ n + m ≤ 250 ;R'1 est un radical choisi dans le groupe constitué par un H, un groupe acyle linéaire en C2 à C10, un groupe acyle ramifié en C4 à C10, un benzyle, une unité « acide aminé » terminale et un pyroglutamate,
- R'2 est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa dans laquelle Hy est un radical de formule V ou de formule VI, avec r=1.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa dans laquelle Hy est un radical de formule V ou de formule VI, avec GpR de formule II.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est un radical de formule V.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est de formule V et GpC est un radical de formule IVd.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est de formule V et GpC est un radical de formule IVd dans lequel x = 13.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est de formule V et GpC est un radical de formule IVd dans lequel x = 15.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est de formule V et GpC est un radical de formule IVd dans lequel x = 17.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est de formule V et GpC est un radical de formule IVd dans lequel x = 19.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est un radical de formule VI.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est un radical de formule VI dans laquelle r=1 et GpR de formule II.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est un radical de formule VI dans laquelle r=1, GpR de formule II et pour GpC, b=0.

Dans un mode de réalisation, la composition selon l'invention est caractérisée en ce que le co-polyaminoacide porteur de charges carboxylates et de radicaux hydrophobes est choisi parmi les co-polyaminoacides de formules VIIa, dans laquelle Hy est de formule VI, GpR de formule II et GpC est un radical de formule IVd.

Dans un mode de réalisation, la composition selon l'invention est caractérisée en ce que le co-polyaminoacide porteur de charges carboxylates et de radicaux hydrophobes est choisi parmi les co-polyaminoacides de formules VIIa, dans laquelle Hy est de formule VI, GpR de formule II et GpC est un radical de formule IVd et r=1.

Dans un mode de réalisation, la composition selon l'invention est caractérisée en ce que le co-polyaminoacide porteur de charges carboxylates et de radicaux hydrophobes est choisi parmi les co-polyaminoacides de formules VIIa, dans laquelle Hy est de formule VI, GpR de formule II et GpC est un radical de formule IVd dans lequel x est compris entre 11 et 15.

Dans un mode de réalisation, la composition selon l'invention est caractérisée en ce que lorsque le co-polyaminoacides comprend des unités aspartate, alors le co-polyaminoacides peut en outre comprendre des unités monomériques de formule VIII et/ou VIII' :

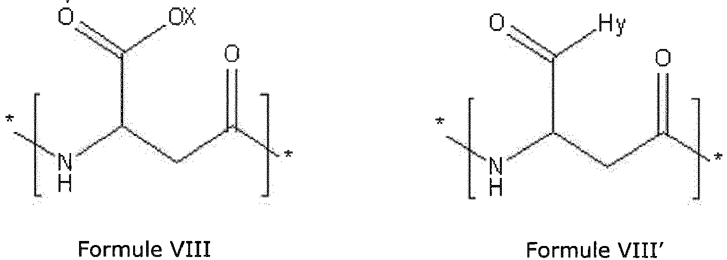

Dans un mode de réalisation, la composition est caractérisée en ce que R₁ est un radical choisi dans le groupe constitué par un groupe acyle linéaire en C₂ à C₁₀, un groupe acyle ramifié en C₄ à C₁₀, un benzyle, une unité « acide aminé » terminale et un pyroglutamate.

Dans un mode de réalisation, la composition est caractérisée en ce que R₁ est un radical choisi dans le groupe constitué par un groupe acyle linéaire en C₂ à C₁₀ ou un groupe acyle ramifié en C₄ à C₁₀.

Dans un mode de réalisation, la composition est caractérisée en ce que le co-polyaminoacide porteur de charges carboxylates et de radicaux hydrophobes est choisi parmi les co-polyaminoacides de formule VIIa dans laquelle le groupe D est un groupe -CH₂- (unité aspartique).

Dans un mode de réalisation, la composition est caractérisée en ce que le co-polyaminoacide porteur de charges carboxylates et de radicaux hydrophobes est choisi parmi les co-polyaminoacides de formule VIIa dans laquelle le groupe D est un groupe -CH₂-CH₂- (unité glutamique).

Dans un mode de réalisation, la composition est caractérisée en ce que le ratio i entre le nombre de radicaux hydrophobes et le nombre d'unités glutamiques ou aspartiques est compris entre 0,007 et 0,3.

Dans un mode de réalisation, la composition est caractérisée en ce que le ratio i entre le nombre de radicaux hydrophobes et le nombre d'unités glutamiques ou aspartiques est compris entre 0,01 et 0,3.

Dans un mode de réalisation, la composition est caractérisée en ce que le ratio i entre le nombre de radicaux hydrophobes et le nombre d'unités glutamiques ou aspartiques est compris entre 0,02 et 0,2.

Dans un mode de réalisation, la composition est caractérisée en ce que le radical hydrophobe répond à la formule VI et le ratio i entre le nombre de radicaux hydrophobes et le nombre d'unités glutamiques ou aspartiques est compris entre 0,007 et 0,15.

Dans un mode de réalisation, la composition est caractérisée en ce que le radical hydrophobe répond à la formule VI et le ratio i entre le nombre de radicaux hydrophobes et le nombre d'unités glutamiques ou aspartiques est compris entre 0,01 et 0,1.

Dans un mode de réalisation, la composition est caractérisée en ce que le radical hydrophobe répond à la formule VI et le ratio i entre le nombre de radicaux hydrophobes et le nombre d'unités glutamiques ou aspartiques est compris entre 0,02 et 0,08.

Dans un mode de réalisation, la composition est caractérisée en ce que le radical hydrophobe répond à la formule VI dans laquelle le radical Cx comprend entre 9 et 10 atomes de carbone et le ratio i entre le nombre de radicaux hydrophobes et le nombre d'unités glutamiques ou aspartiques est compris entre 0,03 et 0,15.

Dans un mode de réalisation, la composition est caractérisée en ce que le radical hydrophobe répond à la formule VI dans laquelle le radical Cx comprend entre 11 et 12 atomes de carbone et le ratio i entre le nombre de radicaux hydrophobes et le nombre d'unités glutamiques ou aspartiques est compris entre 0,015 et 0,1.

Dans un mode de réalisation, la composition est caractérisée en ce que le radical hydrophobe répond à la formule VI dans laquelle le radical Cx comprend entre 11 et 12 atomes de carbone et le ratio i entre le nombre de radicaux hydrophobes et le nombre d'unités glutamiques ou aspartiques est compris entre 0,02 et 0,08.

Dans un mode de réalisation, la composition est caractérisée en ce que le radical hydrophobe répond à la formule VI dans laquelle le radical Cx comprend entre 13 et 15 atomes de carbone et le ratio i entre le nombre de radicaux hydrophobes et le nombre d'unités glutamiques ou aspartiques est compris entre 0,01 et 0,1.

Dans un mode de réalisation, la composition est caractérisée en ce que le radical hydrophobe répond à la formule VI dans laquelle le radical Cx comprend entre 13 et 15 atomes de carbone et le ratio i entre le nombre de radicaux hydrophobes et le nombre d'unités glutamiques ou aspartiques est compris entre 0,01 et 0,06.

Dans un mode de réalisation, la composition est caractérisée en ce que le radical hydrophobe répond à la formule V et le ratio i entre le nombre de radicaux hydrophobes et le nombre d'unités glutamiques ou aspartiques est compris entre 0,007 et 0,3.

Dans un mode de réalisation, la composition est caractérisée en ce que le radical hydrophobe répond à la formule V et le ratio i entre le nombre de radicaux hydrophobes et le nombre d'unités glutamiques ou aspartiques est compris entre 0,01 et 0,3.

Dans un mode de réalisation, la composition est caractérisée en ce que le radical hydrophobe répond à la formule V et le ratio i entre le nombre de radicaux hydrophobes et le nombre d'unités glutamiques ou aspartiques est compris entre 0,015 et 0,2.

Dans un mode de réalisation, la composition est caractérisée en ce que le radical hydrophobe répond à la formule V dans laquelle le radical Cx comprend entre 11 et 14 atomes de carbone et le ratio i entre le nombre de radicaux hydrophobes et le nombre d'unités glutamiques ou aspartiques est compris entre 0,1 et 0,2.

Dans un mode de réalisation, la composition est caractérisée en ce que le radical hydrophobe répond à la formule V dans laquelle le radical Cx comprend entre 15 et 16 atomes de carbone et le ratio i entre le nombre de radicaux hydrophobes et le nombre d'unités glutamiques ou aspartiques est compris entre 0,04 et 0,15.

Dans un mode de réalisation, la composition est caractérisée en ce que le radical hydrophobe répond à la formule V dans laquelle le radical Cx comprend entre 17 et 18 atomes de carbone et le ratio i entre le nombre de radicaux hydrophobes et le nombre d'unités glutamiques ou aspartiques est compris entre 0,02 et 0,06.

Dans un mode de réalisation, la composition est caractérisée en ce que le radical hydrophobe répond à la formule V dans laquelle le radical Cx comprend entre 19 et 25 atomes de carbone et le ratio i entre le nombre de radicaux hydrophobes et le nombre d'unités glutamiques ou aspartiques est compris entre 0,01 et 0,06.

Dans un mode de réalisation, la composition est caractérisée en ce que le radical hydrophobe répond à la formule V dans laquelle le radical Cx comprend entre 19 et 25 atomes de carbone et le ratio i entre le nombre de radicaux hydrophobes et le nombre d'unités glutamiques ou aspartiques est compris entre 0,01 et 0,05.

Dans un mode de réalisation, la composition selon l'invention est caractérisée en ce que n + m est compris entre 10 et 250.

Dans un mode de réalisation, la composition selon l'invention est caractérisée en ce que n + m est compris entre 10 et 200.

Dans un mode de réalisation, la composition selon l'invention est caractérisée en ce que n + m est compris entre 15 et 150.

Dans un mode de réalisation, la composition selon l'invention est caractérisée en ce que n + m est compris entre 15 et 100.

Dans un mode de réalisation, la composition selon l'invention est caractérisée en ce que n + m est compris entre 15 et 80.

Dans un mode de réalisation, la composition selon l'invention est caractérisée en ce que n + m est compris entre 15 et 65.

Dans un mode de réalisation, la composition selon l'invention est caractérisée en ce que n + m est compris entre 20 et 60.

Dans un mode de réalisation, la composition selon l'invention est caractérisée en ce que n + m est compris entre 20 et 50.

Dans un mode de réalisation, la composition selon l'invention est caractérisée en ce que n + m est compris entre 20 et 40.

L'invention concerne également lesdits co-polyaminoacides porteurs de charges carboxylates et de radicaux hydrophobes de formule I et les précurseurs desdits radicaux hydrophobes.

Les co-polyaminoacides porteurs de charges carboxylates et de radicaux hydrophobes de formule I sont solubles dans l'eau distillée à un pH compris entre 6 et 8, à une température de 25°C et à une concentration inférieure à 100 mg/ml.

L'invention concerne en outre en une méthode de préparation de compositions injectables stables.

On entend par « soluble », susceptible de permettre de préparer une solution limpide et dépourvue de particules à une concentration inférieure à 100 mg/ml dans de l'eau distillée à 25°C.

On entend par « solution » une composition liquide dépourvue de particules visibles, en utilisant la procédure conforme aux pharmacopées EP 8.0, au point 2.9.20, et US <790>.

On entend par « composition stable physiquement » des compositions qui après une certaine durée de stockage à une certaine température satisfont aux critères de l'inspection visuelle décrite dans la pharmacopée européenne, américaine et internationale, c'est-à-dire des compositions qui sont limpides et qui ne contiennent pas de particules visibles, mais également incolores.

On entend par « composition stable chimiquement » des compositions qui, après stockage un certain temps et à une certaine température, présentent une recouvrance minimum des principes actifs et sont conformes aux cahiers des charges applicables aux produits pharmaceutiques.
Une méthode classique pour mesurer les stabilités des protéines ou peptides consiste à mesurer la formation de fibrilles à l'aide de Thioflavine T, encore appelée ThT. Cette méthode permet de mesurer dans des conditions de température et d'agitation qui permettent une accélération du phénomène, le temps de latence avant la formation de fibrilles par mesure de l'augmentation de la fluorescence. Les compositions selon l'invention ont un temps de latence avant la formation de fibrilles nettement supérieur à celui du glucagon au pH d'intérêt.

On entend par « solution aqueuse injectable » des solutions à base d'eau qui répondent aux conditions des pharmacopées EP et US, et qui sont suffisamment liquides pour être injectées.

On entend par « co-polyaminoacide étant constitué d'unités glutamiques ou aspartiques » des enchainements linéaires non cycliques d'unités acide glutamique ou acide aspartique liées entre elles par des liaisons peptidiques, lesdits enchainements présentant une partie C terminale, correspondant à l'acide carboxylique d'une extrémité, et une partie N-terminale, correspondant à l'amine de l'autre extrémité de l'enchainement.

On entend par « radical alkyl » une chaine carbonée, linéaire ou ramifiée, qui ne comprend pas d'hétéroatome.

Le co-polyaminoacide est un co-polyaminoacide statistique ou bloc.

Le co-polyaminoacide est un co-polyaminoacide statistique dans l'enchaînement des unités glutamiques et/ou aspartiques.

Dans les formules les * indiquent les sites de rattachements des différents éléments représentés.

Dans les formules I, V et VI, les * indiquent les sites de rattachement des radicaux hydrophobes au co-polyaminoacide. Les radicaux Hy sont rattachés au co-polyaminoacide via des fonctions amides.

Dans les formules II et II', les * indiquent, de gauche à droite respectivement, les sites de rattachement de GpR :
- au co-polyaminoacide et
- à GpA si a = 1 ou à GPC si a = 0.

Dans les formules III et III', les * indiquent, de gauche à droite respectivement, les sites de rattachement de GpA :
- à GpR si r = 1 ou au co-polyaminoacide si r = 0 et
- à GpC.

Dans la formule IV, le * indique le site de rattachement de GpC :
- à GpA si a = 1, GpR si r = 1 et a = 0 ou au co-polyaminoacide si r = 0 et a = 0.

Tous les rattachements entre les différents groupes GpR, GpA et GpC sont des fonctions amides.

Les radicaux Hy, GpR, GpA, GpC, et D sont chacun indépendamment identiques ou différents d'une unité monomérique à l'autre.

Lorsque le co-polyaminoacide comprend une ou plusieurs d'unité(s) aspartique(s), celle(s)-ci peu(ven)t subir des réarrangements structuraux.

Dans un mode de réalisation la composition selon l'invention est caractérisée en ce que les co-polyaminoacides peuvent en outre comprendre des unités monomériques de formule VIII et/ou VIII' :

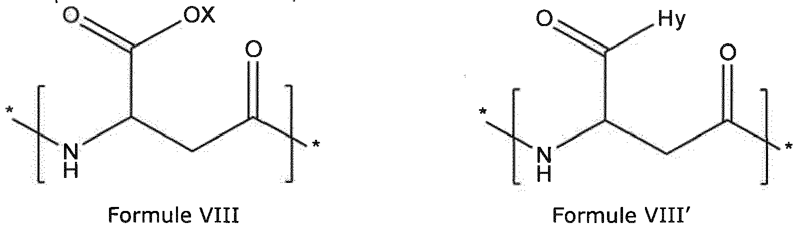

Dans un mode de réalisation, le au moins un radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVd dans laquelle x = 15 et Cx est

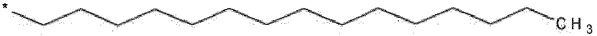

Dans un mode de réalisation, le au moins un radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVd dans laquelle x = 16 et Cx est

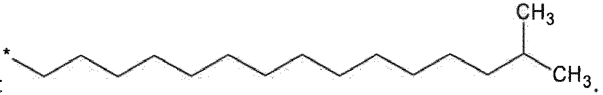

Dans un mode de réalisation, le au moins un radical hydrophobe de formule I est choisi parmi les radicaux de formule I dans laquelle, r = 1, a = 0, p = 1, GpR répond à la formule II dans laquelle R est 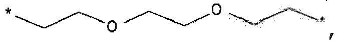 GpC répond à la formule IVd dans laquelle x = 15 et Cx est

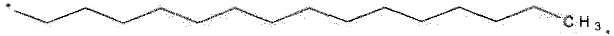

Dans un mode de réalisation, le au moins un radical hydrophobe de formule I est choisi parmi les radicaux de formule I dans laquelle, r = 1, a = 0, p = 1, GpR répond à la formule II dans laquelle R est 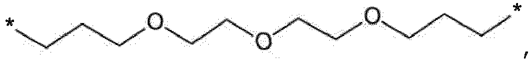 GpC répond à la formule IVd dans laquelle x = 15 et Cx est

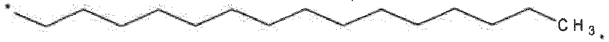

Dans un mode de réalisation, le au moins un radical hydrophobe de formule I est choisi parmi les radicaux de formule I dans laquelle, r = 1, a = 0, p = 1, GpR répond à la formule II dans laquelle R est 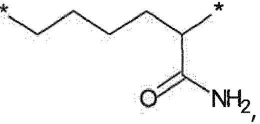 GpC répond à la formule IVd dans laquelle x = 15 et Cx est

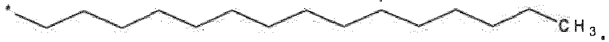

Dans un mode de réalisation, le au moins un radical hydrophobe de formule I est choisi parmi les radicaux de formule I dans laquelle, r = 1, a = 0, p = 1, GpR répond à la formule II dans laquelle R est 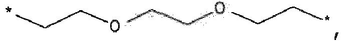 GpC répond à la formule IVd dans laquelle x = 17 et Cx est

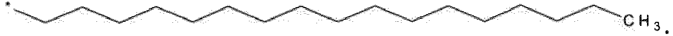

Dans un mode de réalisation, le au moins un radical hydrophobe de formule I est choisi parmi les radicaux de formule I dans laquelle, r = 1, a = 0, p = 1, GpR répond à la formule II dans laquelle R est 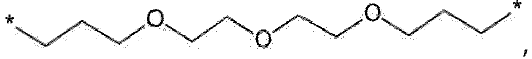 GpC répond à la formule IVd dans laquelle x = 19 et Cx est

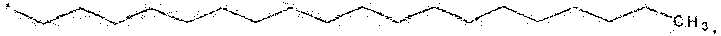

Dans un mode de réalisation, le au moins un radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVa dans laquelle b = 1, B est 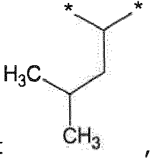 x = 15 et Cx est

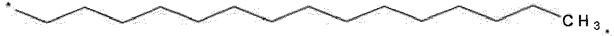

Dans un mode de réalisation, le au moins un radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVa dans laquelle b = 1, B est 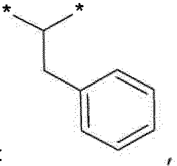 x = 11 et Cx est

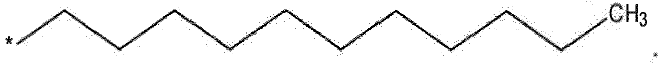

Dans un mode de réalisation, le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 9 et Cx est

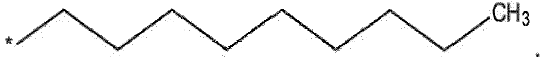

Dans un mode de réalisation, le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 11 et Cx est

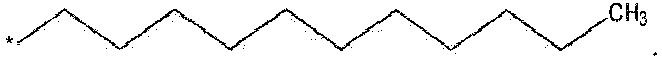

Dans un mode de réalisation, le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 13 et Cx est

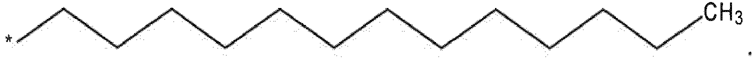

Dans un mode de réalisation, le au moins un radical hydrophobe de formule I est choisi parmi les radicaux de formule I dans laquelle, r = 1, a = 1, p = 2, GpR répond à la formule II dans laquelle R est 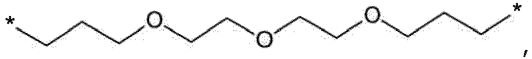 GpA répond à la formule IIIb, GpC répond à la formule IVd dans laquelle x = 13 et Cx est

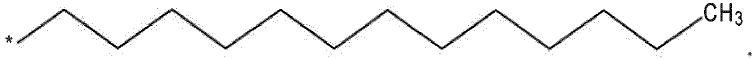

Dans un mode de réalisation, le co-polyaminoacide porteur de charges carboxylates et de radicaux hydrophobes est un co-polyaminoacide de formule VII ou VIIa, dans laquelle DP = 23 +/- 5, i = 0,05 +/- 0,02 et le au moins un radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVd dans laquelle x = 15 et Cx est

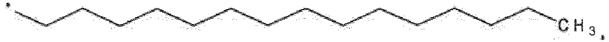

Les valeurs de degré de polymérisation DP et de ratio i sont estimés par RMN ¹H dans D₂O en comparant l'intégration des signaux provenant des groupes hydrophobes à celle des signaux provenant de la chaine principale du co-polyaminoacide.

Dans un mode de réalisation, le co-polyaminoacide porteur de charges carboxylates et de radicaux hydrophobes est un co-polyaminoacide de formule VII ou VIIa, dans laquelle DP = 35 +/- 5, i = 0,05 +/- 0,02 et le au moins un radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVd dans laquelle x = 15 et Cx est

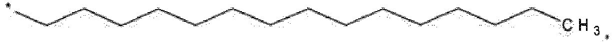

Dans un mode de réalisation, le co-polyaminoacide porteur de charges carboxylates et de radicaux hydrophobes est un co-polyaminoacide de formule VII ou VIIa, dans laquelle DP = 35 +/- 5, i = 0,10 +/- 0,02 et le au moins un radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVd dans laquelle x = 15 et Cx est

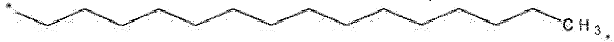

Dans un mode de réalisation, le co-polyaminoacide porteur de charges carboxylates et de radicaux hydrophobes est un co-polyaminoacide de formule VII ou VIIa, dans laquelle DP = 35 +/- 5, i = 0,05 +/- 0,02 et le au moins un radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVd dans laquelle x = 16 et Cx est

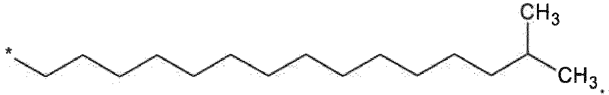

Dans un mode de réalisation, le co-polyaminoacide porteur de charges carboxylates et de radicaux hydrophobes est un co-polyaminoacide de formule VII ou VIIa, dans laquelle DP = 23 +/- 5, i = 0,05 +/- 0,02 et le au moins un radical hydrophobe de formule I est choisi parmi les radicaux de formule I dans laquelle, r = 1, a = 0, p = 1, GpR répond à la formule II dans laquelle R est 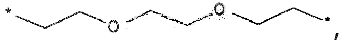 GpC répond à la formule IVd dans laquelle x = 17 et Cx est

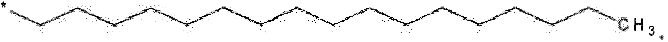

Dans un mode de réalisation, le co-polyaminoacide porteur de charges carboxylates et de radicaux hydrophobes est un co-polyaminoacide de formule VII ou VIIa, dans laquelle DP = 22 +/- 5, i = 0,05 +/- 0,02 et le au moins un radical hydrophobe de formule I est choisi parmi les radicaux de formule I dans laquelle, r = 1, a = 0, p = 1, GpR répond à la formule II dans laquelle R est 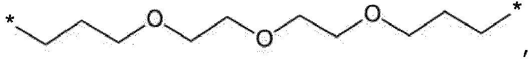 GpC répond à la formule IVd dans laquelle x = 19 et Cx est

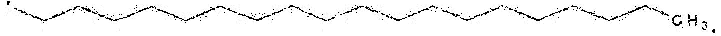

Dans un mode de réalisation, le co-polyaminoacide porteur de charges carboxylates et de radicaux hydrophobes est un co-polyaminoacide de formule VII ou VIIa, dans laquelle DP = 30 +/- 5, i = 0,10 +/- 0,03 et le au moins un radical hydrophobe de formule I est choisi parmi les radicaux de formule I dans laquelle, r = 1, a = 0, p = 1, GpR répond à la formule II dans laquelle R est 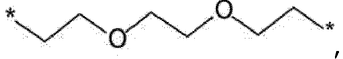 GpC répond à la formule IVd dans laquelle x = 15 et Cx est

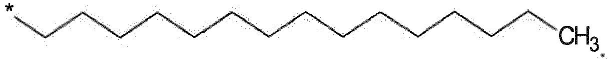

Dans un mode de réalisation, le co-polyaminoacide porteur de charges carboxylates et de radicaux hydrophobes est un co-polyaminoacide de formule VII ou VIIa, dans laquelle DP = 23 +/- 5, i = 0,07 +/- 0,02 et le au moins un radical hydrophobe de formule I est choisi parmi les radicaux de formule I dans laquelle, r = 1, a = 0, p = 1, GpR répond à la formule II dans laquelle R est 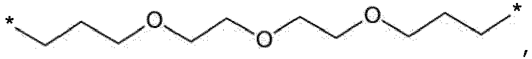 GpC répond à la formule IVd dans laquelle x = 15 et Cx est

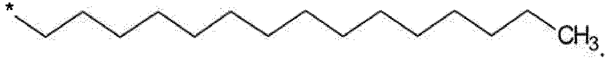

Dans un mode de réalisation, le co-polyaminoacide porteur de charges carboxylates et de radicaux hydrophobes est un co-polyaminoacide de formule VII ou VIIa, dans laquelle DP = 23 +/- 5, i = 0,05 +/- 0,02 et le au moins un radical hydrophobe de formule I est choisi parmi les radicaux de formule I dans laquelle, r = 1, a = 0, p = 1, GpR répond à la formule II dans laquelle R est 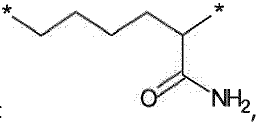 GpC répond à la formule IVd dans laquelle x = 15 et Cx est

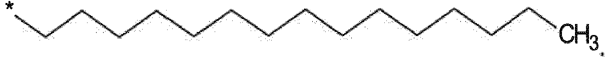

Dans un mode de réalisation, le co-polyaminoacide porteur de charges carboxylates et de radicaux hydrophobes est un co-polyaminoacide de formule VII ou VIIa, dans laquelle DP = 26 +/- 5, i = 0,04 +/- 0,02 et le au moins un radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVa dans laquelle b = 1, B est 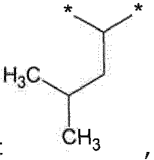 x = 15 et Cx est

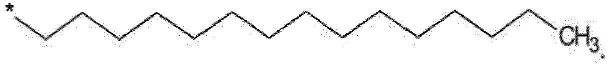

Dans un mode de réalisation, le co-polyaminoacide porteur de charges carboxylates et de radicaux hydrophobes est un co-polyaminoacide de formule VII ou VIIa, dans laquelle DP = 35 +/- 5, i = 0,13 +/- 0,04 et le au moins un radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVa dans laquelle b = 1, B est 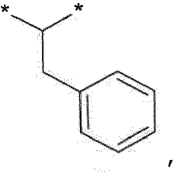 x = 11 et Cx est

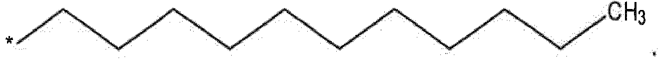

Dans un mode de réalisation, le co-polyaminoacide porteur de charges carboxylates et de radicaux hydrophobes est un co-polyaminoacide de formule VII ou VIIa, dans laquelle DP = 23 +/- 5, i = 0,05 +/- 0,02 et le au moins un radical hydrophobe de formule I est choisi parmi les radicaux de formule I dans laquelle, r = 1, a = 0, p = 1, GpR répond à la formule II dans laquelle R est 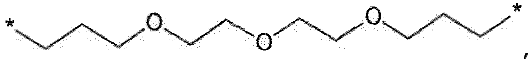 GpC répond à la formule IVd dans laquelle x = 19 et Cx est

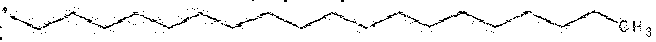

Dans un mode de réalisation, le co-polyaminoacide porteur de charges carboxylates et de radicaux hydrophobes est un co-polyaminoacide de formule VII ou VIIb, dans laquelle DP = 25 +/- 5, 0,033 ≤ i ≤ 0,05 et le radical hydrophobe de formule I est choisi parmi les radicaux de formule I dans laquelle, r = 1, a = 0, p = 1, GpR répond à la formule II dans laquelle R est -CH₂-CH₂-, GpC répond à la formule IVd dans laquelle x = 15 et Cx est

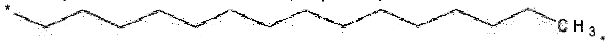

Dans un mode de réalisation, le co-polyaminoacide porteur de charges carboxylates et de radicaux hydrophobes est un co-polyaminoacide de formule VII ou VIIb, dans laquelle DP = 30 +/- 5, 0,028 ≤ i ≤ 0,04et le au moins un radical hydrophobe de formule I est choisi parmi les radicaux de formule I dans laquelle, r = 1, a = 0, p = 1, GpR répond à la formule II dans laquelle R est 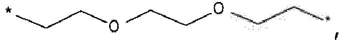 GpC répond à la formule IVd dans laquelle x = 17 et Cx est

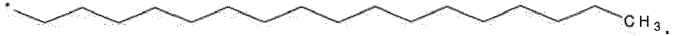

Dans un mode de réalisation, le co-polyaminoacide porteur de charges carboxylates et de radicaux hydrophobes est un co-polyaminoacide de formule VII ou VIIb, dans laquelle DP = 45 +/- 10, 0,018 ≤ i ≤ 0,028 et le au moins un radical hydrophobe de formule I est choisi parmi les radicaux de formule I dans laquelle, r = 1, a = 0, p = 1, GpR répond à la formule II dans laquelle R est 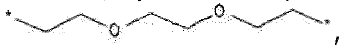 GpC répond à la formule IVd dans laquelle x = 17 et Cx est

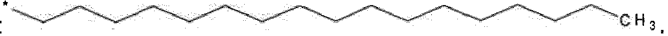

Dans un mode de réalisation, le co-polyaminoacide porteur de charges carboxylates et de radicaux hydrophobes est un co-polyaminoacide de formule VII ou VIIb, dans laquelle DP = 60 +/- 10, 0,014 ≤ i ≤ 0,02 et le au moins un radical hydrophobe de formule I est choisi parmi les radicaux de formule I dans laquelle, r = 1, a = 0, p = 1, GpR répond à la formule II dans laquelle R est 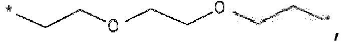 GpC répond à la formule IVd dans laquelle x = 17 et Cx est

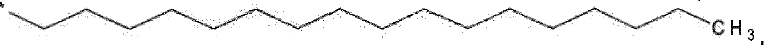

Dans un mode de réalisation, le co-polyaminoacide porteur de charges carboxylates et de radicaux hydrophobes est un co-polyaminoacide de formule VII ou VIIb, dans laquelle DP = 25 +/- 5, 0,033 ≤ i ≤ 0,05 et le radical hydrophobe de formule I est choisi parmi les radicaux de formule I dans laquelle, r = 1, a = 0, p = 1, GpR répond à la formule II dans laquelle R est 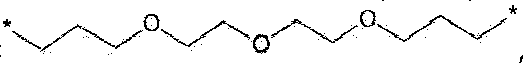 GpC répond à la formule IVd dans laquelle x = 19 et Cx est

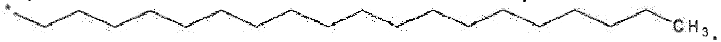

Dans un mode de réalisation, le co-polyaminoacide porteur de charges carboxylates et de radicaux hydrophobes est un co-polyaminoacide de formule VII ou VIIa, dans laquelle DP = 22 +/- 5, i = 0,05 +/- 0,02 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb , GpC répond à la formule IVd dans laquelle x = 11 et Cx est

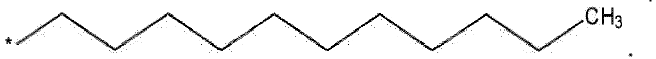

Dans un mode de réalisation, le co-polyaminoacide porteur de charges carboxylates et de radicaux hydrophobes est un co-polyaminoacide de formule VII ou VIIa, dans laquelle DP = 35 +/- 5, i = 0,05 +/- 0,02 et le au moins un radical hydrophobe de formule I est choisi parmi les radicaux de formule I dans laquelle, r = 1, a = 1, p = 2, GpR répond à la formule II et dans laquelle R est -CH₂-CH₂-, GpA répond à la formule IIIb , GpC répond à la formule IVd dans laquelle x = 11 et Cx est

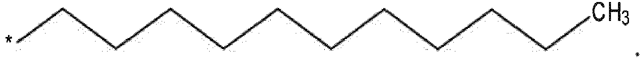

Dans un mode de réalisation, le co-polyaminoacide porteur de charges carboxylates et de radicaux hydrophobes est un co-polyaminoacide de formule VII ou VIIa, dans laquelle DP = 65 +/- 5, i = 0,05 +/- 0,02 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb , GpC répond à la formule IVd dans laquelle x = 11 et Cx est

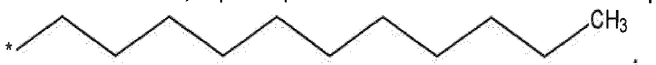

Dans un mode de réalisation, le co-polyaminoacide porteur de charges carboxylates et de radicaux hydrophobes est un co-polyaminoacide de formule VII ou VIIa, dans laquelle DP = 22 +/- 5, i = 0,04 +/- 0,02 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb , GpC répond à la formule IVd dans laquelle x = 13 et Cx est

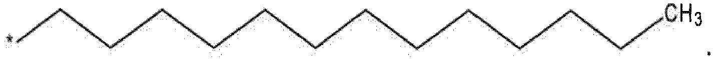

Dans un mode de réalisation, le co-polyaminoacide porteur de charges carboxylates et de radicaux hydrophobes est un co-polyaminoacide de formule VII ou VIIa, dans laquelle DP = 22 +/- 5, 0,03 +/- 0,01 et le au moins un radical hydrophobe de formule I est choisi parmi les radicaux de formule I dans laquelle, r = 1, a = 1, p = 2, GpR répond à la formule II dans laquelle R est 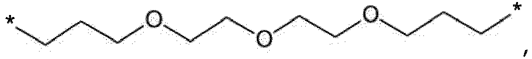 GpA répond à la formule IIIb , GpC répond à la formule IVd dans laquelle x = 13 et Cx est

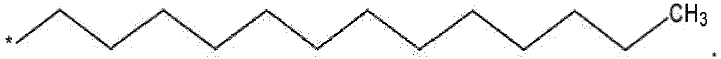

Dans un mode de réalisation, le co-polyaminoacide porteur de charges carboxylates et de radicaux hydrophobes est un co-polyaminoacide de formule VII ou VIIa, dans laquelle DP = 22 +/- 5, i = 0,07 +/- 0,02 et le au moins un radical hydrophobe de formule I est choisi parmi les radicaux de formule I dans laquelle, r = 1, a = 1, p = 2, GpR répond à la formule II dans laquelle R est 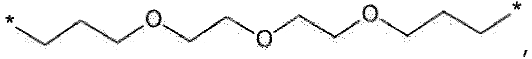 GpA répond à la formule IIIb , GpC répond à la formule IVd dans laquelle x = 9 et Cx est

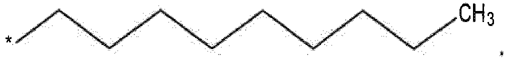

Dans un mode de réalisation, le co-polyaminoacide porteur de charges carboxylates et de radicaux hydrophobes est un co-polyaminoacide de formule VII ou VIIb, dans laquelle DP = 27 +/- 5, 0,031 ≤ i ≤ 0,045 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb , GpC répond à la formule IVd dans laquelle x = 11 et Cx est

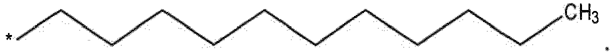

Dans un mode de réalisation, le co-polyaminoacide porteur de charges carboxylates et de radicaux hydrophobes est un co-polyaminoacide de formule VII ou VIIb, dans laquelle DP = 22 +/- 5, 0,037 ≤ i ≤ 0,055 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb , GpC répond à la formule IVd dans laquelle x = 13 et Cx est

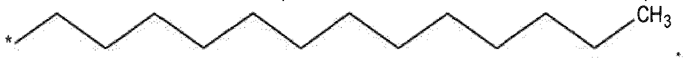

Dans un mode de réalisation, le co-polyaminoacide porteur de charges carboxylates et de radicaux hydrophobes est un co-polyaminoacide de formule VII ou VIIb, dans laquelle DP = 22 +/- 5, 0,037 ≤ i ≤ 0,055 et le au moins un radical hydrophobe de formule I est choisi parmi les radicaux de formule I dans laquelle, r = 1, a = 1, p = 2 GpR répond à la formule II dans laquelle R est 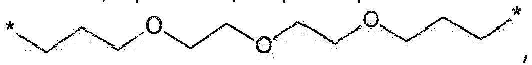 GpA répond à la formule IIIb , GpC répond à la formule IVd dans laquelle x = 13 et Cx est

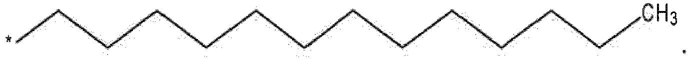

Dans un mode de réalisation, le co-polyaminoacide porteur de charges carboxylates et de radicaux hydrophobes est un co-polyaminoacide de formule VII ou VIIb, dans laquelle DP = 60 +/- 10, 0,014 ≤ i ≤ 0,02 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb , GpC répond à la formule IVd dans laquelle x = 13 et Cx est

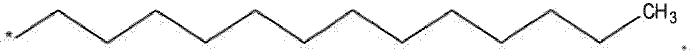

Dans un mode de réalisation, le co-polyaminoacide porteur de charges carboxylates et de radicaux hydrophobes est un co-polyaminoacide de formule VII ou VIIb, dans laquelle DP = 40 +/- 5, 0,022 ≤ i ≤ 0,029 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb , GpC répond à la formule IVd dans laquelle x = 13 et Cx est

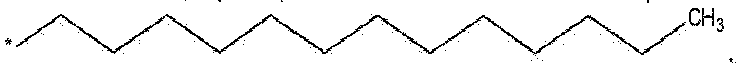

Dans un mode de réalisation, la composition selon l'invention est caractérisée en ce que le co-polyaminoacide est issu d'un polyaminoacide obtenu par polymérisation.

Dans un mode de réalisation, la composition selon l'invention est caractérisée en ce que le co-polyaminoacide est issu d'un polyaminoacide obtenu par polymérisation par ouverture de cycle d'un dérivé de N-carboxyanhydride d'acide glutamique ou d'un dérivé de N-carboxyanhydride d'acide aspartique.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique comme décrit dans l'article de revue Adv. Polym. Sci. 2006, 202, 1-18 (Deming, T.J.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique choisi dans le groupe constitué par le N-carboxyanhydride glutamate de méthyle (GluOMe-NCA), le N-carboxyanhydride glutamate de benzyle (GluOBzl-NCA) et le N-carboxyanhydride glutamate de t-butyle (GluOtBu-NCA).

Dans un mode de réalisation, le dérivé de N-carboxyanhydride d'acide glutamique est le N-carboxyanhydride L-glutamate de méthyle (L-GluOMe-NCA).

Dans un mode de réalisation, le dérivé de N-carboxyanhydride d'acide glutamique est le N-carboxyanhydride L-glutamate de benzyle (L-GluOBzl-NCA).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur un complexe organométallique d'un métal de transition comme décrit dans la publication Nature 1997, 390, 386-389 (Deming, T.J.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'ammoniaque ou une amine primaire comme décrit dans le brevet FR 2,801,226 (Touraud, F. ; et al.) et les références citées par ce brevet.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'hexaméthyldisilazane comme décrit dands la publication J. Am. Chem. Soc. 2007, 129, 14114-14115 (Lu H. ; et al.) ou une amine silylée comme décrit dans la publication J. Am. Chem. Soc. 2008, 130, 12562-12563 (Lu H. ; et al.).

Dans un mode de réalisation, la composition selon l'invention est caractérisée en ce que le procéde de synthèse du polyaminoacide obtenu par polymérisation d'un dérivé de N-carboxyanhydride d'acide glutamique ou d'un dérivé de N-carboxyanhydride d'acide aspartique dont est issu le co-polyaminoacide comprend une étape d'hydrolyse de fonctions ester.

Dans un mode de réalisation, cette étape d'hydrolyse de fonctions ester peut consister en une hydrolyse en milieu acide ou une hydrolyse en milieu basique ou être effectuée par hydrogénation.

Dans un mode de réalisation, cette étape d'hydrolyse de groupements ester est une hydrolyse en milieu acide.

Dans un mode de réalisation, cette étape d'hydrolyse de groupements ester est effectuée par hydrogénation.

Dans un mode de réalisation, la composition selon l'invention est caractérisée en ce que le co-polyaminoacide est issu d'un polyaminoacide obtenu par dépolymérisation d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation enzymatique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation chimique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation enzymatique et chimique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation d'un polyaminoacide de plus haut poids moléculaire choisi dans le groupe constitué par le polyglutamate de sodium et le polyaspartate de sodium.

Dans un mode de réalisation, la composition selon l'invention est caractérisée en ce que le co-polyaminoacide est issu d'un polyaminoacide obtenu par dépolymérisation d'un polyglutamate de sodium de plus haut poids moléculaire.

Dans un mode de réalisation, la composition selon l'invention est caractérisée en ce que le co-polyaminoacide est issu d'un polyaminoacide obtenu par dépolymérisation d'un polyaspartate de sodium de plus haut poids moléculaire.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide en utilisant les procédés de formation de liaison amide bien connus de l'homme de l'art.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide en utilisant les procédés de formation de liaison amide utilisés pour la synthèse peptidique.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide comme décrit dans le brevet FR 2,840,614 (Chan, Y.P. ; et al.).

Dans un mode de réalisation, la concentration en co-polyaminoacide porteur de charges carboxylates et de radicaux hydrophobes est au plus de 40 mg/mL.

Dans un mode de réalisation, la concentration en co-polyaminoacide porteur de charges carboxylates et de radicaux hydrophobes est au plus de 30 mg/mL.

Dans un mode de réalisation, la concentration en co-polyaminoacide porteur de charges carboxylates et de radicaux hydrophobes est au plus de 20 mg/mL.

Dans un mode de réalisation, la concentration en co-polyaminoacide porteur de charges carboxylates et de radicaux hydrophobes est au plus de 10 mg/mL.

Dans un mode de réalisation, la concentration en co-polyaminoacide porteur de charges carboxylates et de radicaux hydrophobes est au plus de 5 mg/mL.

Dans un mode de réalisation, la concentration en co-polyaminoacide porteur de charges carboxylates et de radicaux hydrophobes est au plus de 2,5 mg/mL.

Dans un mode de réalisation, la concentration en co-polyaminoacide porteur de charges carboxylates et de radicaux hydrophobes est au plus de 1 mg/mL.

Dans un mode de réalisation, la concentration en co-polyaminoacide porteur de charges carboxylates et de radicaux hydrophobes est au plus de 0,5 mg/mL.

Dans un mode de réalisation, le ratio massique co-polyaminoacide porteur de charges carboxylates et de radicaux hydrophobes sur glucagon est compris entre 1,5 et 25.

Dans un mode de réalisation, le ratio massique co-polyaminoacide porteur de charges carboxylates et de radicaux hydrophobes sur glucagon est compris entre 2 et 20.

Dans un mode de réalisation, le ratio massique co-polyaminoacide porteur de charges carboxylates et de radicaux hydrophobes sur glucagon est compris entre 2,5 et 15.

Dans un mode de réalisation, le ratio massique co-polyaminoacide porteur de charges carboxylates et de radicaux hydrophobes sur glucagon est compris entre 2 et 10.

Dans un mode de réalisation, le ratio massique co-polyaminoacide porteur de charges carboxylates et de radicaux hydrophobes sur glucagon est compris entre 2 et 7.

Le glucagon humain est utilisé à des posologies qui varient en fonction des applications.

En traitement d'urgence des hypoglycémies la posologie recommandée est de 1 mg par voie intramusculaire ou intraveineuse (0,5 mg si la masse corporelle est inférieur à 25 kg). Cette administration est effectuée avec une solution de glucagon humain à la concentration de 1 mg/ml.

Dans les pompes, la dose journalière envisagée est d'environ 0,5 mg, les solutions peuvent ainsi comprendre de 0,25 mg/ml à 5 mg/ml de glucagon humain.

Selon un mode de réalisation les solutions peuvent comprendre de 0,5 mg/ml à 3 mg/ml de glucagon humain.

Dans le traitement de l'obésité la dose journalière envisagée est d'environ 0,5 mg, les solutions peuvent ainsi comprendre de 0,25 mg/ml à 5 mg/ml de glucagon humain.

Dans un mode de réalisation, la concentration en glucagon humain est comprise entre 0,25 et 5 mg/mL.

Dans un mode de réalisation, la concentration en glucagon humain est comprise entre 0,5 et 4 mg/mL.

Dans un mode de réalisation, la concentration en glucagon humain est comprise entre 0,75 et 3 mg/mL.

Dans un mode de réalisation, la concentration en glucagon humain est comprise entre 0,75 et 2,5 mg/mL.

Dans un mode de réalisation, la concentration en glucagon humain est comprise entre 0,75 et 2 mg/mL.

Dans un mode de réalisation, la concentration en glucagon humain est comprise entre 1 et 2 mg/mL.

Dans un mode de réalisation, le ratio molaire [radical hydrophobe]/[glucagon humain] est inférieur à 20.

Dans un mode de réalisation, le ratio molaire [radical hydrophobe]/[glucagon humain] est inférieur à 15.

Dans un mode de réalisation, le ratio molaire [radical hydrophobe]/[glucagon humain] est inférieur à 10.

Dans un mode de réalisation, le ratio molaire [radical hydrophobe]/[glucagon humain] est inférieur à 5.

Dans un mode de réalisation, le ratio molaire [radical hydrophobe]/[glucagon humain] est inférieur à 2,5.

Dans un mode de réalisation, le ratio molaire [radical hydrophobe]/[glucagon humain] est inférieur à 1,5.

Le glucagon humain est un polypeptide hautement conservé comprenant une chaîne simple de 29 résidus d'acides aminés présentant la séquence suivante H-His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr-OH.

Il peut être obtenu de différentes manières, par synthèse peptidique par recombinaison.

Le glucagon humain est disponible via de nombreuses sources. Par exemple il peut s'agir du glucagon humain produit par Bachem via synthèse peptidique, notamment sous la référence 407473.

Dans un mode de réalisation, la composition comprend en outre un composé nicotinique ou un de ses dérivés.

Dans un mode de réalisation, la composition comprend de la nicotinamide.

Dans un mode de réalisation, la concentration de nicotinamide va de 10 à 160 mM.

Dans un mode de réalisation, la concentration de nicotinamide va de 20 à 150 mM.

Dans un mode de réalisation, la concentration de nicotinamide va de 40 à 120 mM.

Dans un mode de réalisation, la concentration de nicotinamide va de 60 à 100 mM.

Dans un mode de réalisation, la compoosition comprend en outre un composé polyanionique.

Dans un mode de réalisation, le composé polyanionique est choisie dans le groupe constitué des polyacides carboxyliques et leurs sels de Na⁺, K⁺, Ca²⁺ ou Mg²⁺.

Dans un mode de réalisation, le polyacide carboxylique est choisi dans le groupe constitué par l'acide citrique, l'acide tartrique, et leurs sels de Na⁺, K⁺, Ca²⁺ ou Mg²⁺.

Dans un mode de réalisation, le composé polyanionique est choisi dans le groupe constitué des polyacides phosphoriques et leurs sels de Na⁺, K⁺, Ca²⁺ ou Mg²⁺.

Dans un mode de réalisation, le polyacide phosphorique est le triphosphate et ses sels de Na⁺, K⁺, Ca²⁺ ou Mg²⁺.

Dans un mode de réalisation, le composé polyanionique est l'acide citrique et ses sels de Na⁺, K⁺, Ca²⁺ ou Mg²⁺.

Dans un mode de réalisation, le composé polyanionique est l'acide tartrique et ses sels de Na⁺, K⁺, Ca²⁺ ou Mg²⁺.

Dans un mode de réalisation, le composé polyanionique est l'acide triphosphorique et ses sels de Na⁺, K⁺, Ca²⁺ ou Mg²⁺.

Dans un mode de réalisation, la concentration en composé polyanionique est comprise entre 1 et 20 mM.

Dans un mode de réalisation, la concentration en composé polyanionique est comprise entre 2 et 15 mM.

Dans un mode de réalisation, la concentration en composé polyanionique est comprise entre 3 et 12 mM.

Dans un mode de réalisation, la concentration en composé polyanionique est de 10 mM.

Dans un mode de réalisation, la concentration en composé polyanionique est de 5 mM.

Dans un mode de réalisation, la concentration en composé polyanionique est de 10 mM pour des concentrations en glucagon comprises entre 0,5 mg/ml et 3 mg/ml.

Dans un mode de réalisation, la concentration en composé polyanionique est de 10 mM pour des concentrations en glucagon comprises entre 0,5 mg/ml et 2 mg/ml.

Dans un mode de réalisation, la concentration en composé polyanionique est de 10 mM pour des concentrations en glucagon comprises entre 1 mg/ml et 2 mg/ml.

Dans un mode de réalisation, la concentration en composé polyanionique est de 5 mM pour des concentrations en glucagon comprises entre 0,5 mg/ml et 3 mg/ml.

Dans un mode de réalisation, la concentration en composé polyanionique est de 5 mM pour des concentrations en glucagon comprises entre 0,5 mg/ml et 2 mg/ml.

Dans un mode de réalisation, la concentration en composé polyanionique est de 5 mM pour des concentrations en glucagon comprises entre 1 mg/ml et 2 mg/ml.

Dans un mode de réalisation, la concentration en acide citrique et ses sels de Na⁺, K⁺, Ca²⁺ ou Mg²⁺ est comprise entre 1 et 20 mM.

Dans un mode de réalisation, la concentration en acide citrique et ses sels de Na⁺, K⁺, Ca²⁺ ou Mg²⁺ est comprise entre 2 et 15 mM.

Dans un mode de réalisation, la concentration en acide citrique et ses sels de Na⁺, K⁺, Ca²⁺ ou Mg²⁺ est comprise entre 3 et 12 mM.

Dans un mode de réalisation, la concentration en acide citrique et ses sels de Na⁺, K⁺, Ca²⁺ ou Mg²⁺ est de 10 mM.

Dans un mode de réalisation, la concentration en acide citrique et ses sels de Na⁺, K⁺, Ca²⁺ ou Mg²⁺ est de 5 mM.

Dans un mode de réalisation, la concentration en acide citrique et ses sels de Na⁺, K⁺, Ca²⁺ ou Mg²⁺ est de 10 mM pour des concentrations en glucagon comprises entre 0,5 mg/ml et 3 mg/ml.

Dans un mode de réalisation, la concentration en acide citrique et ses sels de Na⁺, K⁺, Ca²⁺ ou Mg²⁺ est de 10 mM pour des concentrations en glucagon comprises entre 0,5 mg/ml et 2 mg/ml.

Dans un mode de réalisation, la concentration en acide citrique et ses sels de Na⁺, K⁺, Ca²⁺ ou Mg²⁺ est de 10 mM pour des concentrations en glucagon comprises entre 1 mg/ml et 2 mg/ml.

Dans un mode de réalisation, la concentration en acide citrique et ses sels de Na⁺, K⁺, Ca²⁺ ou Mg²⁺ est de 5 mM pour des concentrations en glucagon comprises entre 0,5 mg/ml et 3 mg/ml.

Dans un mode de réalisation, la concentration en acide citrique et ses sels de Na⁺, K⁺, Ca²⁺ ou Mg²⁺ est de 5 mM pour des concentrations en glucagon comprises entre 0,5 mg/ml et 2 mg/ml.

Dans un mode de réalisation, la concentration en acide citrique et ses sels de Na⁺, K⁺, Ca²⁺ ou Mg²⁺ est de 5 mM pour des concentrations en glucagon comprises entre 1 mg/ml et 2 mg/ml.

Dans un mode de réalisation, les compositions selon l'invention comprennent en outre une hormone gastrointestinale.

On entend par « hormones gastrointestinales », les hormones choisies dans le groupe constitué par les GLP-1 RA pour agonistes du récepteur Glucagon humain-Like Peptide-1 (Glucagon like peptide-1 receptor agonist)Glucagon like) et le GIP (Glucose-dependent insulinotropic peptide), l'oxyntomoduline (un dérivé du proglucagon humain), le peptide YY, l'amyline, la cholecystokinine, le polypeptide pancréatique (PP), la ghreline et l'entérostatine, leurs analogues ou dérivés et/ou leurs sels pharmaceutiquement acceptables.

Dans un mode de réalisation, les hormones gastro-intestinales sont des analogues ou dérivés de GLP-1 RA (Glucagon like peptide-1 receptor agonist) choisis dans le groupe constitué par l'exenatide ou Byetta® (ASTRA-ZENECA), le liraglutide ou Victoza® (NOVO NORDISK), le lixisenatide ou Lyxumia® (SANOFI), l'albiglutide ou Tanzeum® (GSK) ou le dulaglutide ou Trulicity® (ELI LILLY & CO), leurs analogues ou dérivés et leurs sels pharmaceutiquement acceptables.

Dans un mode de réalisation, l'hormone gastrointestinale est le pramlintide ou Symlin®(ASTRA-ZENECA).

Dans un mode de réalisation, l'hormone gastrointestinale est l'exenatide ou Byetta® ses analogues ou dérivés et leurs sels pharmaceutiquement acceptables.

Dans un mode de réalisation, l'hormone gastrointestinale est le liraglutide ou Victoza® ses analogues ou dérivés et leurs sels pharmaceutiquement acceptables.

Dans un mode de réalisation, l'hormone gastrointestinale est le lixisenatide ou Lyxumia® ses analogues ou dérivés et leurs sels pharmaceutiquement acceptables.

Dans un mode de réalisation, l'hormone gastrointestinale est l'albiglutide ou Tanzeum® ses analogues ou dérivés et leurs sels pharmaceutiquement acceptables.

Dans un mode de réalisation, l'hormone gastrointestinale est le dulaglutide ou Trulicity® ses analogues ou dérivés et leurs sels pharmaceutiquement acceptables.

Dans un mode de réalisation, l'hormone gastrointestinale est le pramlintide ou Symlin®, ses analogues ou dérivés et leurs sels pharmaceutiquement acceptables.

On entend par « analogue », lorsqu'il est utilisé par référence à un peptide ou une protéine, un peptide ou une protéine, dans lequel un ou plusieurs résidus d'acides aminés constitutifs ont été substitués par d'autres résidus d'acides aminés et/ou dans lequel un ou plusieurs résidus d'acides aminés constitutifs ont été supprimés et/ou dans lequel un ou plusieurs résidus d'acides aminés constitutifs ont été ajoutés. Le pourcentage d'homologie admis pour la présente définition d'un analogue est de 50%.

On entend par « dérivé », lorsqu'il est utilisé par référence à un peptide ou une protéine, un peptide ou une protéine ou un analogue chimiquement modifié par un substituant qui n'est pas présent dans le peptide ou la protéine ou l'analogue de référence, c'est-à-dire un peptide ou une protéine qui a été modifié par création de liaisons covalentes, pour introduire des substituants.

Dans un mode de réalisation, le substituant est choisi dans le groupe constitué des chaines grasses.

Dans un mode de réalisation, la concentration en hormone gastrointestinale est comprise dans un intervalle de 0,01 à 10 mg/mL.

Dans un mode de réalisation, la concentration en exenatide, ses analogues ou dérivés et leurs sels pharmaceutiquement acceptables est comprise dans un intervalle de 0,04 à 0,5 mg/mL.

Dans un mode de réalisation, la concentration en liraglutide, ses analogues ou dérivés et leurs sels pharmaceutiquement acceptables est comprise dans un intervalle de 1 à 10 mg/mL.

Dans un mode de réalisation, la concentration en lixisenatide, ses analogues ou dérivés et leurs sels pharmaceutiquement acceptables est comprise dans un intervalle de 0,01 à 1 mg/mL.

Dans un mode de réalisation, la concentration en pramlintide, ses analogues ou dérivés et leurs sels pharmaceutiquement acceptables est comprise entre 0,1 à 5 mg/mL.

Dans un mode de réalisation, les compositions selon l'invention sont réalisées par mélange de solutions de glucagon humain obtenues par reconstitution de lyophilisat et de solutions de GLP-1 RA (Glucagon like peptide-1 receptor agonist) GLP-1 RA, d'analogue ou de dérivé de GLP-1 RA lesdites solutions de GLP-1 RA étant commerciales ou reconstituées à partir de lyophilisat.

Dans un mode de réalisation, les compositions selon l'invention comprennent en outre des tampons.

Dans un mode de réalisation, les compositions selon l'invention comprennent des tampons à des concentrations comprises entre 0 et 100 mM.

Dans un mode de réalisation, les compositions selon l'invention comprennent des tampons à des concentrations comprises entre 15 et 50 mM.

Dans un mode de réalisation, les compositions selon l'invention comprennent un tampon choisi dans le groupe constitué par un tampon phosphate, le Tris (trishydroxyméthylaminométhane) ou le citrate de sodium.

Dans un mode de réalisation, le tampon est le phosphate de sodium.

Dans un mode de réalisation, le tampon est le Tris (trishydroxyméthylaminométhane).

Dans un mode de réalisation, le tampon est le citrate de sodium.

Dans un mode de réalisation, la composition comprend en outre un sel de zinc, en particulier du chlorure de zinc.

Dans un mode de réalisation, la concentration en sel de zinc est comprise entre 50 et 5000 µM.

Dans un mode de réalisation, la concentration en sel de zinc est comprise entre 100 et 2000 µM.

Dans un mode de réalisation, la concentration en sel de zinc est comprise entre 200 et 1500 µM.

Dans un mode de réalisation, la concentration en sel de zinc est comprise entre 200 et 1000 µM.

Dans un mode de réalisation, la concentration en zinc est telle que le ratio molaire [zinc]/[glucagon] est compris entre 0,1 et 2,5.

Dans un mode de réalisation, la concentration en zinc est telle que le ratio molaire [zinc]/[glucagon] est compris entre 0,2 et 2.

Dans un mode de réalisation, la concentration en zinc est telle que le ratio molaire [zinc]/[glucagon] est compris entre 0,5 et 1,5.

Dans un mode de réalisation, la concentration en zinc est telle que le ratio molaire [zinc]/[glucagon] est 1.

Dans un mode de réalisation, les compositions selon l'invention comprennent en outre des conservateurs.

Dans un mode de réalisation, les conservateurs sont choisis dans le groupe constitué par le m-crésol et le phénol seuls ou en mélange.

Dans un mode de réalisation, les compositions selon l'invention comprennent en outre des antioxydants.

Dans un mode de réalisation, les antioxydants sont choisis parmi la méthionine.

Dans un mode de réalisation, la concentration des conservateurs est comprise entre 10 et 50 mM.

Dans un mode de réalisation, la concentration des conservateurs est comprise entre 10 et 40 mM.

Dans un mode de réalisation, les compositions selon l'invention comprennent en outre un tensioactif.

Dans un mode de réalisation, le tensioactif est choisi dans le groupe constitué par le propylène glycol ou le polysorbate.

Les compositions selon l'invention peuvent en outre comprendre des additifs tels que des agents de tonicité.

Dans un mode de réalisation, les agents de tonicité sont choisis dans le groupe constitué par le chlorure de sodium, le mannitol, du sucrose, du sobitol et le glycérol.

Les compositions selon l'invention peuvent comprendre en outre tous les excipients conformes aux pharmacopées et compatibles avec le glucagon humain et les GLP-1 RA utilisés aux concentrations d'usage.

L'invention concerne également une formulation pharmaceutique selon l'invention, caractérisée en ce qu'elle est obtenue par séchage et/ou lyophilisation.

Dans le cas des libérations locale et systémique, les modes d'administration envisagés sont par voie intraveineuse, sous-cutanée, intradermique ou intramusculaire.

Les voies d'administration transdermique, orale, nasale, vaginale, oculaire, buccale, pulmonaire sont également envisagées.

L'invention concerne également des formulations unidoses à pH compris entre 6,6 et 7,8 comprenant du glucagon humain.

L'invention concerne également des formulations unidoses à pH compris entre 6,6 et 7,8 comprenant du glucagon humain et une hormone gastrointestinale, telle que définie précédemment.

Dans un mode de réalisation les formulations unidoses comprennent en outre un co-polyaminoacide substitué tel que défini précédemment.

Dans un mode de réalisation, les formulations sont sous forme d'une solution injectable. Dans un mode de réalisation, le GLP-1 RA, analogue ou dérivé de GLP-1 RA est choisi dans le groupe comprenant exenatide (Byetta®), liraglutide (Victoza®), lixisenatide (Lyxumia®), albiglutide (Tanzeum®), dulaglutide (Trulicity®) ou l'un de leurs dérivés.

Dans un mode de réalisation, l'hormone gastrointestinale est l'exenatide.

Dans un mode de réalisation, l'hormone gastrointestinale est le liraglutide.

Dans un mode de réalisation, l'hormone gastrointestinale est le lixisenatide.

Dans un mode de réalisation, l'hormone gastrointestinale est l'albiglutide.

Dans un mode de réalisation, l'hormone gastrointestinale est le dulaglutide.

Par ailleurs et de façon toute aussi importante, la demanderesse a pu vérifier que le glucagon humain en présence d'un co-polyaminoacide porteur de charges carboxylates et d'au moins un radical hydrophobe selon l'invention conserve son action que ce soit seul ou en combinaison avec une hormone gastrointestinale.

La préparation d'une composition selon l'invention présente l'avantage de pouvoir être réalisée par simple mélange d'une solution de glucagon humain, d'une solution de GLP-1 RA, d'un analogue ou un dérivé de GLP-1 RA, et d'un co-polyaminoacide porteurs de charges carboxylates et d'au moins un radical hydrophobe selon l'invention, en solution aqueuse ou sous forme lyophilisée. Si nécessaire, le pH de la préparation est ajusté à pH 7.

Dans un mode de réalisation le mélange de glucagon humain et de co-polyaminoacide substitué est concentré par ultrafiltration avant le mélange avec de GLP-1 RA, d'un analogue ou un dérivé de GLP-1 RA en solution aqueuse ou sous forme lyophilisée.

Si nécessaire, la composition du mélange est ajustée en excipients tels que glycérol, m-crésol, et polysorbate (Tween®) par ajout de solutions concentrées de ces excipients au sein du mélange. Si nécessaire, le pH de la préparation est ajusté à 7.

### Partie A

### AA : Synthèse des molécules hydrophobes dans lesquelles p = 1

Les radicaux hydrophobes sont représentés dans le tableau suivant par la molécule hydrophobe correspondante avant greffage sur le co-polyaminoacide.

**Tableau 1A : liste et structures des molécules hydrophobes synthétisées selon l'invention.**

| **N°** | **Structure de la molécule hydrophobe avant greffage sur le co-polyaminoacide** |
|---|---|
| AA1 | 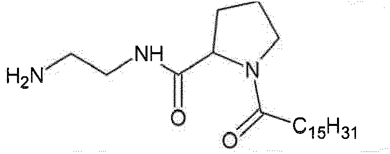 |
| AA2 | 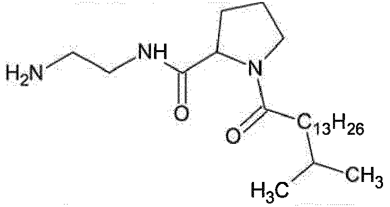 |
| AA3 | 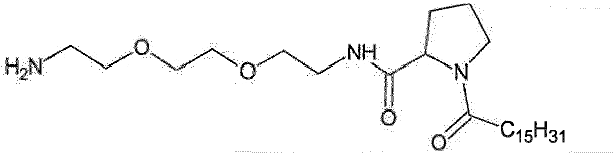 |
| AA4 | 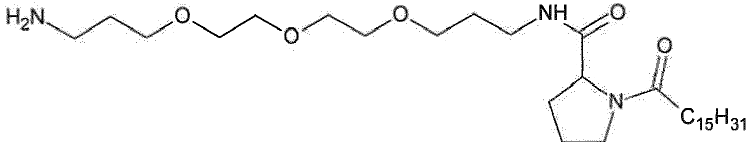 |
| AA5 | 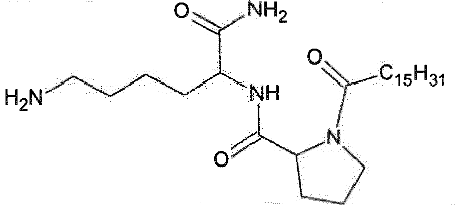 |
| AA6 | 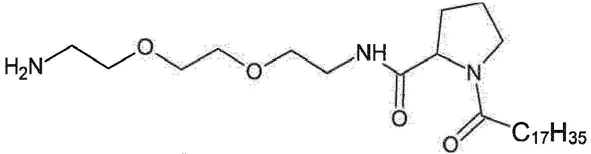 |
| AA7 | 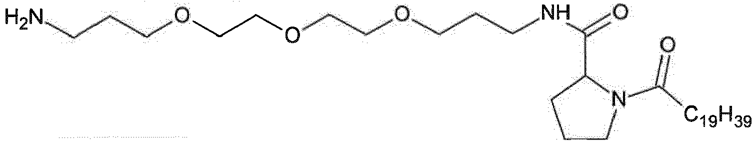 |
| AA8 | 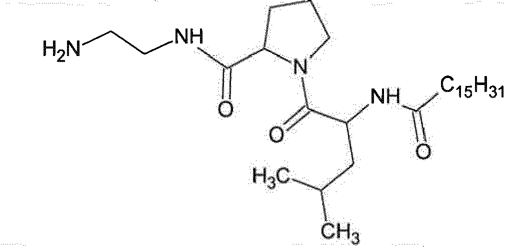 |
| AA9 | 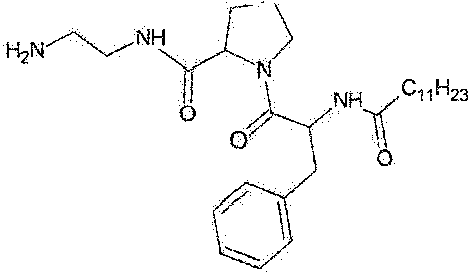 |
| AA10 | 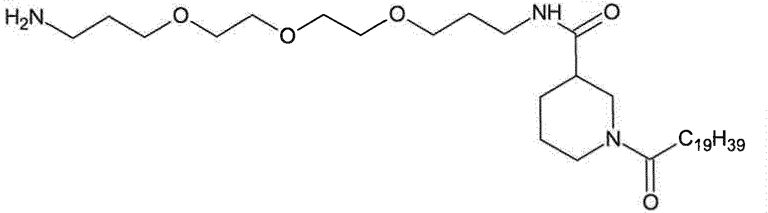 |
| AA12 | 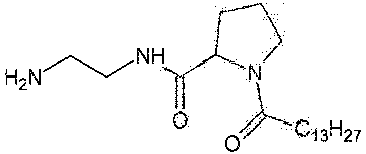 |
| AA14 | 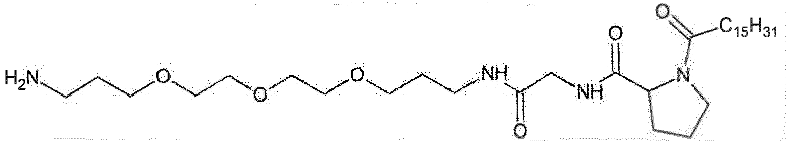 |

### Exemple AA1 : molécule AA1

### Molécule A1 : produit obtenu par la réaction entre le chlorure de palmitoyle et la L-proline.

A une solution de L-proline (10,6 g, 92,1 mmol) dans de la soude aqueuse 1 N (230 mL ; 230 mmol) est ajoutée goutte à goutte pendant 90 minutes une solution de chlorure de palmitoyle (23,0 g, 83,7 mmol) dans l'acétone (167 mL). Après 14 h d'agitation à température ambiante, le mélange hétérogène est refroidi à 0°C, puis filtré sur fritté pour donner un solide blanc qui est lavé avec de l'eau (2 x 100 mL) puis du diisopropyléther (100 mL). Le solide est séché sous pression réduite. Le solide est alors dissous à reflux dans 200 mL d'eau puis 8 mL d'une solution d'acide chlorhydrique à 37% sont ajoutés pour obtenir un pH = 1. Le milieu réactionnel opalescent est alors refroidi à 0°C. Le précipité obtenu est filtré sur fritté puis lavé avec de l'eau (5 x 50 mL) jusqu'à obtenir des filtrats de pH physiologique compris entre 6,0 et 8,0 pour être ensuite séché dans une étuve à 50°C sous vide pendant une nuit. Le produit est purifié par recristallisation dans le diisopropyléther. Un solide blanc est obtenu.
Rendement : 22,7 g (77%).
RMN ¹H (CDCl₃, ppm) : 0,88 (3H) ; 1,19-1,45 (24H) ; 1,58-1,74 (2H) ; 1,88-2,14 (3H) ; 2,15-2,54 (3H) ; 3,47 (1H) ; 3,58 (1H) ; 4,41 (0,1H) ; 4,61 (0,9H) 6,60-8,60 (1H).

### Molécule A2 : produit obtenu par réaction entre la molécule A1 et la Boc-ethylènediamine.

A une solution de molécule A1 (75,1 g, 212,4 mmol) dans 1500 mL de chloroforme sont ajoutés successivement et à température ambiante de la *N,N-*diisopropyléthylamine (DIPEA) (68,8 g, 532,3 mmol), du 1-hydroxybenzotriazole (HOBt) (37,1 g, 274,6 mmol) puis du *N*-(3-dimethylaminopropyl)-*N'*-ethylcarbodiimide (EDC) (53,1 g, 277,0 mmol). Après 15 minutes d'agitation à température ambiante, une solution de Boc-ethylènediamine (Boc-éthylènediamine) (37,6 g, 234,7 mmol) dans 35 mL de chloroforme est additionnée. Après 18 h d'agitation à température ambiante, une solution de HCl 0,1 N (2,1 L), puis une solution saturée de NaCl (1 L) sont ajoutées. Les phases sont séparées puis la phase organique est lavée successivement avec une solution de HCl 0,1 N / NaCl saturée (2,1 L/1 L), une solution de NaCl saturée (2 L), une solution de NaHCO₃ saturée (2 L), puis une solution de NaCl saturée (2 L). La phase organique est séchée sur sulfate de sodium anhydre, filtrée puis concentrée sous pression réduite. Le solide obtenu est purifié par triturations dans le diisopropyléther (3 x 400 mL), pour donner un solide après séchage sous vide à 40°C.
Rendement : 90,4 g (86%).
RMN ¹H (CDCl₃, ppm) : 0,88 (3H) ; 1,20-1,37 (24H) ; 1,44 (9H) ; 1,54-1,70 (2H) ; 1,79-1,92 (1H) ; 1,92-2,04 (1H) ; 2,03-2,17 (1H) ; 2,17-2,44 (3H) ; 3,14-3,36 (4H) ; 3,43 (1H) ; 3,56 (1H) ; 4,29 (0,1 H) ; 4,51 (0,9 H) ; 4,82 (0,1H) ; 5,02 (0,9H) ; 6,84 (0,1H) ; 7,22 (0,9H).

### Molécule AA1

A une solution de molécule A2 (20,1 g, 40,5 mmol) dans 330 mL de dichlorométhane est ajoutée goutte à goutte et à 0°C une solution d'acide chlorhydrique 4 M dans le dioxane (100 mL, 400 mmol). Après 3h30 d'agitation à température ambiante, la solution est concentrée sous pression réduite. Le résidu est purifié par chromatographie flash (méthanol, dichlorométhane) pour donner un solide blanc de molécule AA1 sous forme de sel de chlorhydrate.
Rendement : 16,3 g (93%).
RMN ¹H (CDCl₃, ppm) : 0,88 (3H) ; 1,07-1,40 (24H) ; 1,49-1,63 (2H) ; 1,77-2,18 (4H) ; 2,18-2,45 (2H) ; 3,14-3,32 (2H) ; 3,42-3,63 (2H) ; 3,63-3,84 (2H) ; 4,37 (0,1H) ; 4,48 (0,9H) ; 6,81-8,81 (4H).
LC/MS (ESI) : 396,5 ; (calculé ([M+H]⁺) : 396,4).

### Exemple AA2 : molécule AA2

### Molécule A3 : 15-méthylhexadécan-1-ol.

Dans un tricol sous argon est introduit du magnésium (9,46 g, 389 mmol) en copeaux. Le magnésium est recouvert de THF (40 mL) anhydre et quelques gouttes de 1-bromo-3-méthylbutane sont ajoutées à température ambiante pour initier la réaction. Après l'observation d'un exotherme et un léger trouble du milieu, le reste du 1-bromo-3-méthylbutane (53,87 g, 357 mmol) est ajouté au goutte à goutte en 90 min alors que la température du milieu reste stable entre 50 et 60°C. Le milieu réactionnel est ensuite chauffé à 70°C pendant 2 h.

Dans un tricol sous argon, à une solution de CuCl (482 mg, 4,86 mmol) dissous dans la NMP (62 mL) à 0°C est ajoutée au goutte à goutte une solution de 12-bromo-1-dodécanol (43 g, 162,1 mmol) dans le THF (60 mL). A cette solution est ensuite ajoutée au goutte à goutte la solution de l'organomagnésien chaude fraîchement préparée de façon à maintenir la température du milieu en dessous de 20°C. Le mélange est ensuite agité à température ambiante pendant 16 h. Le milieu est refroidi à 0°C et la réaction est stoppée par addition d'une solution aqueuse d'HCl 1N jusqu'à pH 1 et le milieu est extrait à l'acétate d'éthyle. Après lavage de la phase organique avec une solution saturée en NaCl et séchage sur Na₂SO₄, la solution est filtrée et concentrée sous vide pour donner une huile. Après purification par DCVC sur gel de silice (cyclohexane, acétate d'éthyle), une huile qui cristallise à température ambiante est obtenue.
Rendement : 32,8 g (74%)
RMN ¹H (CDCl₃, ppm) : 0,87 (6H) ; 1,14 (2H) ; 1,20-1,35 (22H) ; 1,50-1,55 (3H) ; 3,64 (2H).

### Molécule A4 : acide 15-méthylhexadécanoïque.

A une solution de molécule A3 (20,65 g, 80,5 mmol) et bromure de tétrabutylammonium (14,02 g, 42,5 mmol) dans un mélange d'acide acétique/dichloréthane/eau (124/400/320 mL) à température ambiante est ajouté par petites portions du permanganate de potassium (38,2 g, 241,5 mmol). Après agitation à reflux pendant 5 h et retour à température ambiante, le milieu est acidifié à pH 1 par ajout progressif de HCl 5N. Na₂SO₃ (44,6 g, 354,3 mmol) est ensuite ajouté progressivement jusqu'à décoloration du milieu. La phase aqueuse est extraite au dichlorométhane et les phases organiques combinées sont séchées sur Na₂SO₄, filtrées et concentrées sous vide. Après purification par chromatographie sur gel de silice (cyclohexane, acétate d'éthyle, acide acétique), un solide blanc est obtenu.
Rendement : 19,1 g (quantitatif)
RMN ¹H (CDCl₃, ppm) : 0,87 (6H) ; 1,14 (2H) ; 1,22-1,38 (20H) ; 1,51 (1H) ; 1,63 (2H) ; 2,35 (2H).

### Molécule A5 : produit obtenu par réaction entre la molécule A4 et la L-proline.

A une solution de molécule A4 (10 g, 37 mmol) dans le THF (360 mL) à 0°C sont ajoutés successivement du dicyclohexyle carbodiimide (DCC) (8,01 g, 38,8 mmol) et du *N*-hydroxysuccinimide (NHS) (4,47 g, 38,8 mmol). Après 17 h d'agitation à température ambiante, le milieu est refroidi à 0°C pendant 20 min, filtré sur fritté. De la L-proline (4 g, 37,7 mmol), de la triéthylamine (34 mL) et de l'eau (30 mL) sont ajoutées au filtrat. Après 20 h d'agitation à température ambiante, le milieu est traité avec une solution aqueuse d'HCl 1N jusqu'à pH 1. La phase aqueuse est extraite avec du dichlorométhane (2 x 125 mL). Les phases organiques combinées sont lavées avec une solution aqueuse de HCl 1N (2 x 100 mL), de l'eau (100 mL), puis une solution aqueuse saturée en NaCl (100 mL). Après séchage sur Na₂SO₄, la phase organique est filtrée, concentrée sous vide et le résidu est purifié par chromatographie sur gel de silice (cyclohexane, acétate d'éthyle, acide acétique)
Rendement : 9,2 g (72%)
RMN ¹H (CDCl₃, ppm) : 0,86 (6H) ; 1,14 (2H) ; 1,22-1,38 (20H) ; 1,50 (1H) ; 1,67 (2H) ; 1,95-2,10 (3H) ; 2,34 (2H) ; 2,49 (1H) ; 3,47 (1H) ; 3,56 (1H) ; 4,61 (1H). LC/MS (ESI) : 368,3 ; (calculé ([M+H]⁺) : 368,6).

### Molécule A6 : produit obtenu par réaction entre la molécule A5 et la Boc-éthylènediamine.

A une solution de molécule A5 (9,22 g, 25,08 mmol) dans un mélange THF/DMF (200/50 mL) sont ajoutés de la triéthylamine (TEA) (5,23 mL) et du 2-(1H-benzotriazol-1-yl)-1,1,3,3-tétraméthyluronium tétrafluoroborate (TBTU) à température ambiante. Après 10 min d'agitation, de la Boc-éthylènediamine (4,42 g, 27,6 mmol) est ajoutée. Après agitation à température ambiante pendant 17 h, le mélange est dilué avec de l'eau (300 mL) à 0°C et agité à froid pendant 20 min. Le précipité formé est filtré sur fritté et le filtrat est extrait à l'acétate d'éthyle. Les phases organiques combinées sont lavées avec une solution saturée de NaHCO₃, séchées sur Na₂SO₄, filtrées, concentrées sous vide et le résidu est purifié par chromatographie flash (acétate d'éthyle, méthanol).
Rendement : 6,9 g (54%)
RMN ¹H (CDCl₃, ppm) : 0,86 (6H) ; 1,15 (2H) ; 1,22-1,38 (20H) ; 1,43 (9H) ; 1,50 (1H) ; 1,64 (4H) ; 1,85 (1H) ; 1,95 (1H) ; 2,10 (1H) ; 2,31 (2H) ; 3,20-3,35 (3H) ; 3,45 (1H) ; 3,56 (1H) ; 4,51 (1H) ; 5,05 (1H) ; 7,24 (1H).
LC/MS (ESI) : 510,6 ; (calculé ([M+H]⁺) : 510,8).

### Molécule AA2

A une solution de la molécule A6 (5,3 g, 10,40 mmol) dans le dichlorométhane (50 mL) à 0°C est ajoutée une solution de HCl 4N dans le dioxane (13 mL). Après 5 h d'agitation à 0°C, le milieu est concentré sous vide, repris dans de l'eau et lyophilisé pour donner un solide blanc de molécule AA2 sous forme de sel de chlorhydrate.
Rendement : 4,6 g (99%)
RMN ¹H (D₂O, ppm) : 0,91 (6H) ; 1,22 (2H) ; 1,22-1,50 (20H) ; 1,63 (3H) ; 1,98 (1H) ; 2,10 (2H) ; 2,26 (1H) ; 2,39 (1H) ; 2,43 (1H) ; 3,22 (2H) ; 3,45-3,60 (3H) ; 3,78 (1H) ; 4,42 (1H).
LC/MS (ESI) : 410,4 ; (calculé ([M+H]⁺) : 410,7).

### Exemple AA3 : molécule AA3

### Molécule A7 : produit obtenu par la réaction entre la molécule A1 et la Boctri(éthylèneglycol)diamine.

Par un procédé similaire à celui utilisé pour la préparation de la molécule A2 appliqué à la molécule A1 (4,0 g, 11,3 mmol) et à la Boc-tri(éthylèneglycol)diamine (3,1 g, 12,4 mmol), une huile incolore est obtenue après purification par chromatographie flash (méthanol, toluène).
Rendement : 5,5 g (84%).
RMN ¹H (CDCl₃, ppm) : 0,88 (3H) ; 1,09-1,39 (24H) ; 1,44 (9H) ; 1,64 (2H) ; 1,79-2,01 (2H) ; 2,06-2,43 (4H) ; 3,23-3,68 (14H) ; 4,33 (0,2H) ; 4,56 (0,8H) ; 5,25 (1H) ; 6,49 (0,2H) ; 7,13-7,50 (0,8H).

### Molécule AA3

Par un procédé similaire à celui utilisé pour la préparation de la molécule AA1 appliqué à la molécule A7 (5,5 g, 9,4 mmol), un solide blanc de molécule AA3 sous forme de sel de chlorhydrate est obtenu après purification par chromatographie flash (méthanol, dichlorométhane).
Rendement : 4,3 g (92%).
RMN ¹H (DMSO-d6, ppm) : 0,85 (3H) ; 1,08-1,40 (24H) ; 1,40-1,52 (2H) ; 1,71-2,02 (4H) ; 2,02-2,31 (2H) ; 2,90-2,98 (2H) ; 3,15-3,47 (5H) ; 3,50-3,66 (7H) ; 4,24 (0,6H) ; 4,32 (0,4H) ; 7,83 (0,6H) ; 7,95 (3H) ; 8,17 (0,4H).
LC/MS (ESI) : 484,6 ; (calculé ([M+H]⁺) : 484,4).

### Exemple AA4 : molécule AA4

### Molécule A8: produit obtenu par la réaction entre la molécule A1 et la Boc-1-amino-4,7,10-trioxa-13-tridécane amine.

Par un procédé similaire à celui utilisé pour la préparation de la molécule A2 appliqué à la molécule A1 (4,5 g, 12,7 mmol) et à la Boc-1-amino-4,7,10-trioxa-13-tridécane amine (4,5 g, 14,0 mmol), une huile jaune est obtenue après purification par chromatographie flash (méthanol, dichlorométhane).
Rendement : 7,7 g (92%).
RMN ¹H (CDCl₃, ppm) : 0,88 (3H) ; 1,22-1,37 (24H) ; 1,44 (9H) ; 1,59-1,67 (2H) ; 1,67-2,00 (6H) ; 2,06-2,45 (4H) ; 3,18-3,76 (18H) ; 4,28 (0,2H) ; 4,52 (0,8H) ; 4,69-5,04 (1H) ; 6,77 (0,2H) ; 7,20 (0,8H).

### Molécule AA4

Par un procédé similaire à celui utilisé pour la préparation de la molécule AA1 appliqué à la molécule A8 (7,7 g, 11,8 mmol), une huile jaune est obtenue après purification par chromatographie flash (méthanol, dichlorométhane). Une coévaporation avec du diisopropyléther permet d'obtenir la molécule AA4 sous forme de sel de chlorhydrate sous forme d'un solide blanc qui est séché sous vide à 50°C.
Rendement : 5,4 g (76%).
RMN ¹H (CDCl₃, ppm) : 0,88 (3H) ; 1,08-1,40 (24H) ; 1,49-1,65 (2H) ; 1,76-2,39 (10H) ; 3,07-3,28 (3H) ; 3,34-3,80 (15H) ; 4,34 (0,05H) ; 4,64 (0,95H) ; 7,35 (0,05H) ; 7,66-8,58 (3,95H).
LC/MS (ESI) : 556,7 ; (calculé ([M+H]⁺) : 556,5).

### Exemple AA5 : molécule AA5

Molécule A9 : produit obtenu par réaction entre la molécule A1 et l'ester méthylique de la *N*-Boc-L-lysine.

Par un procédé similaire à celui utilisé pour la préparation de la molécule A2 appliqué à la molécule A1 (4 g, 11,3 mmol) et à l'ester méthylique de la N-Boc-L-lysine (3,2 g, 12,4 mmol), une huile incolore est obtenue après purification par chromatographie flash (méthanol, dichlorométhane).
Rendement : 4,9 g (73%).
RMN ¹H (CDCl₃, ppm) : 0,88 (3H) ; 0,99-1,54 (37H) ; 1,54-1,75 (3H) ; 1,75-2,04 (3H) ; 2,04-2,41 (4H) ; 2,94-3,19 (2H) ; 3,19-3,81 (5H) ; 4,28-4,64 (2H) ; 4,94 (1H) ; 6,45 (0,1H) ; 7,36 (0,9H).
LC/MS (ESI) : 596,7 ; (calculé ([M+H]⁺) : 596,5).

### Molécule A10 : produit obtenu par traitement de la molécule A9 avec de l'ammoniaque.

A une suspension de molécule A9 (4,9 g, 8,2 mmol) dans 10 mL de méthanol sont ajoutés 320 mL d'une solution d'ammoniaque 7 N dans le méthanol. Après 19 h d'agitation à température ambiante en atmosphère fermée, 100 mL supplémentaires de solution d'ammoniaque sont ajoutés. Après 24 h d'agitation à température ambiante en atmosphère fermée, le milieu réactionnel est concentré sous pression réduite. Le résidu est purifié par trituration dans le diisopropyléther à reflux (100 mL), pour donner un solide blanc qui est séché sous vide à 50°C.
Rendement : 4,1 g (85%).
RMN ¹H (CDCl₃, ppm) : 0,88 (3H) ; 1,06-1,57 (37H) ; 1,57-1,79 (3H) ; 1,88-2,41 (7H) ; 3,09 (2H) ; 3,49 (1H) ; 3,62 (1H) ; 4,34 (1H) ; 4,51 (1H) ; 4,69-4,81 (1H) ; 5,43 (0,95H) ; 5,57 (0,05H) ; 6,25 (0,05H) ; 6,52 (0,95H) ; 6,83 (0,05H) ; 7,11 (0,95H).

### Molécule AA5

Par un procédé similaire à celui utilisé pour la préparation de la molécule AA1 appliqué à la molécule A10 (388 mg, 0,67 mmol), un solide blanc de molécule AA5 sous forme de sel de chlorhydrate est obtenu après purification par trituration dans le diisopropyléther.
Rendement : 292 mg (85%).
RMN ¹H (DMSO-d6, ppm) : 0,85 (3H) ; 1,06-2,34 (38H) ; 2,61-2,81 (2H) ; 3,29-3,68 (2H) ; 4,05-4,17 (1,7H) ; 4,42 (0,3H) ; 7,00 (1H) ; 7,16 (0,7H) ; 7,43 (0,3H) ; 7,73-8,04 (3,7H) ; 8,16 (0,3H).
LC/MS (ESI): 481,6 ; (calculé ([M+H]⁺) : 481,4).

### Exemple AA6 : molécule AA6

### Molécule A11 : produit obtenu par la réaction entre le chlorure de stéaroyle et la L-proline.

Par un procédé similaire à celui utilisé pour la préparation de la molécule A1 appliqué à la L-proline (5,0 g, 43,4 mmol) et au chlorure de stéaroyle (12,0 g, 39,6 mmol), un solide blanc est obtenu après purification par chromatographie flash (méthanol, dichlorométhane).
Rendement : 5,37 g (36%)
RMN ¹H (CDCl₃, ppm) : 0,88 (3H) ; 1,26-1,37 (28H) ; 1,64-1,70 (2H) ; 1,88-2,10 (3H) ; 2,36 (2H) ; 2,54-2,58 (1H) ; 3,46 (1H) ; 3,56 (1H) ; 4,62 (1H).
LC/MS (ESI) : 382,6 ; (calculé ([M+H]⁺) : 382,3).

### Molécule A12 : produit obtenu par réaction entre la molécule A11 et la Boctri(éthylèneglycol)diamine.

Par un procédé similaire à celui utilisé pour la préparation de la molécule A6 appliqué à la molécule A11 (33,81 g, 88,6 mmol) et à la Boctri(éthylèneglycol)diamine (26,4 g, 106,3 mmol) dans le THF en utilisant la DIPEA au lieu de la TEA, un solide blanc est obtenu après purification par chromatographie flash (acétate d'éthyle, méthanol).
Rendement : 43,3 g (80%)
RMN ¹H (CDCl₃, ppm) : 0,87 (3H) ; 1,24 (30H) ; 1,43 (9H) ; 1,61 (2H) ; 1,82 (1H) ; 1,96 (1H) ; 2,25-2,45 (2H) ; 3,25-3,65 (14H) ; 4,30 (0,15H) ; 4,53 (0,85H) ; 5,25 (1H) ; 6,43 (0,15H) ; 7,25 (0,85H).
LC/MS (ESI) : 612,6 ; (calculé ([M+H]⁺): 612,9).

### Molécule AA6

Par un procédé similaire à celui utilisé pour la préparation de la molécule AA2 appliqué à la molécule A12 (43 g, 70,3 mmol), le résidu obtenu après concentration sous vide est trituré dans l'acétonitrile. La suspension est filtrée et le solide lavé avec de l'acétonitrile puis de l'acétone. Après séchage sous vide, un solide blanc de molécule AA6 sous forme de sel de chlorhydrate est obtenu.
Rendement : 31,2 g (81%)
RMN ¹H (DMSO-d₆, ppm) : 0,85 (3H) ; 1,23 (28H) ; 1,45 (2H) ; 1,70-2,05 (4H) ; 2,13 (1H) ; 2,24 (1H) ; 2,95 (2H) ; 3,10-3,25 (2H) ; 3,30-3,65 (10H) ; 4,20-4,45 (1H) ; 7,85-8,25 (4H).
LC/MS (ESI) : 512,4 ; (calculé ([M+H]⁺) : 512,8).

### Exemple AA7 : molécule AA7

### Molécule A13 : produit obtenu par réaction entre l'acide arachidique et la L-proline.

Par un procédé similaire à celui utilisé pour la préparation de la molécule A5 appliqué à l'acide arachidique (15,51 g, 49,63 mmol) et à la L-proline (6 g, 52,11 mmol) en utilisant la DIPEA à la place de la TEA, un solide blanc est obtenu après purification par colonne chromatographique sur gel de silice (cyclohexane, acétate d'éthyle, acide acétique).
Rendement : 12,9 g (63%)
RMN ¹H (CDCl₃, ppm) : 0,88 (3H) ; 1,28 (34H) ; 1,66 (2H) ; 1,95-2,15 (2H) ; 2,34 (2H) ; 2,45 (1H) ; 3,47 (1H) ; 3,56 (1H) ; 4,60 (1H).
LC/MS (ESI) : 410,4 ; (calculé ([M+H]⁺) : 410,6).

### Molécule A14 : produit obtenu par la réaction entre la molécule A13 et la Boc-1-amino-4,7,10-trioxa-13-tridécane.

Par un procédé similaire à celui utilisé pour la préparation de la molécule A12 appliqué à la molécule A13 (10,96 g, 26,75 mmol) et à la Boc-1-amino-4,7,10-trioxa-13-tridécane (10,29 g, 32,11 mmol), un solide est obtenu après purification par colonne chromatographique sur gel de silice (cyclohexane, acétate d'éthyle, méthanol).
Rendement : 14,2 g (75%)
RMN ¹H (CDCl₃, ppm) : 0,88 (3H) ; 1,24 (32H) ; 1,43 (9H) ; 1,57-2,00 (8H); 2,10-2,45 (4H) ; 3,20-3,75 (18H) ; 4,30 (0,20H) ; 4,55 (0,80H) ; 5,03 (1H) ; 6,75 (0,20H) ; 7,20 (0,80H).
LC/MS (ESI) : 712,8 ; (calculé ([M+H]⁺) : 713,1).

### Molécule AA7

Après un procédé similaire à celui utilisé pour la préparation de la molécule AA2 appliqué à la molécule A14 (14,25 g, 20,01 mmol), le résidu obtenu après concentration sous vide du milieu réactionnel est dissous dans le méthanol et évaporé sous pression réduite, l'opération étant répétée 4 fois pour donner un solide blanc de molécule AA7 sous forme de sel de chlorhydrate.
Rendement : 12,7 g (98%)
RMN ¹H (DMSO-d₆, ppm) : 0,85 (3H) ; 1,23 (32H) ; 1,45 (2H) ; 1,64 (2H) ; 1,70-2,05 (6H) ; 2,10-2,30 (2H) ; 2,82 (2H) ; 3,08 (2H) ; 3,30-3,60 (14H) ; 4,15-4,30 (1H) ; 7,73-8,13 (4H).
LC/MS (ESI) : 612,7 ; (calculé ([M+H]⁺) : 612,9).

### Exemple AA8 : molécule AA8

### Molécule A15 : produit obtenu par la réaction entre la L-leucine et le chlorure de palmitoyle.

Par un procédé similaire à celui utilisé pour la préparation de la molécule A1 appliqué à de la L-leucine (15,0 g, 114,4 mmol) et au chlorure de palmitoyle (34,5 g, 125 mmol), un solide blanc est obtenu par trituration dans le diisopropyléther.
Rendement : 13,0 g (31%)
RMN ¹H (CDCl₃, ppm) : 0,88 (3H) ; 0,96 (6H) ; 1,16-1,35 (24H) ; 1,55-1,77 (5H) ; 2,23 (2H) ; 4,55-4,60 (1H) ; 5,88 (1H).

### Molécule A16 : produit obtenu par la réaction entre la molécule A15 et l'ester méthylique de la L-proline

Par un procédé similaire à celui utilisé pour la préparation de la molécule A2 appliqué à la molécule A15 (6,00 g, 16,2 mmol) et à l'ester méthylique de la L-proline (3,23 g, 19,5 mmol), une huile légèrement jaune est obtenue après purification par chromatographie flash (méthanol, dichlorométhane).
Rendement : 5,8 g (74%)
RMN ¹H (CDCl₃, ppm) : 0,83-1,00 (9H) ; 1,18-1,32 (24H) ; 1,40-1,73 (5H) ; 1,84-2,33 (6H) ; 3,47-3,89 (2H) ; 3,70 (1,14H) ; 3,71 (1,21H) ; 3,74 (0,53H) ; 3,76 (0,12H) ; 4,40-4,56 (1H) ; 4,63-4,67 (0,04H) ; 4,84 (0,38) ; 4,90 (0,40) ; 5,06 (0,18) ; 5,99 (0,18H) ; 6,08-6,21 (0,82).
LC/MS (ESI) : 481,6 ; (calculé ([M+H]⁺) : 481,4).

### Molécule A17 : produit obtenu par la saponification de l'ester méthylique de la molécule A16.

A une solution de molécule A16 (5,8 g, 12,06 mmol) dans 30 mL de méthanol est ajoutée de la soude 1 N (13,5 mL, 13,5 mmol). Après 20 h d'agitation à température ambiante, la solution est diluée avec de l'eau puis acidifiée par 20 mL d'acide chlorhydrique 1 N à 0°C. Le précipité est filtré puis rincé avec de l'eau (50 mL) avant d'être solubilisé dans 50 mL de dichlorométhane. La phase organique est séchée sur Na₂SO₄, filtrée puis concentrée sous pression réduite pour donner une huile incolore.
Rendement : 4,5 g (80%)
RMN ¹H (CDCl₃, ppm) : 0,85-0,99 (9H) ; 1,14-1,41 (24H) ; 1,43-1,72 (5H) ; 1,87-2,47 (7H) ; 3,48-3,55 (0,6H) ; 3,56-3,62 (0,4H) ; 3,83-3,90 (0,4H) ; 3,90-3,96 (0,6H) ; 4,52-4,56 (0,6H) ; 4,56-4,59 (0,4H) ; 4,80-4,86 (0,4H) ; 4,86-4,91 (0,6H) ; 6,05 (0,4H) ; 6,11 (0,6H).
LC/MS (ESI) : 467,6 ; (calculé ([M+H]⁺) : 467,4).

### Molécule A18 : produit obtenu par la réaction entre la Boc-éthylènediamine et la molécule A17.

Par un procédé similaire à celui utilisé pour la préparation de la molécule A2 appliqué à la molécule A17 (4,5 g, 9,64 mmol) et à la Boc-éthylènediamine (1,70 g, 10,61 mmol), une huile incolore est obtenue après purification par chromatographie flash (méthanol, dichlorométhane).
Rendement : 2,0 g (34%)
RMN ¹H (CDCl₃, ppm) : 0,83-0,99 (9H) ; 1,19-1,32 (24H) ; 1,44 (9H) ; 1,48-2,37 (14H) ; 3,09-3,99 (4H) ; 4,28-5,01 (2H) ; 5,64-6,04 (1H) ; 6,87-7,06 (1H).
LC/MS (ESI) : 609,7 ; (calculé ([M+H]⁺) : 609,5).

### Molécule AA8

Par un procédé similaire à celui utilisé pour la préparation de la molécule AA1 appliqué à la molécule A18 (2 g, 3,28 mmol), un solide de molécule AA8 sous forme de sel de chlorhydrate est obtenu après purification par chromatographie flash (méthanol, dichlorométhane).
Rendement : 1,5 g (90%)
RMN ¹H (CDCl₃, ppm) : 0,83-1,00 (9H) ; 1,18-1,32 (24H) ; 1,37-1,77 (5H) ; 1,93-2,41 (6H) ; 3,07-3,97 (6H) ; 4,44-4,77 (2H) ; 7,66-8,21 (2H).
LC/MS (ESI) : 509,6 ; (calculé ([M+H]⁺) : 509,4).

### Exemple AA9 : molécule AA9

### Molécule A19 : produit obtenu par la réaction entre l'acide laurique et la L-phénylalanine.

Par un procédé similaire à celui utilisé pour la préparation de la molécule A5 appliqué à l'acide laurique (8,10 g, 40,45 mmol) et à la L-phénylalanine (7 g, 42,38 mmol), un solide blanc est obtenu.
Rendement : 12,7 g (98%)
RMN ¹H (DMSO-d₆, ppm) : 0,86 (3H) ; 1,10-1,30 (16H) ; 1,36 (2H) ; 2,02 (2H) ; 2,82 (1H) ; 3,05 (1H) ; 4,42 (1H) ; 7,15-7,30 (5H) ; 8,05 (1H) ; 12,61 (1H).
LC/MS (ESI) : 348,2 ; (calculé ([M+H]⁺) : 348,5).

### Molécule A20 : produit obtenu par la réaction entre la molécule A19 et le sel chlorhydrate de l'ester méthylique de la L-proline.

Par un procédé similaire à celui utilisé pour la préparation de la molécule A6 appliqué à la molécule A19 (9,98 g, 28,72 mmol) et au sel chlorhydrate de l'ester méthylique de la L-proline (5,23 g, 31,59 mmol), une huile incolore est obtenue après purification par colonne chromatographique sur gel de silice (cyclohexane, acétate d'éthyle).
Rendement : 5,75 g (44%)
RMN ¹H (CDCl₃, ppm) : 0,88 (3H) ; 1,10-1,30 (16H) ; 1,50-1,75 (3H) ; 1,80-2,02 (3H) ; 2,17 (2H) ; 2,65 (0,5H) ; 2,95 (1H) ; 3,05-3,20 (1,5H) ; 3,50-3,65 (1H) ; 3,75 (3H) ; 4,29 (0,5H) ; 4,46 (0,5H) ; 4,70 (0,1H) ; 4,95 (0,9H) ; 6,20-6,30 (1H) ; 7,15-7,30 (5H).
LC/MS (ESI) : 459,2 ; (calculé ([M+H]⁺) : 459,6).

### Molécule A21 : produit obtenu par saponification de la molécule A20.

A une solution de molécule A20 (5,75 g, 12,54 mmol) dans un mélange THF/méthanol/eau (40/40/40 mL) à 0°C est ajoutée de l'hydroxyde de lithium (LiOH) (600,49 mg, 25,07 mmol) puis le mélange est agité à température ambiante pendant 20 h. Après évaporation des solvants organiques sous vide, la phase aqueuse est diluée dans l'eau, acidifiée avec une solution aqueuse de HCl 1 N jusqu'à pH 1. Le produit est alors extrait à l'acétate d'éthyle. Les phases organiques combinées sont lavées avec une solution aqueuse saturée de NaCl, séchées sur Na₂SO₄, filtrées et concentrées sous pression réduite pour donner une huile incolore.
Rendement : 5,7 g (quantitatif)
RMN ¹H (CDCl₃, ppm) : 0,88 (3H) ; 1,10-1,30 (16H) ; 1,50-1,80 (3H) ; 1,67-2,02 (2H) ; 2,20 (2H) ; 2,25 (0,4H) ; 2,60 (0,6H) ; 2,85-3,10 (2,6H) ; 3,55-3,65 (1,4H) ; 4,35 (0,6H) ; 4,55 (0,4H) ; 4,94 (1H) ; 6,28 (0,4H) ; 6,38 (0,6H) ; 7,20-7,30 (5H).
LC/MS (ESI) : 445,2 ; (calculé ([M+H]⁺) : 445,6).

### Molécule A22 : produit obtenu par réaction entre la Boc-éthylènediamine et la molécule A21.

Par un procédé similaire à celui utilisé pour la préparation de la molécule A6 appliqué à la molécule A21 (5,67 g, 12,75 mmol) et à la Boc-éthylènediamine (2,25 g, 14,03 mmol), une huile incolore est obtenue après purification par colonne chromatographique sur gel de silice (dichlorométhane, méthanol).
Rendement : 5,7 g (76%)
RMN ¹H (CDCl₃, ppm) : 0,88 (3H) ; 1,25 (16H) ; 1,43 (9H) ; 1,58 (2,6H) ; 1,75-1,95 (1,4H) ; 2,15-2,30 (3H) ; 2,64 (0,5H) ; 2,95-3,10 (2,5H) ; 3,20-3,40 (4H) ; 3,45 (0,5H) ; 3,55 (0,2H) ; 3,66 (1H) ; 4,44 (1H) ; 4,50 (0,2H) ; 4,60 (0,6H) ; 4,99 (0,7H) ; 5,54 (0,5H) ; 5,95 (0,2H) ; 6,17 (1H) ; 6,60 (0,5H) ; 7,07 (0,5H) ; 7,20-7,40 (5H).
LC/MS (ESI) : 587,4 ; (calculé ([M+H]⁺) : 587,8).

### Molécule AA9

Après un procédé similaire à celui utilisé pour la préparation de la molécule AA2 appliqué à la molécule A22 (5,66 g, 9,65 mmol), le résidu obtenu après concentration sous vide du milieu réactionnel est dissous dans le méthanol et évaporé sous pression réduite, l'opération étant répétée 4 fois pour donner une mousse blanche de molécule AA9 sous forme de sel de chlorhydrate.
Rendement : 4,9 g (97%)
RMN ¹H (DMSO-d₆, 120°C, ppm) : 0,89 (3H) ; 1,26 (16H) ; 1,43 (2H) ; 1,68 (0,6H) ; 1,75-2,00 (3H) ; 2,05-2,25 (2,4H) ; 2,82-3,05 (5H) ; 3,38 (2H) ; 3,50-3,70 (1,4H) ; 4,25 (0,6H) ; 4,63 (0,4H) ; 4,77 (0,6H) ; 7,25-7,50 (5H) ; 7,55-8,20 (4H).
LC/MS (ESI) : 487,4 ; (calculé ([M+H]⁺) : 487,7).

### Exemple AA10 : molécule AA10

### Molécule A23 : produit obtenu par la réaction entre l'acide nipécotique et l'acide arachidique.

Par un procédé similaire à celui utilisé pour la préparation de la molécule A5 appliqué à l'acide arachidique (2,30 g, 7,37 mmol) et à l'acide nipécotique (1,00 g, 7,74 mmol), un solide blanc est obtenu après filtration de la phase aqueuse acidifiée jusqu'à pH 1 et lavage du solide à l'eau puis au dichlorométhane.
Rendement : 1,65 g (53%)
RMN 1H (CDCl₃, ppm) : 0,88 (3H) ; 1,07-1,88 (37H) ; 2,10 (1H) ; 2,28-2,45 (2H) ; 2,52 (1H) ; 2,91-3,17 (1,5H) ; 3,42 (0,5H) ; 3,72 (0,5H) ; 3,84 (0,5H) ; 4,08 (0,5H) ; 4,56 (0,5H).
LC/MS (ESI) : 424,4 ; 848,0 ; (calculé ([M+H]⁺) : 424,4 ; ([2M+H]⁺) : 847,8).

### Molécule A24 : produit obtenu par la réaction entre la molécule A23 et la Boc-1-amino-4,7,10-trioxa-13-tridécane amine.

A une suspension de molécule A23 (1,65 g, 3,89 mmol) dans 20 mL de THF sont ajoutés successivement et à température ambiante de la DIPEA (1,01 g, 7,79 mmol) et du TBTU (1,31 g, 4,09 mmol). Après 30 minutes d'agitation, de la Boc-1-amino-4,7,10-trioxa-13-tridécane amine (1,37 g, 4,28 mmol) est ajoutée et le milieu réactionnel est agité à température ambiante pendant 18 h. Après évaporation du solvant sous pression réduite, le résidu est dilué avec de l'acétate d'éthyle (100 mL), la phase organique est lavée successivement avec une solution aqueuse saturée en NaHCO₃, une solution aqueuse de HCl 1 N, une solution aqueuse saturée en NaCl, séchée sur Na₂SO₄, filtrée et concentrée sous pression réduite. Un solide blanc est obtenu après purification par chromatographie flash (cyclohexane, acétate d'éthyle, méthanol).
Rendement : 1,97 g (70%)
RMN ¹H (CDCl₃, ppm) : 0,88 (3H) ; 1,15-2,70 (54H) ; 3,10-3,46 (6H) ; 3,46-3,71 (12,6H) ; 3,92 (0,4H) ; 4,17 (0,6H) ; 4,49 (0,4H) ; 4,80-5,16 (1H) ; 6,35-6,76 (1H).
LC/MS (ESI) : 726,8 ; (calculé ([M+H]⁺) : 726,6).

### Molécule AA10

Par un procédé similaire à celui utilisé pour la préparation de la molécule AA1 appliqué à la molécule A24 (1,97 g, 2,71 mmol), un solide blanc de molécule AA10 est obtenu après évaporation du solvant, trituration dans l'acétone, filtration et lavage à l'acétone puis séchage sous pression réduite à 50°C.
Rendement : 1,66 g (92%)
RMN ¹H (DMSO-d₆, ppm) : 0,86 (3H) ; 1,09-1,90 (42H) ; 2,05-2,68 (5H) ; 2,45-2,68 (1H); 2,78-3,19 (6H) ; 3,36-3,44 (2H) ; 3,44-3,60 (10H) ; 3,69-3,87 (1H) ; 4,20 (0,4H) ; 4,35 (0,6H).
LC/MS (ESI) : 626,7 ; (calculé ([M+H]⁺) : 626,5).

### Exemple AA12 : molécule AA12

### Molécule A26 : produit obtenu par la réaction entre le chlorure de myristoyle et la L-proline

A une solution de L-proline (300,40 g, 2,61 mol) dans de la soude aqueuse 2 N (1,63 L) à 0°C est ajouté lentement sur 1 h du chlorure de myristoyle (322 g, 1,30 mol) en solution dans du dichlorométhane (1,63 L). A la fin de l'ajout, le milieu réactionnel est remonté à 20°C en 2 h, puis agité 2 h supplémentaires. Le mélange est refroidi à 0 °C puis une solution de HCl à 37% (215 mL) est ajoutée en 15 minutes. Le milieu réactionnel est agité 10 min à 0°C puis 1 h entre 0°C et 20°C. La phase organique est séparée, lavée avec une solution de HCl 10% (3 x 430 mL), une solution aqueuse saturée en NaCl (430 mL), séchée sur Na₂SO₄, filtrée sur coton puis concentrée sous pression réduite. Le résidu est solubilisé dans de l'heptane (315 mL), puis du pentane (1,6 L) est ajouté sous agitation mécanique. Un solide blanc est obtenu après filtration sur fritté et séchage sous pression réduite.
Rendement : 410,6 g (97%)
RMN ¹H (CDCl₃, ppm) : 0,88 (3H) ; 1,28 (20H) ; 1,70 (2H) ; 1,90-2,10 (3H) ; 2,36 (2H) ; 2,51 (1H) ; 3,47 (1H) ; 3,56 (1H) ; 4,61 (1H).
LC/MS (ESI) : 326,4 ; 651,7 ; (calculé ([M+H]⁺) : 326,3 ; ([2M+H]⁺) : 651,6).

### Molécule A27 : produit obtenu par la réaction entre la molécule A26 et la Boc-éthylènediamine

A une solution de molécule A26 (3,00 g, 9,21 mmol) à température ambiante dans le méthyl-THF (50 mL) sont ajoutés successivement du HOBt (1,83 g, 11,98 mmol) puis de la Boc-éthylènediamine (1,62 g, 10,14 mmol) et le milieu est refroidi à 0°C. De l'EDC (2,29 g, 11,98 mmol) est ajouté puis le mélange est agité 17 h entre 0°C et température ambiante. Le mélange réactionnel est ensuite lavé avec une solution aqueuse saturée en NH₄Cl (50 mL), une solution aqueuse saturée en NaHCO₃ (50 mL) puis une solution aqueuse saturée en NaCl (50 mL), séché sur Na₂SO₄, filtré et concentré sous pression réduite. Un solide blanc est obtenu après recristallisation dans le méthanol.
Rendement : 2,34 g (49%).
RMN ¹H (CDCl₃, ppm) : 0,88 (3H) ; 1,16-1,38 (20H) ; 1,44 (9H) ; 1,56-1,71 (2H) ; 1,78-2,45 (6H) ; 3,11-3,72 (6H) ; 4,30 (0,1H) ; 4,51 (0,9H) ; 4,87 (0,1H) ; 5,04 (0,9H) ; 6,87 (0,1H) ; 7,23 (0,9H).
LC/MS (ESI) : 468,0 ; (calculé ([M+H]⁺) : 468,4).

### Molécule AA12

Par un procédé similaire à celui utilisé pour la préparation de la molécule AA1 appliqué à la molécule A27 (2,34 g, 5,00 mmol), un solide blanc de molécule AA12 est obtenu après évaporation du solvant et triturations dans le diisopropyléther.
Rendement : 1,5 g (74%)
RMN ¹H (MeOD-d4, ppm) : 0,90 (3H) ; 1,21-1,43 (20H) ; 1,54-1,66 (2H) ; 1,85-2,28 (4H) ; 2,39 (2H) ; 3,00-3,17 (2H) ; 3,30-3,40 (1H) ; 3,43-3,71 (3H) ; 4,29 (0,94H) ; 4,48 (0,06H).
LC/MS (ESI) : 368,2 ; (calculé ([M+H]⁺) : 368,3).

### Exemple AA14 : molécule AA14

### Résine AA14-1 : produit obtenu par la réaction entre le 4,7,10-trioxa-1,13-tridecanediamine et la résine 2-Cl-trityl chloride.

A une solution de 4,7,10-trioxa-1,13-tridecanediamine (10,87 mL, 49,60 mmol) dans du dichlorométhane (50 mL) à température ambiante est ajoutée de la DIPEA (8,64 mL, 49,60 mmol). Cette solution est versée sur de la résine 2-Cl-trityl chloride préalablement lavée au dichlorométhane (100-200 mesh, 1% DVB, 1,24 mmol/g) (4,00 g, 4,96 mmol) dans un réacteur adapté à la synthèse peptidique sur support solide. Après 2 h d'agitation à température ambiante, du méthanol grade HPLC (0,8 mL/g résine, 3,2 mL) est ajouté et le milieu est agité à température ambiante pendant 15 minutes. La résine est filtrée, lavée successivement avec du dichlorométhane (3 x 50 mL), du DMF (2 x 50 mL), du dichlorométhane (2 x 50 mL), de l'isopropanol (1 x 50 mL) et du dichlorométhane (3 x 50 mL).

### Résine AA14-2 : produit obtenu par réaction entre la résine AA14-1 et la Fmoc-glycine.

A une suspension de Fmoc-glycine (4,42 g, 14,88 mmol) et de 1-[bis(dimethylamino)methylene]-1H-1,2,3-triazolo[4,5-b]pyridinium 3-oxide hexafluorophosphate (HATU, 5,66 g, 14,88 mmol) dans un mélange DMF/dichlorométhane 1:1 (60 mL) est ajoutée de la DIPEA (5,18 mL, 29,76 mmol). Après solubilisation complète, la solution obtenue est versée sur la résine AA14-1. Après 2 h d'agitation à température ambiante, la résine est filtrée, lavée successivement avec du DMF (3 x 60 mL), de l'isopropanol (1 x 60 mL) et du dichlorométhane (3 x 60 mL).

Résine AA14-3 : produit obtenu par réaction entre la résine AA14-2 et un mélange DMF/pipéridine 80:20. La résine AA14-2 est traitée avec un mélange DMF/pipéridine 80:20 (50 mL). Après 30 minutes d'agitation à température ambiante, la résine est filtrée, lavée successivement avec du DMF (3 x 50 mL), de l'isopropanol (1 x 50 mL) et du dichlorométhane (3 x 50 mL).

### Résine AA14-4 : produit obtenu par réaction entre la résine AA14-3 et la Fmoc-proline.

Par un procédé similaire à celui utilisé pour la résine AA14-2 appliqué à la résine AA14-3 et à la Fmoc-proline (5,02 g, 14,88 mmol) dans du DMF (50 mL), la résine AA14-4 est obtenue.

### Résine AA14-5 : produit obtenu par réaction entre la résine AA14-4 et un mélange DMF/pipéridine 80:20.

Par un procédé similaire à celui utilisé pour la résine AA14-3 appliqué à la résine AA14-4 et un mélange DMF/pipéridine 80:20 (50 mL), la résine AA14-5 est obtenue.

### Résine AA14-6 : produit obtenu par réaction entre la résine AA14-5 et l'acide palmitique.

Par un procédé similaire à celui utilisé pour la préparation de la résine AA14-4 appliqué à la résine AA14-5 et à l'acide palmitique (3,82 g, 14,88 mmol), la résine AA14-6 est obtenue.

**Molécule AA14** La résine AA14-6 est traitée avec un mélange TFA/dichlorométhane 1:1 (50 mL). Après 30 minutes d'agitation à température ambiante, la résine est filtrée et lavée avec du dichlorométhane (3 x 50 mL). Les solvants sont évaporés sous vide. Deux co-évaporations sont ensuite effectuées sur le résidu avec du dichlorométhane (50 mL) puis de l'éther diisopropylique (50 mL). Le résidu est solubilisé dans du dichlorométhane (50 mL) et la phase organique est lavée avec une solution aqueuse de NaOH 1 N (1 x 50 mL) puis une solution saturée de NaCl (2 x 50 mL). Après séchage sur Na₂SO₄, la phase organique est filtrée, concentrée sous vide et le résidu est purifié par chromatographie sur gel de silice (dichlorométhane, méthanol, NH₄OH)
Rendement : 1,65 g (54% global sur 7 étapes)
RMN ¹H (CDCl₃, ppm) : 0,88 (3H) ; 1,18-2,39 (38H) ; 2,79 (2H) ; 3,23-3,44 (2H) ; 3,47-3,69 (14H) ; 3,76 (0,92H) ; 3,82 (0,08H) ; 3,98 (0,08H) ; 4,03 (0,92H) ; 4,34 (0,08H) ; 4,39 (0,92H) ; 7,00-7,40 (2H).
LC/MS (ESI) : 613,7 ; (calculé ([M+H]⁺) : 613,5).

### AB : Synthèse des co-polyaminoacides

### Co-polyaminoacides statistiques de formule VII ou VIIa

**Tableau 1b**

| **n°** | **co-polyaminoacides porteur de charges carboxylates et de radicaux hydrophobes** |
|---|---|
| **AB1** | 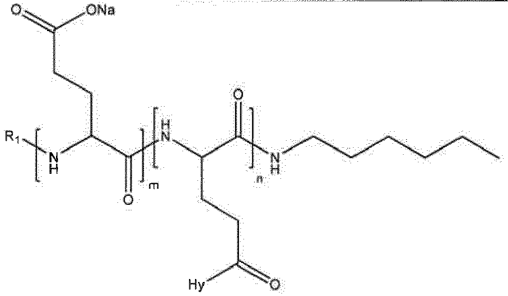 |
| | i = 0,05, DP (m + n) = 23 |
| | 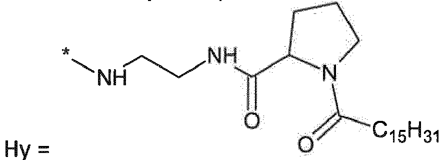 |
| | R₁ = H ou pyroglutamate |
| **AB2** | 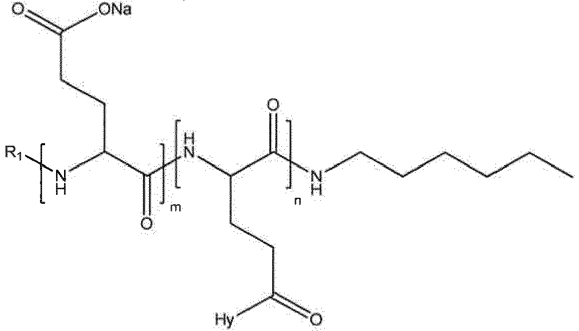 |
| | i = 0,05, DP (m + n) = 35 |
| | 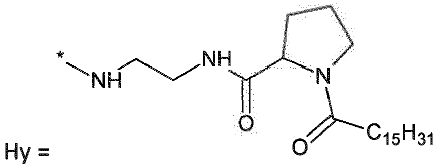 |
| | R₁ = H ou pyroglutamate |
| **AB3** | 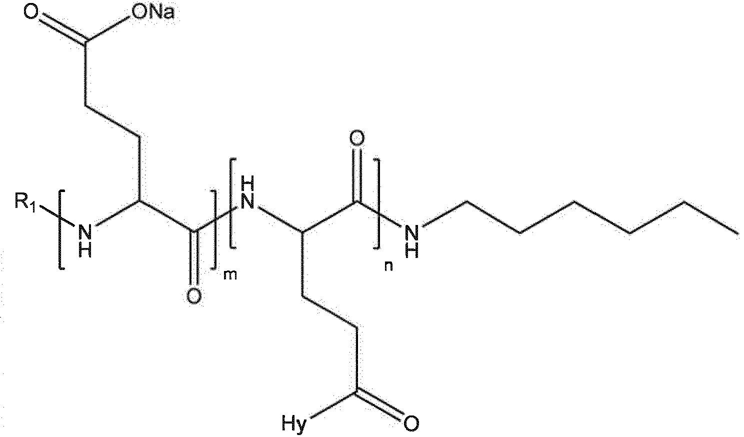 |
| | i = 0,10, DP (m + n) = 35 |
| | 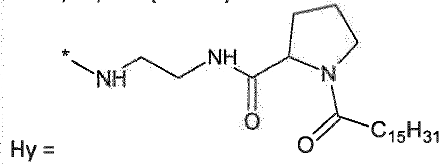 |
| | R₁ = H ou pyroglutamate |
| **AB4** | 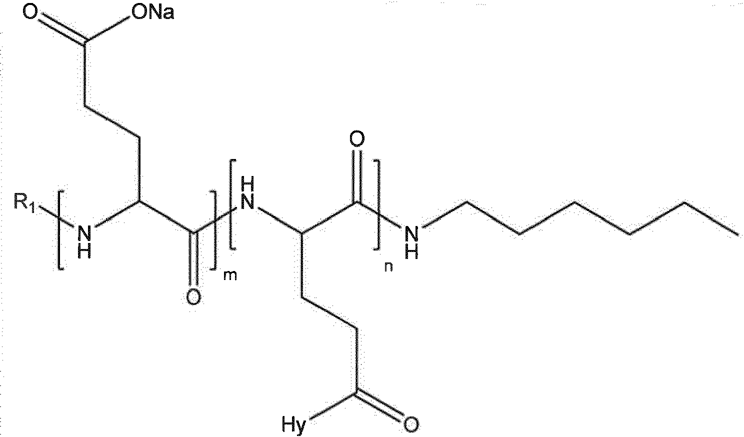 |
| | i = 0,052, DP (m + n) = 35 |
| | 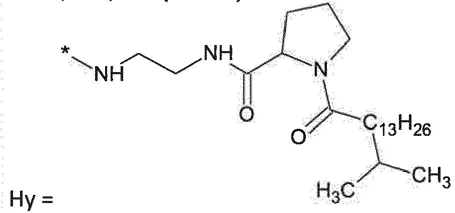 |
| | R₁ = H ou pyroglutamate |
| **AB5** | 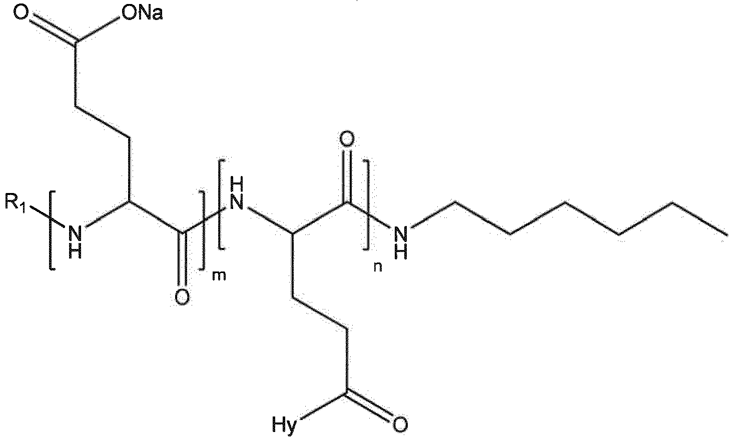 |
| | i = 0,05, DP (m + n) = 23 |
| | 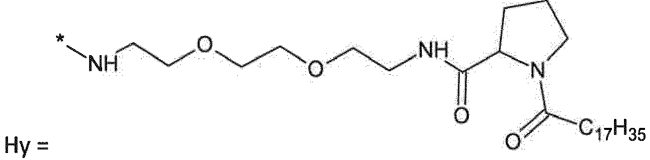 |
| | R₁ = H ou pyroglutamate |
| **AB6** | 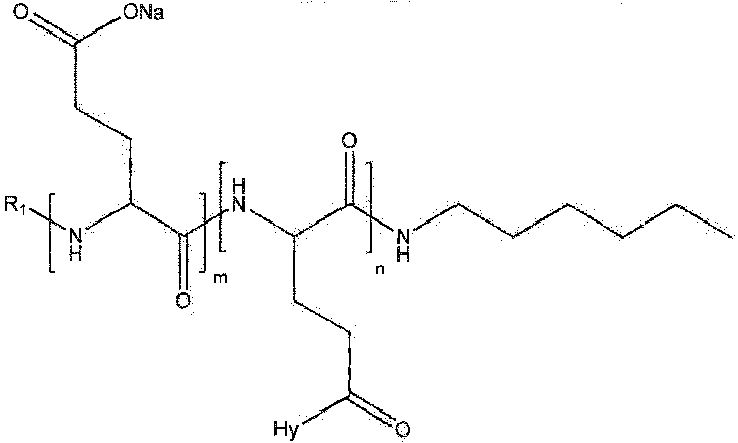 |
| | i = 0,025, DP (m + n) = 20 |
| | 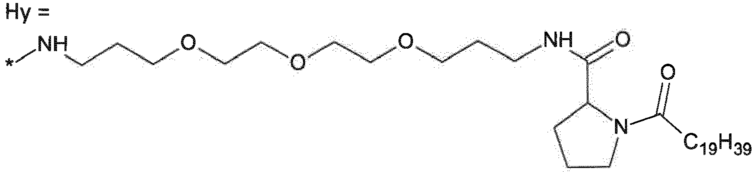 |
| | R₁ = H ou pyroglutamate |
| **AB7** | 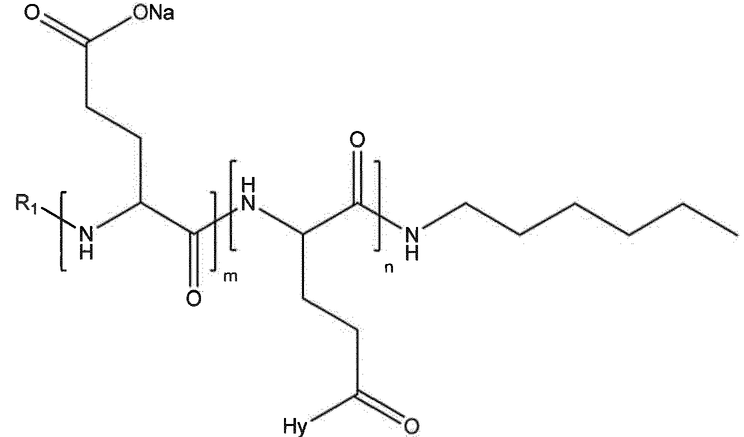 |
| | i = 0,03, DP (m + n) = 21 |
| | 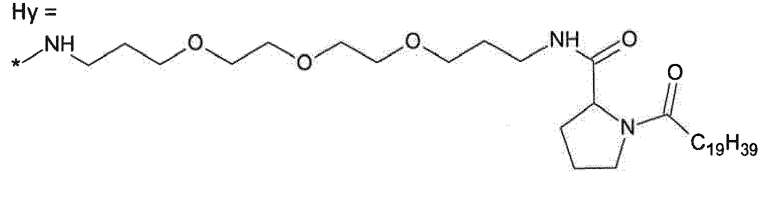 |
| | R₁=CH₃-CO-, H ou pyroglutamate |
| **AB8** | 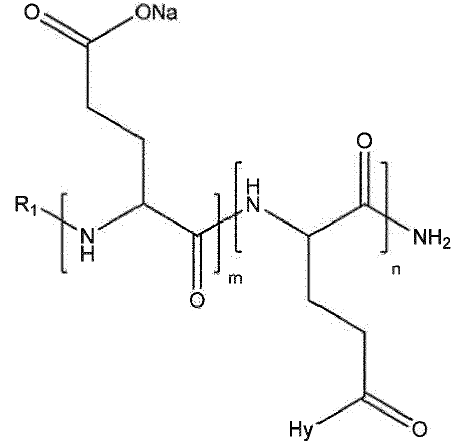 |
| | i = 0,03, DP (m + n) = 24 |
| | 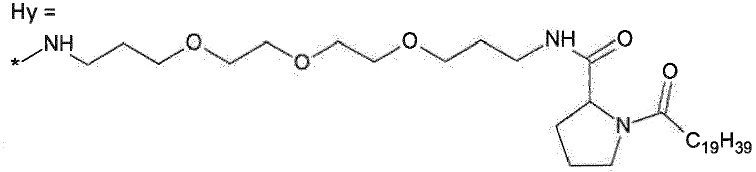 |
| | R₁ = H ou pyroglutamate |
| **AB9** | 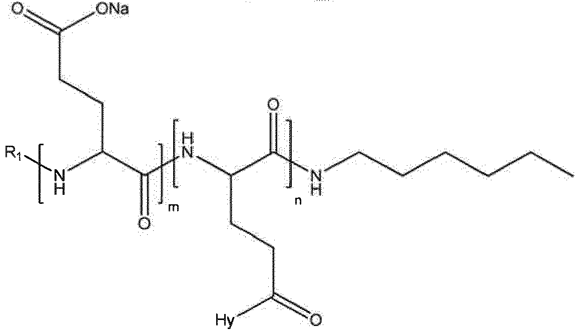 |
| | i = 0,12, DP (m + n) = 30 |
| | 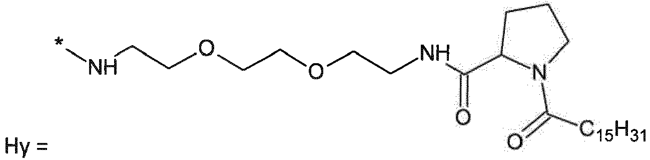 |
| | R₁ = H ou pyroglutamate |
| **AB10** | 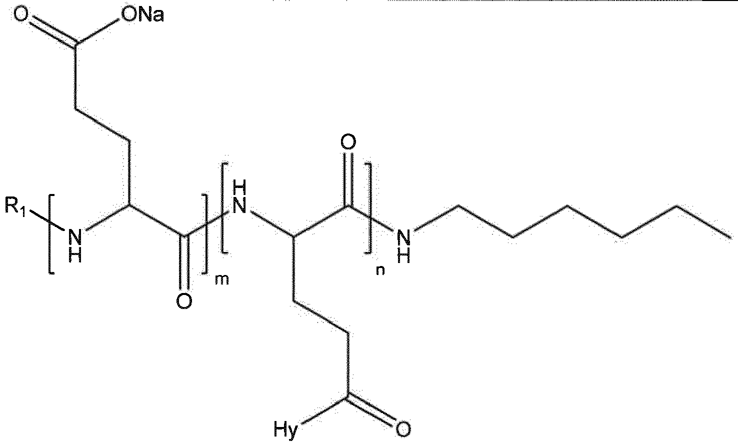 |
| | i = 0,08, DP (m + n) = 25 |
| | 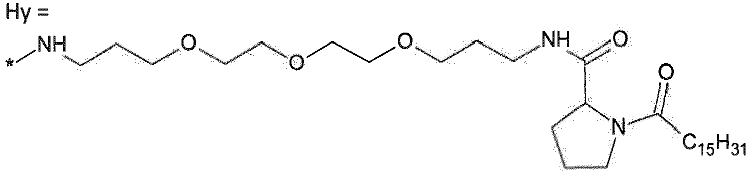 |
| | R₁ = H ou pyroglutamate |
| **AB11** | 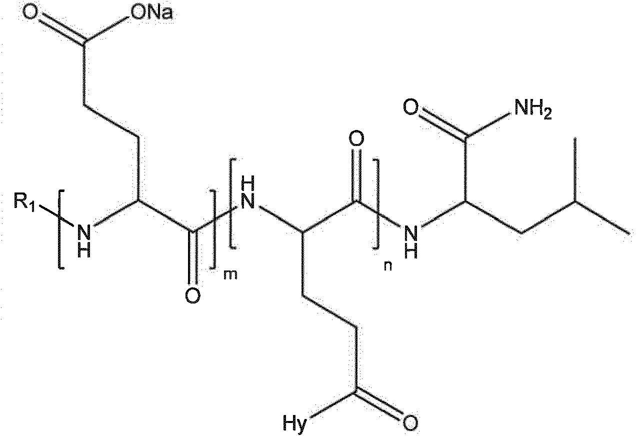 |
| | i = 0,05, DP (m + n) = 23 |
| | 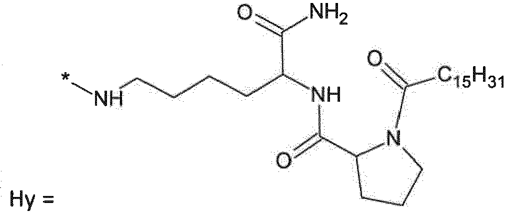 |
| | R₁ = H ou pyroglutamate |
| **AB12** | 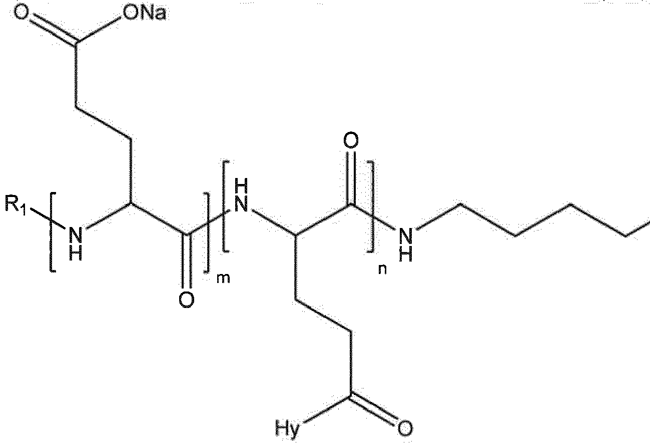 |
| | i = 0,04, DP (m + n) = 26 |
| | 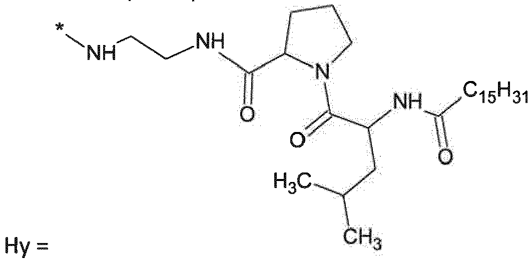 |
| | R₁ = H ou pyroglutamate |
| **AB13** | 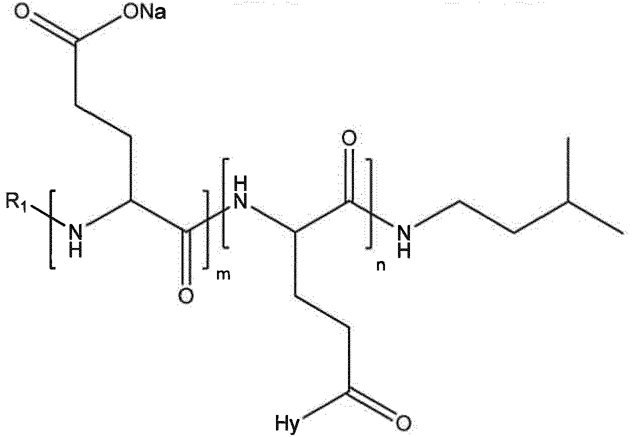 |
| | i = 0,12, DP (m+n) = 35 |
| | 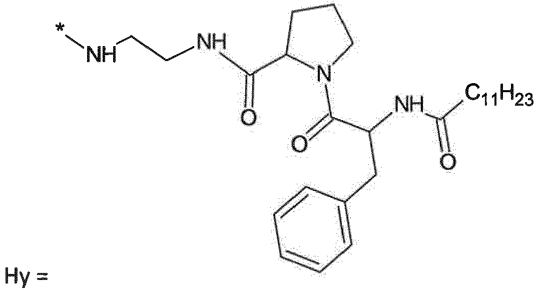 |
| | R₁ = H ou pyroglutamate |
| **AB21** | 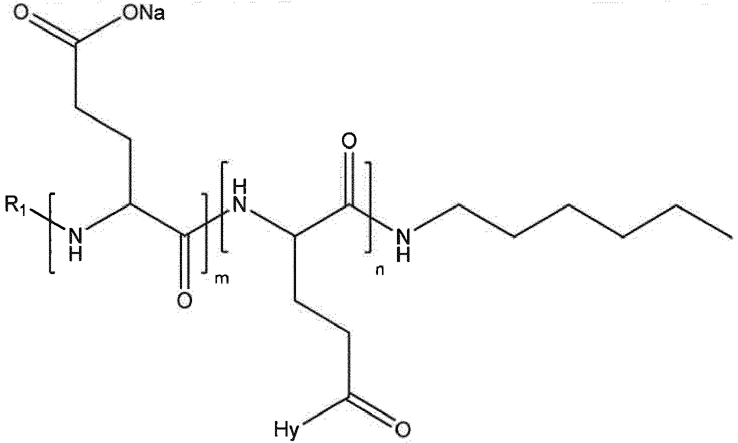 |
| | i = 0,056, DP (m + n) = 22 |
| | 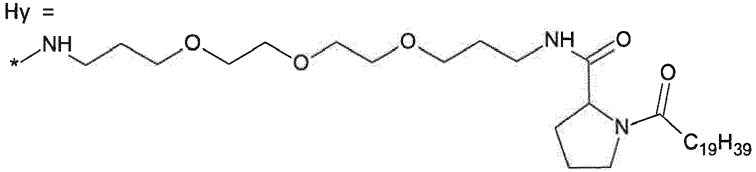 |
| | R₁ = H ou pyroglutamate |
| **AB24** | 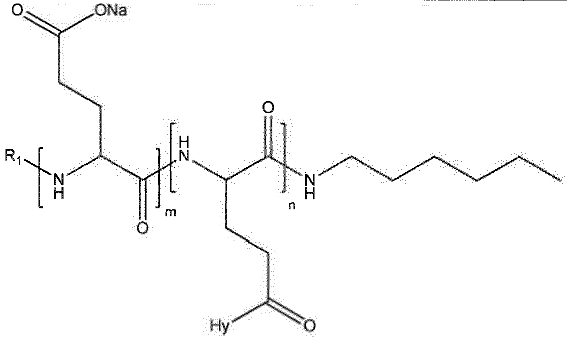 |
| | i =0,089, DP (m + n) = 38 |
| | 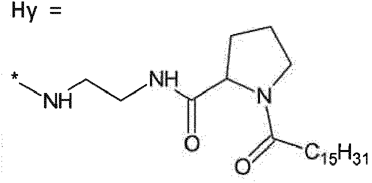 |
| | R₁ = CH₃-CO-, H ou pyroglutamate |
| **AB25** | 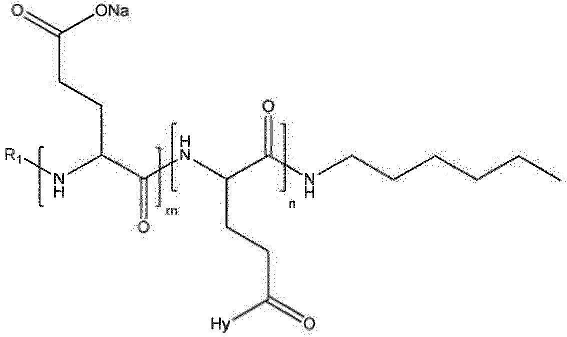 |
| | i = 0,16, DP (m + n) = 38 |
| | 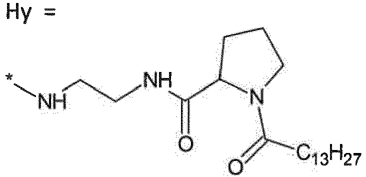 |
| | R₁ = CH₃-CO-, H ou pyroglutamate |
| **AB28** | 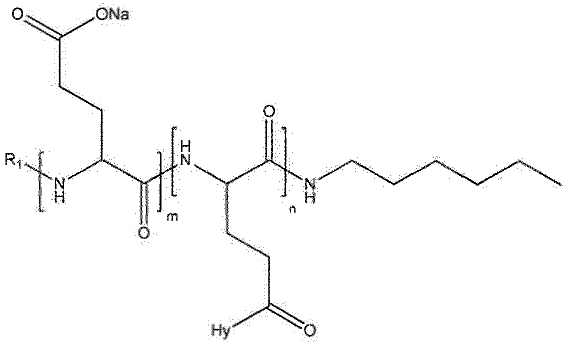 |
| | i = 0,1, DP (m + n) = 38 |
| | 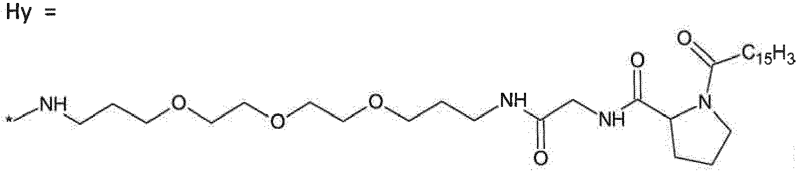 |
| | R₁ = CH₃-CO-, H ou pyroglutamate |

### Partie AB : synthèse des co-polyaminoacides

### Exemple AB1 : Co-polyaminoacide AB1 - poly-L-glutamate de sodium modifié par la molécule AA1 et ayant une masse molaire moyenne en nombre (Mn) de 2900 g/mol

Co-polyaminoacide AB1-1 : acide poly-L-glutamique de masse molaire moyenne en nombre (Mn) relative 3861 g/mol issu de la polymérisation du γ-benzyl-L-glutamate *N*-carboxyanhydride initiée par l'hexylamine

Dans un ballon préalablement séché à l'étuve est placé sous vide du γ-benzyl-L-glutamate *N*-carboxyanhydride (89,9 g, 341 mmol) pendant 30 min, puis du DMF anhydre (200 mL) est introduit. Le mélange est alors agité sous argon jusqu'à complète dissolution, refroidi à 4°C, puis de l'hexylamine (2,05 mL ; 15,5 mmol) est introduite rapidement. Le mélange est agité entre 4°C et température ambiante pendant 2 jours. Le milieu réactionnel est ensuite chauffé à 65°C pendant 2 h, refroidi à température ambiante puis coulé goutte à goutte dans du diisopropyléther (3 L) sous agitation. Le précipité blanc est récupéré par filtration, lavé avec du diisopropyléther (2 x 200 mL) puis séché sous vide à 30°C pour donner un acide poly(gamma-benzyl-L-glutamique) (PBLG).

A une solution de PBLG (74,8 g) dans l'acide trifluoroacétique (TFA, 340 mL) à 4°C est ajoutée goutte à goutte une solution d'acide bromhydrique (HBr) à 33% dans l'acide acétique (240 mL, 1,37 mol). Le mélange est agité à température ambiante pendant 2h, puis coulé goutte à goutte sur un mélange 1:1 (v/v) de diisopropyléther et d'eau sous agitation (4 L). Après 2h d'agitation, le mélange hétérogène est laissé au repos pendant une nuit. Le précipité blanc est récupéré par filtration, lavé avec un mélange 1:1 (v/v) de diisopropyléther et d'eau (340 mL) puis avec de l'eau (340 mL).

Le solide obtenu est alors solubilisé dans de l'eau (1,5 L) en ajustant le pH à 7 par ajout d'une solution aqueuse de soude 10 N puis une solution aqueuse de soude 1 N. Après solubilisation, la concentration théorique est ajustée à 20 g/L théorique par addition d'eau pour obtenir un volume final de 2,1 L.

La solution est filtrée sur filtre 0,45 µm puis purifiée par ultrafiltration contre une solution de NaCl 0,9%, puis de l'eau jusqu'à ce que la conductimétrie du perméat soit inférieure à 50 µS/cm. La solution de co-polyaminoacide est ensuite concentrée jusqu'à obtenir un volume final de 1,8 L.

La solution aqueuse est alors acidifiée par ajout de solution d'acide chlorhydrique 37% jusqu'à atteindre un pH de 2. Après 4 h d'agitation, le précipité obtenu est filtré, lavé avec de l'eau (2 x 340 mL) puis séché sous vide à 30°C pour donner un acide poly-L-glutamique de masse molaire moyenne en nombre (Mn) 3861 g/mol par rapport à un standard de polyoxyéthylène (PEG).

### Co-polyaminoacide AB1

Le co-polyaminoacide AB1-1 (10,0 g) est solubilisé dans le DMF (700 mL) à 30°C-40°C puis refroidi à 0°C. La molécule **AA1** sous forme de sel de chlorhydrate (1,64 g, 3,8 mmol) est mise en suspension dans du DMF (23 mL) et de la triéthylamine (0,39 g, 3,8 mmol) est ensuite ajoutée et le mélange est légèrement chauffé sous agitation jusqu'à complète dissolution. A la solution de co-polyaminoacide à 0°C, de la *N*-méthylmorpholine (NMM, 7,6 g, 75 mmol) dans le DMF (14 mL) et du chloroformate d'éthyle (ECF, 8,2 g, 75 mmol) sont ajoutés. Après 10 min à 0°C, la solution contenant la molécule AA1 est ajoutée et le milieu maintenu à 30°C durant 2h. Le milieu réactionnel est coulé goutte-à-goutte sur 5,5 L d'eau contenant du chlorure de sodium à 15% massique et du HCl (pH 2), puis laissé reposer une nuit. Le précipité est collecté par filtration et séché sous vide pendant environ 30 min. Le solide blanc obtenu est repris dans de l'eau (500 mL) et le pH est ajusté à 7 par ajout lent d'une solution aqueuse de NaOH 1N. Après filtration sur filtre 0,45 µm, la solution limpide obtenue est purifiée par ultrafiltration contre une solution de NaCl 0,9% puis de l'eau, jusqu'à ce que la conductimétrie du perméat soit inférieure à 50 µS/cm. Après déchargement, la solution est filtrée sur filtre 0,2 µm et stockée à 2-8°C.
Extrait sec : 24,9 mg/g
Un degré de polymérisation moyen (DP) de 23 est estimé par RMN ¹H dans D₂O en comparant l'intégration des signaux provenant de l'hydrophobe greffé à celle des signaux provenant de la chaine principale.
D'après la RMN ¹H : i = 0,05
La masse molaire moyenne calculée du co-polyaminoacide AB1 est calculée sur la base des masses molaires des radicaux R₁ et R₂, des résidus aspartate et/ou glutamate (y compris une liaison amide), du radical hydrophobe, du DS et du DP.
La masse molaire moyenne calculée du co-polyaminoacide AB1 est de 3945 g/mol. HPLC-SEC aqueuse (calibrant PEG) : Mn = 2900 g/mol.

### Exemple AB2 : Co-polyaminoacide AB2 - poly-L-glutamate de sodium modifié par la molécule AA1 et ayant une masse molaire moyenne en nombre (Mn) de 3700 g/mol

Par un procédé similaire à celui utilisé pour la préparation du co-polyaminoacide AB1 appliqué au sel de chlorhydrate de la molécule AA1 (1,64 g, 3,8 mmol) et à un acide poly-L-glutamique de Mn relative 5200 g/mol (10,0 g) obtenu par un procédé similaire à celui utilisé pour la préparation du co-polyaminoacide AB1-1, un poly-L-glutamate de sodium modifié par la molécule AA1 est obtenu.
Extrait sec : 14,1 mg/g
DP (estimé d'après la RMN ¹H) : 35
D'après la RMN ¹H : i = 0,05
La masse molaire moyenne calculée du co-polyaminoacide AB2 est de 5972 g/mol. HPLC-SEC aqueuse (calibrant PEG) : Mn = 3700 g/mol.

### Exemple AB3 : Co-polyaminoacide AB3 - poly-L-glutamate de sodium modifié par la molécule AA1 et ayant une masse molaire moyenne en nombre (Mn) de 4900 g/mol

Par un procédé similaire à celui utilisé pour la préparation du co-polyaminoacide AB1 appliqué au sel de chlorhydrate de la molécule AA1 (3,30 g, 7,6 mmol) et à un acide poly-L-glutamique de masse moyenne en nombre (Mn) relative 5200 g/mol (10,0 g) obtenu par un procédé similaire à celui utilisé pour la préparation du co-polyaminoacide AB1-1, un poly-L-glutamate de sodium modifié par la molécule AA1 est obtenu.
Extrait sec : 23,4 mg/g
DP (estimé d'après la RMN ¹H) : 35
La masse molaire moyenne calculée du co-polyaminoacide AB3 est de 6594 g/mol.
D'après la RMN ¹H : i = 0,10
HPLC-SEC aqueuse (calibrant PEG) : Mn = 4900 g/mol.

### Exemple AB4 : Co-polyaminoacide AB4 - poly-L-glutamate de sodium modifié par la molécule AA2 et ayant une masse molaire moyenne en nombre (Mn) de 1800 g/mol

Par un procédé similaire à celui utilisé pour la préparation du co-polyaminoacide AB1 appliqué au sel de chlorhydrate de la molécule AA2 (1,09 g, 2,4 mmol) et à un acide poly-L-glutamique de masse moyenne Mn = 5600 g/mol (6,3 g) obtenu par un procédé similaire à celui utilisé pour la préparation du co-polyaminoacide AB1-1 mais avec une étape de déprotection des esters benzyliques utilisant l'iodure de triméthylsilane selon le protocole décrit dans la publication J. Am. Chem. Soc. 2000, 122, 26-34 (Subramanian G., et al.), un poly-L-glutamate de sodium modifié par la molécule AA2 est obtenu.
Extrait sec : 21,5 mg/g
DP (estimé d'après la RMN ¹H) : 35
D'après la RMN ¹H : i = 0,052
La masse molaire moyenne calculée du co-polyaminoacide AB4 est de 6022 g/mol. HPLC-SEC aqueuse (calibrant PEG) : Mn = 1800 g/mol.

### Exemple AB5 : Co-polyaminoacide AB5 - poly-L-glutamate de sodium modifié par la molécule AA6 et ayant une masse molaire moyenne en nombre (Mn) de 2600 g/mol

Par un procédé similaire à celui utilisé pour la préparation du co-polyaminoacide AB1 appliqué au sel de chlorhydrate de la molécule AA6 (2,06 g, 3,8 mmol) et à un acide poly-L-glutamique (9,8 g) obtenu par un procédé similaire à celui utilisé pour la préparation du co-polyaminoacide AB1-1, un poly-L-glutamate de sodium modifié par la molécule AA6 est obtenu.
Extrait sec : 20,9 mg/g
DP (estimé d'après la RMN ¹H) : 23
D'après la RMN ¹H : i = 0,05
La masse molaire moyenne calculée du co-polyaminoacide AB5 est de 4079 g/mol. HPLC-SEC aqueuse (calibrant PEG) : Mn = 2600 g/mol.

### Exemple AB6 : Co-polyaminoacide AB6 - poly-L-glutamate de sodium modifié par la molécule AA7 et ayant une masse molaire moyenne en nombre (Mn) de 4000 g/mol

Un acide poly-L-glutamique de masse moyenne Mn = 3500 g/mol et de degré de polymérisation 22 (10,0 g) obtenu par un procédé similaire à celui utilisé pour la préparation du co-polyaminoacide AB1-1 est solubilisé dans le DMF (420 mL) à 30°C-40°C puis maintenu à cette température. En parallèle, le sel de chlorhydrate de la molécule AA7 (1,47 g, 2,3 mmol) est mis en suspension dans du DMF (12 mL) et de la triéthylamine (0,23 g, 2,3 mmol) est ajoutée, puis le mélange est légèrement chauffé sous agitation jusqu'à complète dissolution. A la solution de co-polyaminoacide dans le DMF, de la NMM (7,6 g, 75 mmol), la solution de AA7 puis de la N-oxyde de 2-hydroxypyridine (HOPO, 0,84 g, 7,5 mmol) sont ajoutées successivement. Le milieu réactionnel est alors refroidi à 0°C, puis du EDC (1,44 g, 7,5 mmol) est ajouté et le milieu est remonté à température ambiante durant 2 h. Le milieu réactionnel est filtré sur filtre tissé 0,2 mm et coulé au goutte-à-goutte sur 3,5 L d'eau contenant du NaCl à 15% massique et du HCl (pH 2) sous agitation. A la fin de l'ajout, le pH est réajusté à 2 avec une solution de HCl 37%, et la suspension est laissée reposer une nuit. Le précipité est collecté par filtration, puis rincé par 100 mL d'eau. Le solide blanc obtenu est solubilisé dans 500 mL d'eau par ajout lent d'une solution aqueuse de NaOH 1 N jusqu'à pH 7 sous agitation, puis la solution est filtrée sur filtre 0,45 µm. La solution limpide obtenue est purifiée par ultrafiltration contre une solution de NaCl 0,9% puis de l'eau, jusqu'à ce que la conductimétrie du perméat soit inférieure à 50 µS/cm. La solution est filtrée sur filtre 0,2 µm et stockée à 2-8°C.
Extrait sec : 21,6 mg/g
DP (estimé d'après la RMN ¹H) : 20
D'après la RMN ¹H : i = 0,025
La masse molaire moyenne calculée du co-polyaminoacide AB6 est de 3369 g/mol. HPLC-SEC aqueuse (calibrant PEG) : Mn = 4000 g/mol.

### Exemple AB7 : Co-polyaminoacide AB7 - poly-L-glutamate de sodium cappé à une de ses extrémités par un groupement acétyle et modifié par la molécule AA7 et ayant une masse molaire moyenne en nombre (Mn) de 3300 g/mol

Co-polyaminoacide AB7-1 : acide poly-L-glutamique de masse molaire moyenne en nombre (Mn) relative 3600 g/mol et de DP 21 issu de la polymérisation du γ-benzyl-L-glutamate N-carboxyanhydride initiée par l'hexylamine et cappé à l'une de ses extrémités par un groupement acétyle

Dans un ballon préalablement séché à l'étuve est placé sous vide du γ-benzyl-L-glutamate N-carboxyanhydride (Glu(OBn)-NCA, 100,0 g, 380 mmol) pendant 30 minutes puis du DMF anhydre (225 mL) est introduit. Le mélange est alors agité sous argon jusqu'à complète dissolution, refroidi à 4°C, puis de l'hexylamine (1,78 g, 17 mmol) est introduite rapidement. Le mélange est agité entre 4°C et température ambiante pendant 2 jours puis précipité dans du diisopropyléther (3,4 L). Le précipité est récupéré par filtration, lavé deux fois avec du diisopropyléther (225 mL) puis séché pour donner un solide blanc qui est dissous dans 450 mL de THF. A cette solution sont ajoutés successivement de la DIPEA, (31 mL, 176 mmol) puis de l'anhydride acétique (17 mL, 176 mmol). Après une nuit d'agitation à température ambiante, la solution est versée lentement dans du diisopropyléther (3 L) sous agitation. Après 1 h d'agitation, le précipité est filtré, lavé deux fois avec du diisopropyléther (250 mL) puis séché sous vide à 30°C pour donner un acide poly(gamma-benzyl-L-glutamique) cappé à une de ses extrémités par un groupement acétyle.

A une solution du co-polyaminoacide ci-dessus (72 g) dans l'acide trifluoroacétique (TFA, 335 mL) à 4°C est ajoutée goutte à goutte une solution d'acide bromhydrique (HBr) à 33% dans l'acide acétique (235 mL). Le mélange est agité à température ambiante pendant 3 h 30, puis coulé goutte à goutte sur un mélange 1:1 (v/v) de diisopropyléther et d'eau sous agitation (4 L). Après 2 h d'agitation, le mélange hétérogène est laissé au repos pendant une nuit. Le précipité blanc est récupéré par filtration, lavé avec un mélange 1:1 (v/v) de diisopropyléther et d'eau (340 mL) puis avec de l'eau (340 mL).

Le solide obtenu est alors solubilisé dans de l'eau (1,5 L) en ajustant le pH à 7 par ajout d'une solution aqueuse de soude 10 N puis une solution aqueuse de soude 1 N. Après solubilisation, la solution est diluée par addition d'eau pour obtenir un volume final de 2,1 L. La solution est filtrée sur filtre 0,45 µm puis purifiée par ultrafiltration contre une solution de NaCl 0,9%, puis de l'eau jusqu'à ce que la conductimétrie du perméat soit inférieure à 50 µS/cm. La solution de co-polyaminoacide est ensuite concentrée jusqu'à obtenir un volume final de 1,8 L.

La solution aqueuse est alors acidifiée par ajout de solution d'acide chlorhydrique 37% jusqu'à atteindre un pH de 2. Après 4 h d'agitation, le précipité obtenu est filtré, lavé avec de l'eau (330 mL) puis séché sous vide à 30°C pour donner un acide poly-L-glutamique de masse molaire moyenne en nombre (Mn) 3600 g/mol par rapport à un standard de polyoxyéthylène (PEG), et de degré moyen de polymérisation 21.

**Co-polyaminoacide AB7** : Par un procédé similaire à celui utilisé pour la préparation du co-polyaminoacide AB6 appliqué au sel de chlorhydrate de la molécule AA7 (1,43 g, 2,2 mmol) et au co-polyaminoacide AB7-1 (10,0 g), un acide poly-L-glutamate de sodium modifié par la molécule AA7 est obtenu.
Extrait sec : 24,3 mg/g
DP (estimé d'après la RMN ¹H) : 21
D'après la RMN ¹H : i = 0,03
La masse molaire moyenne calculée du co-polyaminoacide AB7 est de 3677 g/mol. HPLC-SEC aqueuse (calibrant PEG) : Mn = 3300 g/mol.

### Exemple AB8 : Co-polyaminoacide AB8 - poly-L-glutamate de sodium modifié par la molécule AA7 et ayant une masse molaire moyenne en nombre (Mn) de 3600 g/mol

Co-polyaminoacide AB8-1 : acide poly-L-glutamique de masse molaire moyenne en nombre (Mn) 3800 g / mol et de degré de polymérisation 24 issu de la polymérisation du γ-méthyl-L-glutamate *N*-carboxyanhydride initiée par l'ammoniac

Par un procédé similaire à celui décrit dans la demande de brevet FR-A-2 801 226 appliqué au γ-méthyl-L-acide glutamique *N*-carboxyanhydride (25,0 g, 133,6 mmol) et à une solution d'ammoniaque 0,5 N dans le dioxanne (12,1 mL, 6,05 mmol), un acide poly-L-glutamique est obtenu.

### Co-polyaminoacide AB8 :

Par un procédé similaire à celui utilisé pour la préparation du co-polyaminoacide AB6 appliqué au sel de chlorhydrate de la molécule AA7 (2,1 g, 3,24 mmol) et au co-polyaminoacide AB8-1 (14,3 g), un poly-L-glutamate de sodium modifié par la molécule AA7 est obtenu.
Extrait sec : 25,2 mg/g
DP (estimé d'après la RMN ¹H) : 24
D'après la RMN ¹H : i = 0,03
La masse molaire moyenne calculée du co-polyaminoacide AB8 est de 4099 g/mol. HPLC-SEC aqueuse (calibrant PEG) : Mn = 3600 g/mol.

### Exemple AB9 : Co-polyaminoacide AB9 - poly-L-glutamate de sodium modifié par la molécule AA3 et ayant une masse molaire moyenne en nombre (Mn) de 3200 g/mol

Par un procédé similaire à celui utilisé pour la préparation du co-polyaminoacide AB1 appliqué au sel de chlorhydrate de la molécule AA3 et à un acide poly-L-glutamique obtenu par un procédé similaire à celui utilisé pour la préparation du co-polyaminoacide AB1-1, un poly-L-glutamate de sodium modifié par la molécule AA3 est obtenu.
Extrait sec : 14,7 mg/g
DP (estimé d'après la RMN ¹H) : 30
D'après la RMN ¹H : i = 0,12
La masse molaire moyenne calculée du co-polyaminoacide AB9 est de 6192 g/mol. HPLC-SEC aqueuse (calibrant PEG) : Mn = 3200 g/mol.

### Exemple AB10 : Co-polyaminoacide AB10 - poly-L-glutamate de sodium modifié par la molécule AA4 et ayant une masse molaire moyenne en nombre (Mn) de 2600 g/mol

Par un procédé similaire à celui utilisé pour la préparation du co-polyaminoacide AB7 appliqué au sel de chlorhydrate de la molécule AA4 et à un acide poly-L-glutamique obtenu par un procédé similaire à celui utilisé pour la préparation du co-polyaminoacide AB1-1, un poly-L-glutamate de sodium modifié par la molécule AA4 est obtenu.
Extrait sec : 18,3 mg/g
DP (estimé d'après la RMN ¹H) : 25
D'après la RMN ¹H : i = 0,08
La masse molaire moyenne calculée du co-polyaminoacide AB10 est de 4870 g/mol. HPLC-SEC aqueuse (calibrant PEG) : Mn = 2600 g/mol.

### Exemple AB11: Co-polyaminoacide AB11 - poly-L-glutamate de sodium modifié par la molécule AA5 et ayant une masse molaire moyenne en nombre (Mn) de 2700 g/mol

Par un procédé similaire à celui utilisé pour la préparation du co-polyaminoacide AB6 appliqué au sel de chlorhydrate de la molécule AA5 et à un acide poly-L-glutamique obtenu par un procédé similaire à celui utilisé pour la préparation du co-polyaminoacide AB1-1, un poly-L-glutamate de sodium modifié par la molécule AA5 est obtenu.
Extrait sec : 20,2 mg/g
DP (estimé d'après la RMN ¹H) : 23
D'après la RMN ¹H : i = 0,05
La masse molaire moyenne calculée du co-polyaminoacide AB11 est de 4072 g/mol. HPLC-SEC aqueuse (calibrant PEG) : Mn = 2700 g/mol.

### Exemple AB12 : Co-polyaminoacide AB12 - poly-L-glutamate de sodium modifié par la molécule AA8 et ayant une masse molaire moyenne en nombre (Mn) de 3000 g/mol

Par un procédé similaire à celui utilisé pour la préparation du co-polyaminoacide AB1 appliqué au sel de chlorhydrate de la molécule AA8 et à un acide poly-L-glutamique obtenu par un procédé similaire à celui utilisé pour la préparation du co-polyaminoacide AB1-1, un poly-L-glutamate de sodium modifié par la molécule AA8 est obtenu.
Extrait sec : 19,5 mg/g
DP (estimé d'après la RMN ¹H) : 26
D'après la RMN ¹H : i = 0,04
La masse molaire moyenne calculée du co-polyaminoacide AB12 est de 4477 g/mol. HPLC-SEC aqueuse (calibrant PEG) : Mn = 3000 g/mol.

### Exemple AB13 : Co-polyaminoacide AB13 - poly-L-glutamate de sodium modifié par la molécule AA9 et ayant une masse molaire moyenne en nombre (Mn) de 3300 g/mol

Par un procédé similaire à celui utilisé pour la préparation du co-polyaminoacide AB6 appliqué au sel de chlorhydrate de la molécule AA9 et à un acide poly-L-glutamique obtenu par un procédé similaire à celui utilisé pour la préparation du co-polyaminoacide AB1-1 en utilisant l'isoamylamine comme initiateur en place de l'hexylamine, un poly-L-glutamate de sodium modifié par la molécule AA9 est obtenu.
Extrait sec : 22,3 mg/g
DP (estimé d'après la RMN ¹H) : 35
D'après la RMN ¹H : i = 0,12
La masse molaire moyenne calculée du co-polyaminoacide AB13 est de 7226 g/mol. HPLC-SEC aqueuse (calibrant PEG) : Mn = 3300 g/mol.

### Exemple AB21: Co-polyaminoacide AB21 - poly-L-glutamate de sodium modifié par la molécule AA7 et ayant une masse molaire moyenne en nombre (Mn) de 3400 g/mol

Par un procédé similaire à celui utilisé pour la préparation du co-polyaminoacide AB6 appliqué au sel de chlorhydrate de la molécule AA7 (2,44 g, 2,4 mmol) et à un acide poly-L-glutamique (10 g) obtenu par un procédé similaire à celui utilisé pour la préparation du co-polyaminoacide AB1-1, un poly-L-glutamate de sodium modifié par la molécule AA7 est obtenu.
Extrait sec : 22,7 mg/g
DP (estimé d'après la RMN ¹H) : 22
D'après la RMN ¹H : i = 0,056
La masse molaire moyenne calculée du co-polyaminoacide AB21 est de 4090 g/mol. HPLC-SEC aqueuse (calibrant PEG) : Mn = 3400 g/mol.

### Exemple AB24 : Co-polyaminoacide AB24 - poly-L-glutamate de sodium cappé à une de ses extrémités par un groupement acétyle et modifié par la molécule AA1 et ayant une masse molaire moyenne en nombre (Mn) de 3900 g/mol

Par un procédé similaire à celui utilisé pour la préparation du co-polyaminoacide AB6 appliqué au sel de chlorhydrate de la molécule AA1 (1,330 g, 3,08 mmol) et à un acide poly-L-glutamique de Mn relative 5400 g/mol (4,0 g) obtenu par un procédé similaire à celui utilisé pour la préparation du co-polyaminoacide AB7-1, un poly-L-glutamate de sodium cappé à une de ses extrémités par un groupement acétyle et modifié par la molécule AA1 est obtenu.
Extrait sec : 18,7 mg/g
DP (estimé d'après la RMN ¹H) : 38
D'après la RMN ¹H : i = 0,089
La masse molaire moyenne calculée du co-polyaminoacide AB24 est de 7088 g/mol. HPLC-SEC aqueuse (calibrant PEG) : Mn = 3900 g/mol.

### Exemple AB25 : Co-polyaminoacide AB25 - poly-L-glutamate de sodium cappé à une de ses extrémités par un groupement acétyle et modifié par la molécule AA12 et ayant une masse molaire moyenne en nombre (Mn) de 3700 g/mol

Un acide poly-L-glutamique de masse moyenne relative Mn 5400 g/mol et de degré de polymérisation 38 (10,0 g), obtenu par un procédé similaire à celui utilisé pour la préparation du co-polyaminoacide AB7-1 est solubilisé dans le DMF (420 mL) à 30°C puis maintenu à cette température. En parallèle, le sel de chlorhydrate de la molécule AA12 (4,56 g, 11,29 mmol) est mis en solution dans du chloroforme (60 mL) et de la triéthylamine (1,14 g, 11,29 mmol) est ajoutée. A la solution de co-polyaminoacide dans le DMF, de la NMM (7,6 g, 75,26 mmol), puis de la HOPO (2,51 g, 22,58 mmol) sont ajoutées successivement. Le milieu réactionnel est alors refroidi à 0°C, puis du EDC (4,33 g, 22,58 mmol) est ajouté, le milieu est agité 1 h à 0°C puis la solution de molécule AA12 est ajoutée. Le mélange réactionnel est agité durant 2 h entre 0°C et température ambiante. Le milieu réactionnel est filtré sur filtre tissé 0,2 mm et coulé au goutte-à-goutte sur 3,95 L d'eau contenant du NaCl à 15% massique et du HCl (pH 2) sous agitation. A la fin de l'ajout, le pH est réajusté à 2 avec une solution de HCl 37%, et la suspension est laissée reposer une nuit. Le précipité est collecté par filtration, puis solubilisé dans 780 mL d'eau par ajout lent d'une solution aqueuse de NaOH 1 N jusqu'à pH 7 sous agitation. Après filtration sur filtre 0,45 µm, la solution est diluée par addition d'eau pour obtenir un volume de 900 mL puis de l'acétone (485 mL) est ajoutée pour obtenir une solution contenant 30% massique d'acétone. Cette solution est filtrée sur filtre de charbon actif (3M R53SLP) puis l'acétone est distillée (40°C, 100 mbar). Après filtration sur filtre 0,45 µm, le produit est purifié par ultrafiltration contre une solution aqueuse de NaCl à 0,9%, une solution de tampon carbonate (150 mM), une solution aqueuse de NaCl à 0,9%, une solution de tampon phosphate (150 mM), une solution aqueuse de NaCl à 0,9%, puis de l'eau jusqu'à ce que la conductimétrie du perméat soit inférieure à 50 µS/cm. La solution est ensuite concentrée jusqu'à obtenir un volume final de 600 mL. La solution est filtrée sur filtre 0,2 µm et stockée à 2-8°C.
Extrait sec : 19,7 mg/g
DP (estimé d'après la RMN ¹H) : 38
D'après la RMN ¹H : i = 0,16
La masse molaire moyenne calculée du co-polyaminoacide AB25 est de 7877 g/mol. HPLC-SEC aqueuse (calibrant PEG) : Mn = 3700 g/mol.

### Exemple AB28 : Co-polyaminoacide AB28 - poly-L-glutamate de sodium cappé à une de ses extrémités par un groupement acétyle et modifié par la molécule AA14 et ayant une masse molaire moyenne en nombre (Mn) de 4700 g/mol

Par un procédé similaire à celui utilisé pour la préparation du co-polyaminoacide AB6 appliqué à la molécule AA14 (1,51 g, 2,46 mmol) et à un acide poly-L-glutamique de Mn relative 5400 g/mol (3,27 g) obtenu par un procédé similaire à celui utilisé pour la préparation du co-polyaminoacide AB7-1, un poly-L-glutamate de sodium cappé à une de ses extrémités par un groupement acétyle et modifié par la molécule AA14 est obtenu après purification par ultrafiltration contre une solution aqueuse de NaCl à 0,9%, une solution de tampon carbonate (150 mM), une solution aqueuse de NaCl à 0,9%, une solution de tampon phosphate (150 mM), une solution aqueuse de NaCl à 0,9%, puis de l'eau jusqu'à ce que la conductimétrie du perméat soit inférieure à 50 µS/cm. La solution de co-polyaminoacide est ensuite concentrée à environ 20 g/L théorique et le pH est ajusté à 7. La solution aqueuse est filtrée sur 0,2 µm et conservée à 4°C.
Extrait sec : 6,1 mg/g
DP (estimé d'après la RMN ¹H) : 38
D'après la RMN ¹H : i = 0,1
La masse molaire moyenne calculée du co-polyaminoacide AB28 est de 8062 g/mol. HPLC-SEC aqueuse (calibrant PEG) : Mn = 4700 g/mol.

### Partie B:

### BB : synthèse des molécules hydrophobes dans lesquelles p = 2

Les radicaux sont représentés dans le tableau suivant par la molécule hydrophobe correspondante avant greffage sur le co-polyaminoacide.

**Tableau 1d : liste des molécules hydrophobes synthétisées selon l'invention.**

| **N°** | **Structure de la molécule hydrophobe avant greffage sur le co-polyaminoacide** |
|---|---|
| **BA1** | 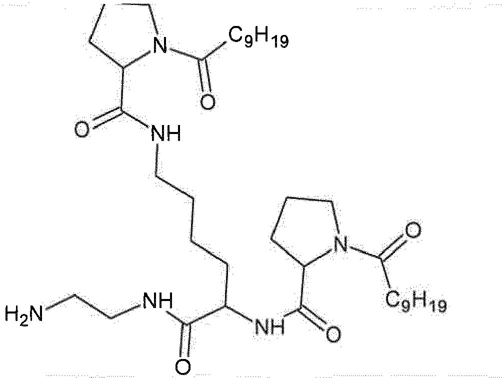 |
| **BA2** | 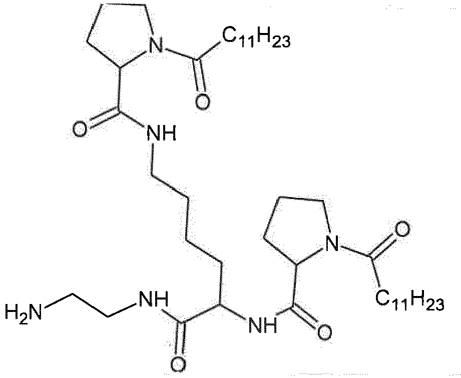 |
| **BA3** | 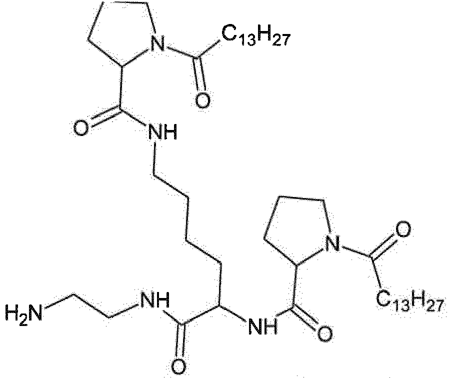 |
| **BA4** | 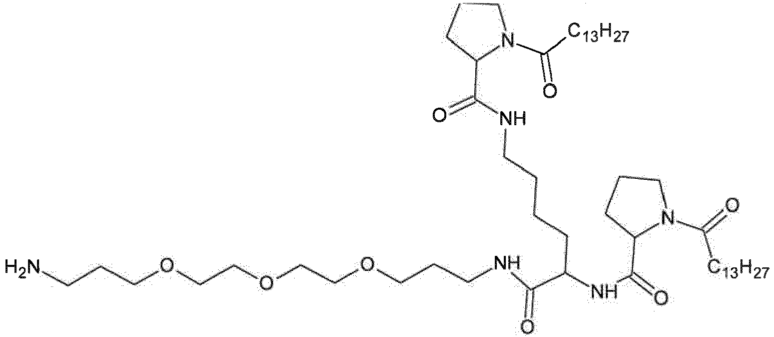 |
| **BA5** | 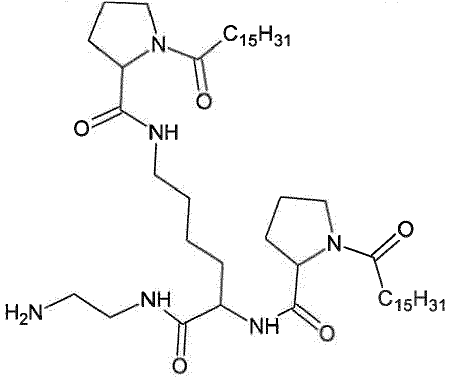 |
| **BA6** | 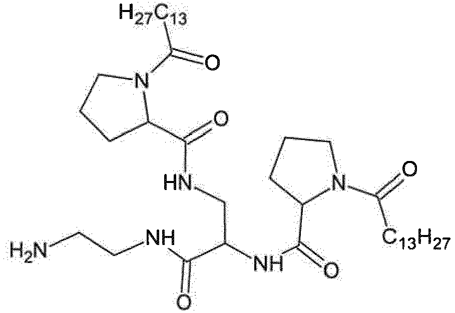 |
| **BA7** | 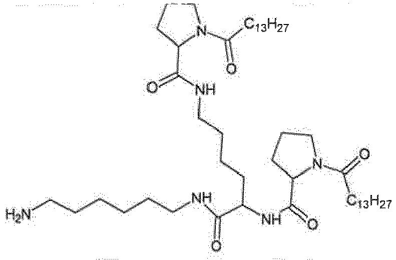 |

### Part BA : synthèse des molécules hydrophobes dans lesquelles p = 2 Exemple BA1 : molécule BA1

### Molécule B1 : produit obtenu par la réaction entre l'acide décanoïque et la L-proline.

A une solution d'acide décanoïque (14,28 g, 82,91 mmol) dans le THF (520 mL) à 0°C sont ajoutés successivement du dicyclohexyle carbodiimide (DCC) (16,29 g, 78,96 mmol) et du *N*-hydroxysuccinimide (NHS) (9,09 g, 78,96 mmol). Après 60 h d'agitation à température ambiante, le milieu est refroidi à 0°C pendant 20 min, filtré sur fritté. De la L-proline (10 g, 86,86 mmol), de la diisopropyléthylamine (DIPEA) (68,8 mL) et de l'eau (60 mL) sont ajoutés au filtrat. Après 24 h d'agitation à température ambiante, le milieu est dilué avec de l'eau (300 mL). La phase aqueuse est lavée avec de l'acétate d'éthyle (2 x 250 mL), acidifiée jusqu'à pH ∼1 avec une solution aqueuse d'HCl 1 N puis extraite avec du dichlorométhane (3 x 150 mL). Les phases organiques combinées sont séchées sur Na₂SO₄, filtrées, concentrées sous vide et le résidu est purifié par chromatographie sur gel de silice (cyclohexane, acétate d'éthyle).
Rendement : 14,6 g (69%)
RMN ¹H (CDCl₃, ppm) : 0,87 (3H) ; 1,26 (12H) ; 1,65 (2H) ; 2,02 (3H) ; 2,34 (2H) ; 2,41 (1H) ; 3,48 (1H) ; 3,56 (1H) ; 4,58 (1H).
LC/MS (ESI) : 270,2 ; (calculé ([M+H]⁺) : 270,4).

### Molécule B2 : produit obtenu par la réaction entre la molécule B1 et la L-lysine.

Par un procédé similaire à celui utilisé pour la préparation de la molécule B1 appliqué à la molécule B1 (14,57 g, 54,07 mmol) et à la L-lysine (4,15 g, 28,39 mmol), une huile jaune est obtenue.
Rendement : 16,4 g (93%)
RMN ¹H (CDCl₃, ppm) : 0,88 (6H) ; 1,26 (24H) ; 1,35-1,65 (8H) ; 1,85-2,35 (12H) ; 2,53 (0,2H) ; 2,90 (0,8H) ; 3,45-3,75 (5H) ; 4,50-4,70 (3H) ; 7,82 (1H).
LC/MS (ESI) : 649,6 ; (calculé ([M+H]⁺) : 649,9).

### Molécule B3 : produit obtenu par réaction entre la molécule B2 et la Boc-éthylènediamine.

A une solution de molécule B2 (16,4 g, 25,27 mmol) dans le THF (170 mL) sont ajoutés de la DIPEA (8,80 mL) et du 2-(1H-benzotriazol-1-yl)-1,1,3,3-tétraméthyluronium tétrafluoroborate (TBTU, 8,52 g, 26,54 mmol) à température ambiante. Après 30 min d'agitation, de la Boc-éthylènediamine (4,45 g, 27,8 mmol) est ajoutée. Après agitation à température ambiante pendant 2 h, le solvant est évaporé sous pression réduite et le résidu est dilué avec de l'acétate d'éthyle (400 mL). La phase organique est lavée avec de l'eau (250 mL), une solution aqueuse saturée de NaHCO₃ (250 ml), une solution aqueuse de 1 N HCl (250 mL), une solution aqueuse saturée en NaCl (250 mL) et est séchée sur Na₂SO₄. Après filtration et concentration sous vide, le résidu obtenu est purifié par chromatographie sur gel de silice (acétate d'éthyle, méthanol) pour donner une huile incolore.
Rendement : 12,8 g (64%)
RMN ¹H (CDCl₃, ppm) : 0,87 (6H) ; 1,25-1,60 (42H) ; 1,80-2,05 (4H) ; 2,15-2,45 (9H) ; 3,10-3,75 (10H) ; 4,30 (1H) ; 4,50 (2H) ; 5,50 (0,6H) ; 5,89 (0,2H) ; 6,15 (0,2H) ; 7,03 (1H) ; 7,47 (1H).
LC/MS (ESI) : 791,8 ; (calculé ([M+H]⁺) : 792,1).

### Molécule BA1

A une solution de la molécule B3 (12,78 g, 16,15 mmol) dans le dichlorométhane (110 mL) à 5°C est ajoutée une solution de HCl 4 N dans le dioxane (20,2 mL). Après 20 h d'agitation à 5°C, le milieu est concentré sous vide. Le résidu obtenu est dissous dans le méthanol et évaporé sous vide, cette opération étant répétée 4 fois pour donner un solide blanc de molécule BA1 sous forme de sel de chlorhydrate.
Rendement : 11,4 g (97%)
RMN ¹H (DMSO-d₆, ppm) : 0,85 (6H) ; 1,25-1,50 (33H) ; 1,57 (1H) ; 1,70-2,40 (12H) ; 2,82 (2H) ; 3,00 (2H) ; 3,25-3,70 (6H) ; 4,05-4,50 (3H) ; 7,75-8,45 (6H).
LC/MS (ESI) : 691,6 ; (calculé ([M+H]⁺) : 692,0).

### Exemple BA2 : molécule BA2

### Molécule B4 : produit obtenu par la réaction entre l'acide laurique et la L-proline.

Par un procédé similaire à celui utilisé pour la préparation de la molécule B1 appliqué à l'acide laurique (31,83 g, 157,9 mmol) et à la L-proline (20 g, 173,7 mmol), une huile jaune est obtenue.
Rendement : 34,3 g (73%)
RMN ¹H (CDCl₃, ppm) : 0,87 (3H) ; 1,26 (16H) ; 1,70 (2H) ; 1,90-2,10 (3H) ; 2,35 (2H) ; 2,49 (1H) ; 3,48 (1H) ; 3,56 (1H) ; 4,60 (1H).
LC/MS (ESI) : 298,2 ; (calculé ([M+H]⁺) : 298,4).

### Molécule B5 : produit obtenu par la réaction entre la molécule B4 et la L-lysine.

Par un procédé similaire à celui utilisé pour la préparation de la molécule B1 appliqué à la molécule B4 (33,72 g, 113,36 mmol) et à la L-lysine (8,70 g, 59,51 mmol), un solide blanc est obtenu.
Rendement : 26,2 g (66%)
RMN ¹H (CDCl₃, ppm) : 0,88 (6H) ; 1,26 (32H) ; 1,35-1,65 (8H) ; 1,85-2,35 (15H) ; 2,87 (1H) ; 3,40-3,75 (5H) ; 4,50-4,75 (3H) ; 7,87 (1H).
LC/MS (ESI) : 705,6 ; (calculé ([M+H]⁺) : 706,0).

### Molécule B6 : produit obtenu par réaction entre la Boc-éthylènediamine et la molécule B5.

Par un procédé similaire à celui utilisé pour la préparation de la molécule B3 appliqué à la molécule B5 (25,74 g, 36,51 mmol) et à la Boc-éthylènediamine (6,43 g, 40,16 mmol), une huile incolore est obtenue.
Rendement : 30,9 g (quantitatif)
RMN ¹H (CDCl₃, ppm) : 0,88 (6H) ; 1,35-1,65 (50H) ; 1,85-2,35 (13H) ; 3,05-3,75 (10H) ; 4,25-4,65 (3H) ; 5,50 (0,4H) ; 5,88 (0,2H) ; 6,16 (0,2H) ; 7,08 (1H) ; 7,26 (1H) ; 7,49 (0,2H)
LC/MS (ESI) : 847,8 ; (calculé ([M+H]⁺) : 848,2).

### Molécule BA2

Après un procédé similaire à celui utilisé pour la préparation de la molécule BA1 appliqué à la molécule B6 (30,9 g, 36,47 mmol), le résidu obtenu après concentration sous vide est dissous dans le méthanol et évaporé sous vide, cette opération étant répétée 4 fois pour donner un solide blanc de molécule BA2 sous forme de sel de chlorhydrate après séchage sous pression réduite.
Rendement : 27,65 g (97%)
RMN ¹H (DMSO-d₆, ppm) : 0,85 (6H) ; 1,10-2,40 (54H) ; 2,75-3,15 (4H) ; 3,25-3,60 (6H) ; 4,05-4,50 (3H) ; 7,50-8,50 (6H).
LC/MS (ESI) : 747,6 ; (calculé ([M+H]⁺) : 748,1).

### Exemple BA3 : molécule BA3

### Molécule B7 : produit obtenu par la réaction entre l'acide myristique et la L-proline.

Par un procédé similaire à celui utilisé pour la préparation de la molécule B1 appliqué à l'acide myristique (18,93 g, 82,91 mmol) et à la L-proline (10 g, 86,86 mmol), une huile jaunâtre est obtenue.
Rendement : 20 g (78%)
RMN ¹H (CDCl₃, ppm) : 0,88 (3H) ; 1,28 (20H) ; 1,70 (2H) ; 1,90-2,10 (3H) ; 2,36 (2H) ; 2,51 (1H) ; 3,47 (1H) ; 3,56 (1H) ; 4,61 (1H).
LC/MS (ESI) : 326,2 ; (calculé ([M+H]⁺) : 326,6).

### Molécule B8 : produit obtenu par la réaction entre la molécule B7 et la L-lysine

Par un procédé similaire à celui utilisé pour la préparation de la molécule B1 appliqué à la molécule B7 (20,02 g, 61,5 mmol) et à la L-lysine (4,72 g, 32,29 mmol), un solide blanc est obtenu.
Rendement : 12,3 g (53%)
RMN ¹H (DMSO-d₆, ppm) : 0,85 (6H) ; 1,26 (40H) ; 1,35-1,50 (6H) ; 1,50-2,10 (10H) ; 2,10-2,25 (4H) ; 3,01 (2H) ; 3,31-3,55 (4H) ; 4,10-4,40 (3H) ; 7,68 (0,6H) ; 7,97 (1H) ; 8,27 (0,4H) ; 12,50 (1H).
LC/MS (ESI) : 761,8 ; (calculé ([M+H]⁺) : 762,1).

### Molécule B9 : produit obtenu par la réaction entre la Boc-éthylènediamine et la molécule B8.

Par un procédé similaire à celui utilisé pour la préparation de la molécule B3 appliqué à la molécule B8 (12 g, 15,77 mmol) et à la Boc-éthylènediamine (3,03 g, 18,92 mmol), une huile incolore est obtenue après purification par colonne chromatographique sur gel de silice (acétate d'éthyle, méthanol).
Rendement : 12,5 g (88%)
RMN ¹H (DMSO-d₆, ppm) : 0,85 (6H) ; 1,20-1,55 (55H) ; 1,50-2,25 (14H) ; 2,95-3,10 (6H) ; 3,31-3,55 (4H) ; 4,10-4,40 (3H) ; 6,74 (1H) ; 7,60-8,25 (3H).
LC/MS (ESI) : 904,1 ; (calculé ([M+H]⁺) : 904,3).

### Molécule BA3

Après un procédé similaire à celui utilisé pour la préparation de la molécule BA1 appliqué à la molécule B9 (12,5 g, 13,84 mmol), le résidu obtenu après concentration sous vide est dissous dans le méthanol et évaporé sous vide, cette opération étant répétée 4 fois pour donner un solide blanc de molécule BA3 sous forme de sel de chlorhydrate après séchage sous pression réduite.
Rendement : 9,2 g (79%)
RMN ¹H (DMSO-d₆, ppm) : 0,85 (6H) ; 1,10-1,65 (48H) ; 1,70-2,35 (12H) ; 2,85 (2H) ; 3,01 (2H) ; 3,25-3,65 (6H) ; 4,10-4,50 (3H) ; 7,70-8,40 (6H).
LC/MS (ESI) : 803,9 ; (calculé ([M+H]⁺) : 804,2).

### Exemple BA4 : molécule BA4

### Molécule B10 : produit obtenu par la réaction entre la molécule B8 et le Boc-1-amino-4,7,10-trioxa-13-tridécane.

Par un procédé similaire à celui utilisé pour la préparation de la molécule B3 appliqué à la molécule B8 (29,80 g, 39,15 mmol) et au Boc-1-amino-4,7,10-trioxa-13-tridécane (15,05 g, 46,96 mmol), une huile épaisse incolore est obtenue.
Rendement : 25,3 g (61%)
RMN ¹H (DMSO-d₆, ppm) : 0,85 (6H) ; 1,25-2,35 (75H) ; 2,85-3,20 (6H) ; 3,25-3,65 (16H) ; 4,10-4,45 (3H) ; 6,38 (0,1H) ; 6,72 (0,9H) ; 7,50-8,25 (3H).
LC/MS (ESI) : 1064,2 ; (calculé ([M+H]⁺) : 1064,5).

### Molécule BA4

Après un procédé similaire à celui utilisé pour la préparation de la molécule BA1 appliqué à la molécule B10 (25,3 g, 23,8 mmol), le résidu obtenu après concentration sous vide est dissous dans le méthanol et évaporé sous vide, cette opération étant répétée 4 fois pour donner un solide blanc de molécule BA4 sous forme de sel de chlorhydrate après séchage sous pression réduite.
Rendement : 20,02 g (84%)
RMN ¹H (DMSO-d₆, ppm) : 0,85 (6H) ; 1,15-2,35 (66H) ; 2,80-3,20 (6H) ; 3,30-3,65 (16H) ; 4,10-4,45 (3H) ; 7,55-8,60 (6H).
LC/MS (ESI) : 964,9 ; (calculé ([M+H]⁺) : 964,6).

### Exemple BA5 : molécule BA5

### Molécule B11 : produit obtenu par réaction entre le chlorure de palmitoyle et la L-proline

Par un procédé similaire à celui utilisé pour la préparation de la molécule A26 appliqué au chlorure de palmitoyle (15,39 g, 55,99 mmol) et à la L-proline (12,89 g, 111,98 mmol), un solide blanc de molécule B11 est obtenu.
Rendement : 19,10 g (96%)
RMN ¹H (CDCl₃, ppm) : 0,88 (3H) ; 1,19-1,45 (24H) ; 1,58-1,74 (2H) ; 1,88-2,14 (3H) ; 2,15-2,54 (3H) ; 3,47 (1H) ; 3,58 (1H) ; 4,41 (0,1H) ; 4,61 (0,9H) 6,60-8,60 (1H).
LC/MS (ESI) : 354,4 ; 707,8 ; (calculé ([M+H]⁺) : 354,3 ; ([2M+H]⁺) : 707,6).

### Molécule B12 : produit obtenu par réaction entre la molécule B11 et la L-Lysine

Par un procédé similaire à celui utilisé pour la préparation de la molécule B1 appliqué à la molécule B11 (19,10 g, 54,02 mmol) et à la L-lysine (4,15 g, 28,36 mmol), un résidu huileux est obtenu après concentration du milieu réactionnel sous pression réduite. Ce résidu est dilué dans de l'eau (150 mL), lavé à l'acétate d'éthyle (2 x 75 mL) puis la phase aqueuse est acidifiée jusqu'à pH 1 par addition lente de HCl 6 N. Le produit est extrait 3 fois au dichlorométhane, la phase organique est séchée sur Na₂SO₄ puis filtrée et concentrée sous pression réduite pour donner 11,2 g de résidu huileux jaune. Parallèlement, la phase organique d'acétate d'éthyle précédente est lavée avec une solution aqueuse de HCl 2 N (2 x 75 mL), une solution aqueuse saturée en NaCl (75 mL), séchée sur Na₂SO₄, filtrée et concentrée pour donner 10,2 g de résidu huileux jaune. Un solide blanc est obtenu après recristallisation de chacun de ces résidus dans l'acétone.
Rendement : 11,83 g (54%)
RMN ¹H (CDCl₃, ppm) : 0,87 (6H) ; 1,06-2,44 (70H) ; 2,78-2,96 (1H) ; 3,35-3,75 (5H) ; 4,28-4,43 (0,1H) ; 4,43-4,52 (0,2H) ; 4,52-4,61 (1,8H) ; 4,61-4,75 (0,9H) ; 7,74-8,02 (2H).
LC/MS (ESI) : 818,0 ; (calculé ([M+H]⁺) : 818,7).

### Molécule B13 : produit obtenu par couplage entre la molécule B12 et la Boc-éthylènediamine

Par un procédé similaire à celui utilisé pour la préparation de la molécule A27 appliqué à la molécule B12 (18,00 g, 22,02 mmol) en solution dans le THF et à la Boc-éthylènediamine (4,23 g, 26,43 mmol), un solide blanc est obtenu après recristallisation deux fois dans l'acétonitrile
Rendement : 17,5 g (83%)
RMN ¹H (DMSO-d6, ppm) : 0,85 (6H) ; 1,15-2,29 (79H) ; 2,92-3,12 (6H) ; 3,30-3,59 (4H) ; 4,06-4,13 (0,65H) ; 4,16-4,29 (2H) ; 4,38-4,42 (0,35H) ; 6,71-6,76 (1H) ; 7,60-7,69 (1,3H) ; 7,76-7,81 (0,65H) ; 7,93-7,97 (0,35H) ; 8,00-8,04 (0,35H) ; 8,10-8,17 (0,35H).
LC/MS (ESI) : 960,4 ; (calculé ([M+H]⁺) : 960,8).

### Molécule BA5

Par un procédé similaire à celui utilisé pour la préparation de la molécule BA1 appliqué à la molécule B13 (24,4 g, 25,43 mmol), le résidu obtenu après concentration sous vide est solubilisé dans du dichlorométhane (150 mL), la phase organique est lavée 2 fois avec une solution aqueuse de soude 2 M (90 mL). De l'acétonitrile (120 mL) est ajouté et le dichlorométhane est éliminé par concentration sous pression réduite. Le milieu est ensuite laissé au repos pendant 72 h et un solide blanc est obtenu après filtration et rinçage à l'acétonitrile puis séchage sous pression réduite. Cette opération est répétée 4 fois.
Rendement : 14,28 g (65%)
RMN ¹H (DMSO-d6, ppm) : 0,85 (6H) ; 1,06-2,32 (70H) ; 2,53-2,63 (2H) ; 2,89-3,61 (10H) ; 4,04-4,43 (3H) ; 7,55-7,62 (0,65H) ; 7,65-7,72 (0,65H) ; 7,80 (0,65H) ; 7,91 (0,35H) ; 8,03 (0,35H) ; 8,14-8,23 (0,35H).
LC/MS (ESI) : 860,0 ; (calculé ([M+H]⁺) : 860,8).

### Exemple BA6 : molécule BA6

### Molécule B14 : produit obtenu par couplage entre la molécule A26 et l'acide 2,3-diaminopropionique

Par un procédé similaire à celui utilisé pour la préparation de la molécule B1 appliqué à la molécule A26 (80,00 g, 245,78 mmol) et au dichlorhydrate de l'acide 2,3-diaminopropionique (22,84 g, 129,04 mmol), un solide blanc est obtenu après recristallisation dans l'acétonitrile.
Rendement : 69 g (78%)
RMN ¹H (DMSO-d6, ppm) : 0,86 (6H) ; 1,08-1,38 (40H) ; 1,40-1,55 (4H) ; 1,68-2,30 (12H) ; 3,16-3,66 (6H) ; 4,20-4,39 (3H) ; 7,67-8,31 (2H) ; 12,70 (1H).
LC/MS (ESI) : 719,4 ; 741,5 ; (calculé ([M+H]⁺) : 719,6 ; ([M+Na]⁺) : 741,6).

### Molécule B15 : produit obtenu par couplage entre la molécule B14 et la Boc-éthylènediamine

Par un procédé similaire à celui utilisé pour la préparation de la molécule A27 appliqué à la molécule B14 (32,00 g, 44,50 mmol) en solution dans le dichlorométhane et à la Boc-éthylènediamine (8,56 g, 53,40 mmol), une huile incolore est obtenue après purification par chromatographie sur gel de silice (acétate d'éthyle, méthanol).
Rendement : 24,5 g (64%)
RMN ¹H (DMSO-d6, ppm) : 0,85 (6H) ; 1,16-2,42 (65H) ; 2,89-3,14 (4H) ; 3,17-3,66 (6H) ; 4,11-4,43 (3H) ; 6,77 (1H) ; 7,38-8,23 (3H).
LC/MS (ESI) : 861,7 ; (calculé ([M+H]⁺) : 861,7).

### Molécule BA6

Par un procédé similaire à celui utilisé pour la préparation de la molécule BA5 appliqué à la molécule B15 (24,50 g, 28,45 mmol), un solide blanc est obtenu après recristallisation dans l'acétonitrile.
Rendement : 19,7 g (91%)
RMN ¹H (DMSO-d6, ppm) : 0,85 (6H) ; 1,10-2,40 (58H) ; 2,51-2,62 (2H) ; 2,90-3,16 (2H) ; 3,16-3,67 (6H) ; 4,04-4,47 (3H) ; 7,33-8,27 (3H).
LC/MS (ESI) : 761,5 ; (calculé ([M+H]⁺) : 761,6).

### Exemple BA7 : molécule BA7

### Molécule B16 : produit obtenu par la réaction entre le N-(tert-butoxycarbonyl)-1,6-diaminohexane et la molécule B8.

Par un procédé similaire à celui utilisé pour la préparation de la molécule A27 appliqué à la molécule B8 (10 g, 13,14 mmol) et au *N*-(*tert*-butoxycarbonyl)-1,6-diaminohexane (3,41 g, 15,77 mmol) dans le dichlorométhane, un solide blanc est obtenu après recristallisation dans l'acétonitrile.
Rendement : 10,7 g (85%)
RMN ¹H (CDCl₃, ppm) : 0,88 (6H) ; 1,17-2,40 (79H) ; 3,00-3,71 (10H) ; 4,26-4,58 (3H) ; 4,67 (1H) ; 6,74 (1H) ; 7,34-7,49 (2H).
LC/MS (ESI) : 959,9 ; (calculé ([M+H]⁺) : 959,8).

### Molécule BA7

Après un procédé similaire à celui utilisé pour la préparation de la molécule BA1 appliqué à la molécule B16 (10,5 g, 10,94 mmol), une solution aqueuse de NaOH 2 N est ajoutée goutte à goutte au milieu réactionnel refroidi à 0°C. La phase aqueuse est extraite au dichlorométhane puis la phase organique est lavée 3 fois avec une solution aqueuse de NaCl 5%. Après séchage sur Na₂SO₄, la phase organique est filtrée, concentrée sous vide et le résidu est recristallisé dans l'acétonitrile.
Rendement : 5,4 g (58%)
RMN ¹H (CDCl₃, ppm) : 0,88 (6H) ; 1,19-2,40 (72H) ; 2,67 (2H) ; 3,03-3,70 (8H) ; 4,26-4,57 (3H) ; 6,71 (1H) ; 7,39-7,49 (2H).
LC/MS (ESI) : 859,8 ; (calculé ([M+H]+) : 859,7).

### BB : Synthèse des copolyaminoacides

### Co-polyaminoacides staistaiques de formules VII ou VIIa

**Tableau le**

| **n°** | **co-polyaminoacides porteurs de charges carboxylates et de radicaux hydrophobes** |
|---|---|
| BB1 | 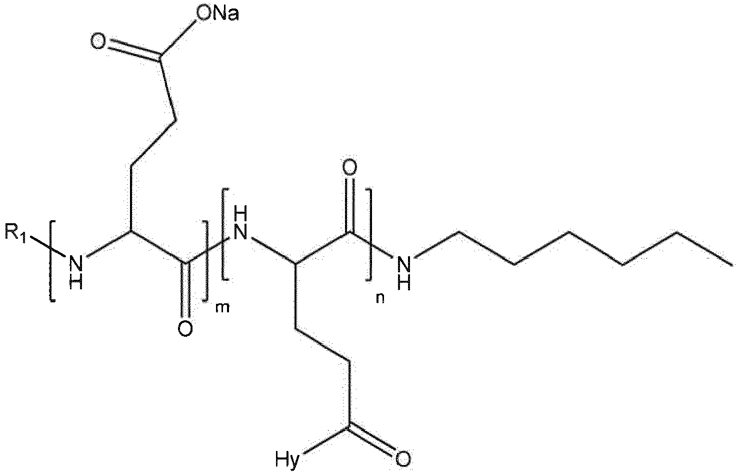 |
| | i = 0,05, DP (m + n) = 23 |
| | 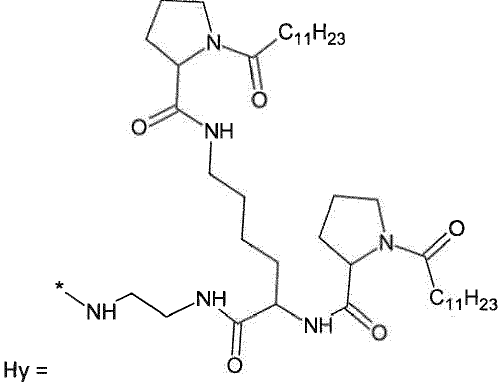 |
| | R₁ = H ou pyroglutamate |
| BB2 | 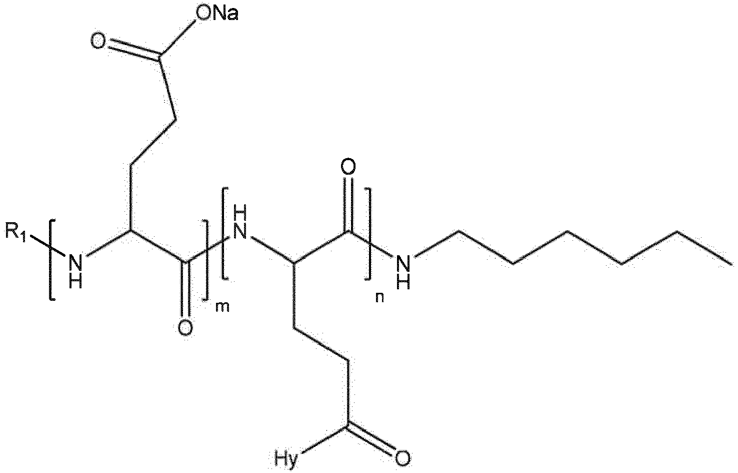 |
| | i = 0,047, DP (m + n) = 21 |
| | 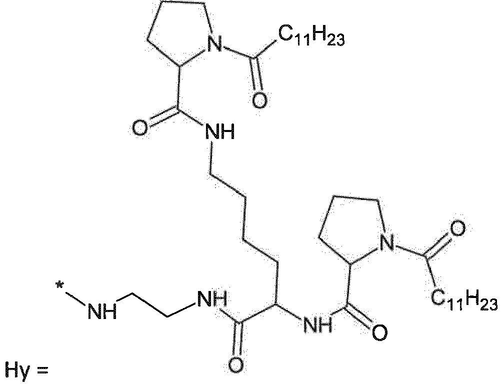 |
| | R₁ = H ou pyroglutamate |
| **BB3** | 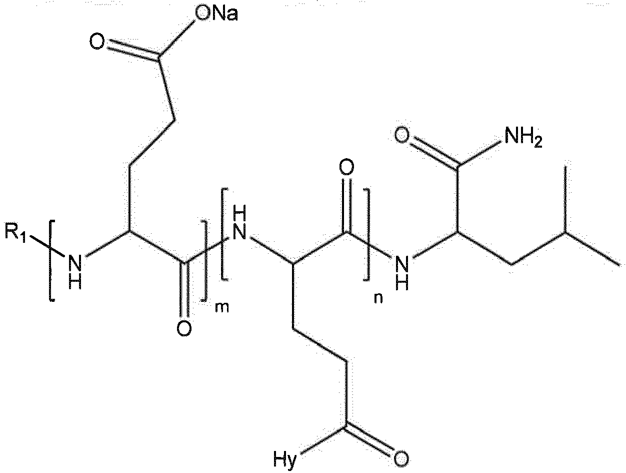 |
| | i = 0,049, DP (m + n) = 34 |
| | 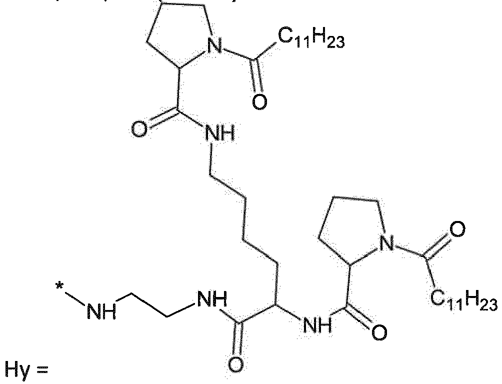 |
| | R₁ = H ou pyroglutamate |
| **BB4** | 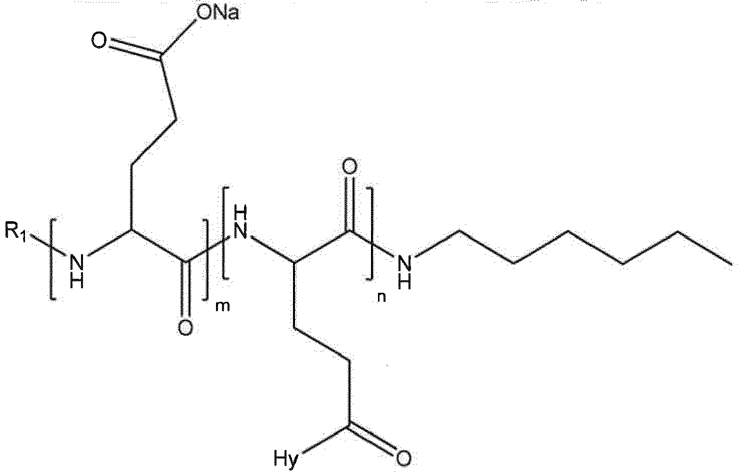 |
| | i = 0,04, DP (m + n) = 65 |
| | 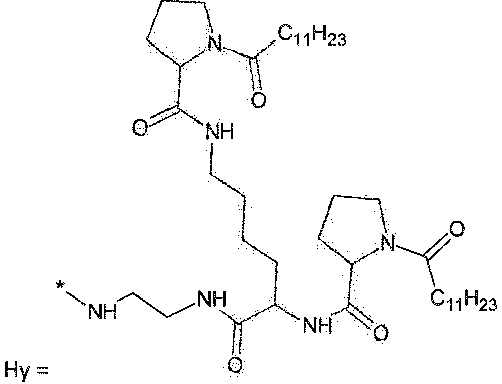 |
| | R₁ = H ou pyroglutamate |
| **BB5** | 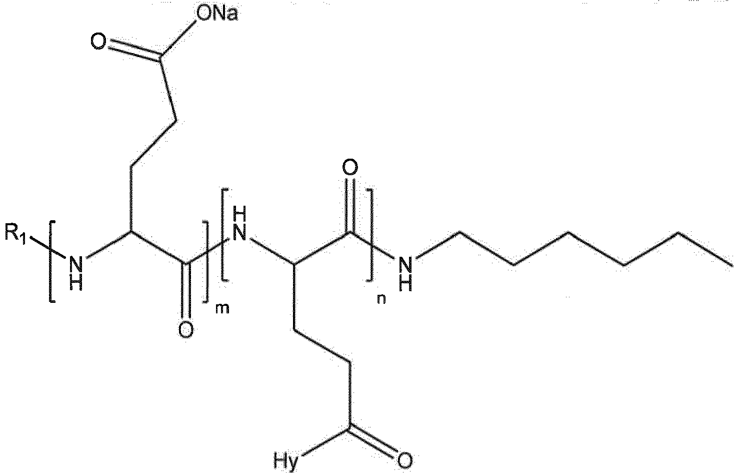 |
| | i = 0,042, DP (m + n) = 23 |
| | 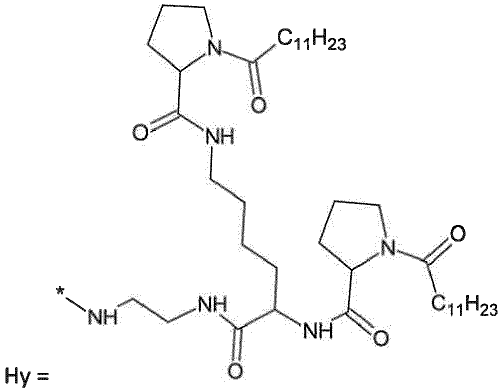 |
| | R1= CH₃-CO-, H ou pyroglutamate |
| **BB6** | 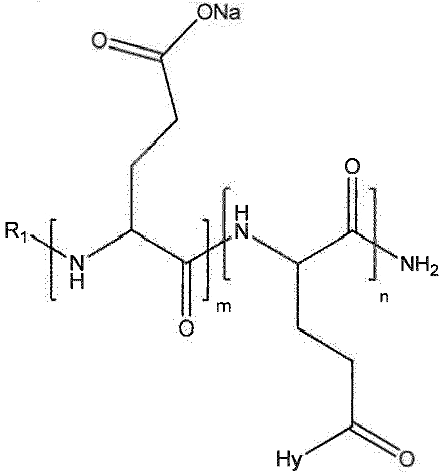 |
| | i = 0,04, DP (m + n) = 24 |
| | 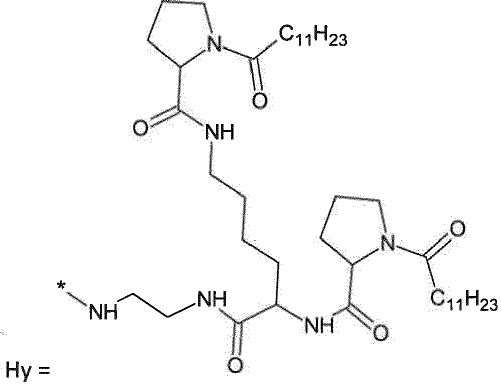 |
| | R1= CH₃-CO-, H ou pyroglutamate |
| **BB7** | 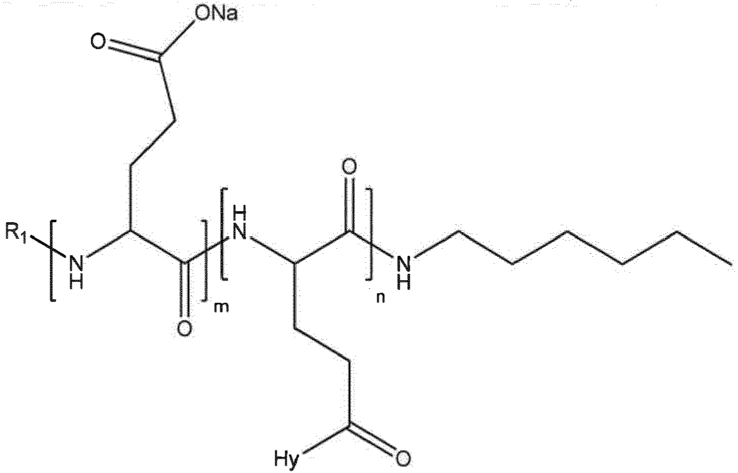 |
| | i = 0,042, DP (m + n) = 22 |
| | 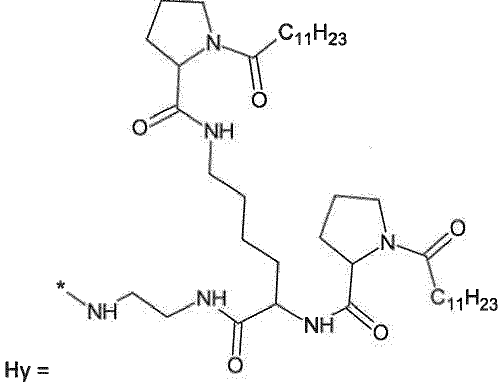 |
| | R₁ = H ou pyroglutamate |
| **BB8** | 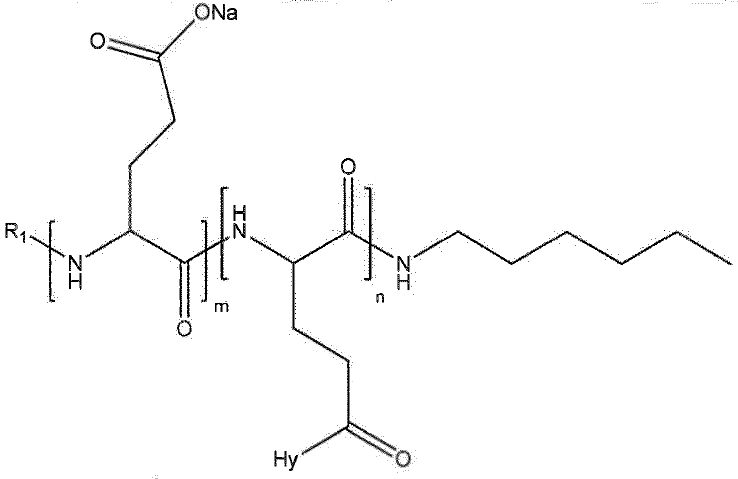 |
| | i = 0,026, DP (m + n) = 21 |
| | 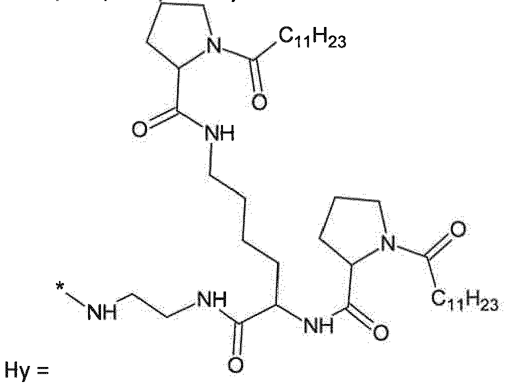 |
| | R₁ = H ou pyroglutamate |
| **BB9** | 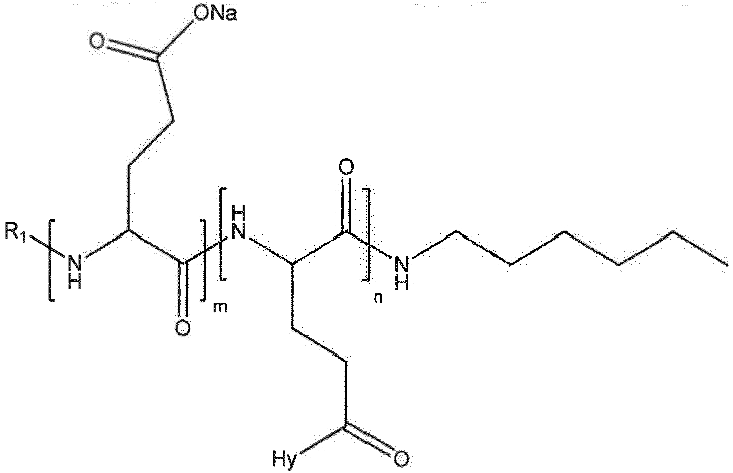 |
| | i = 0,05, DP (m + n) = 26 |
| | 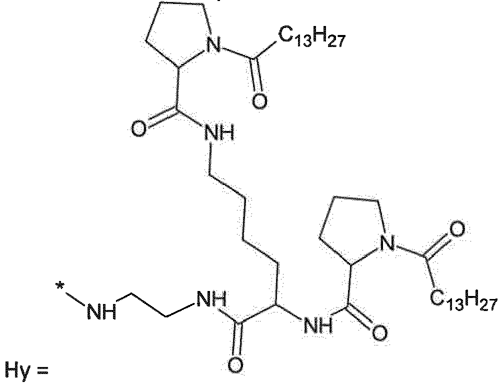 |
| | R₁ = H ou pyroglutamate |
| **BB10** | 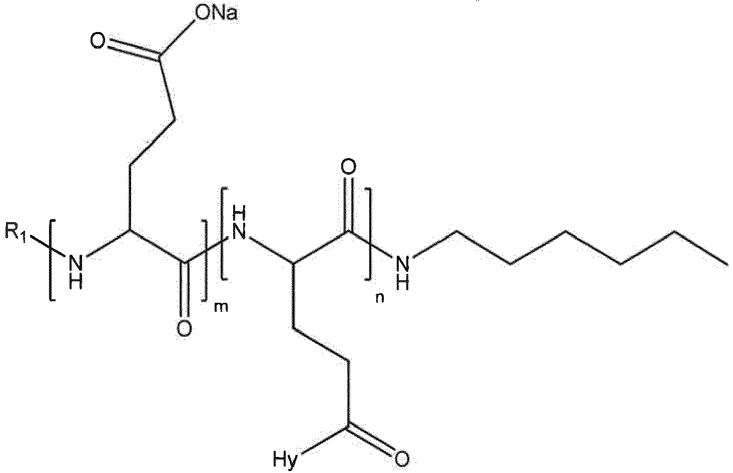 |
| | i = 0,029, DP (m + n) = 22 |
| | 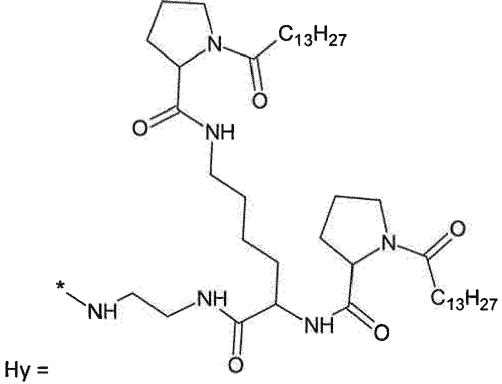 |
| | R₁ = H ou pyroglutamate |
| **BB11** | 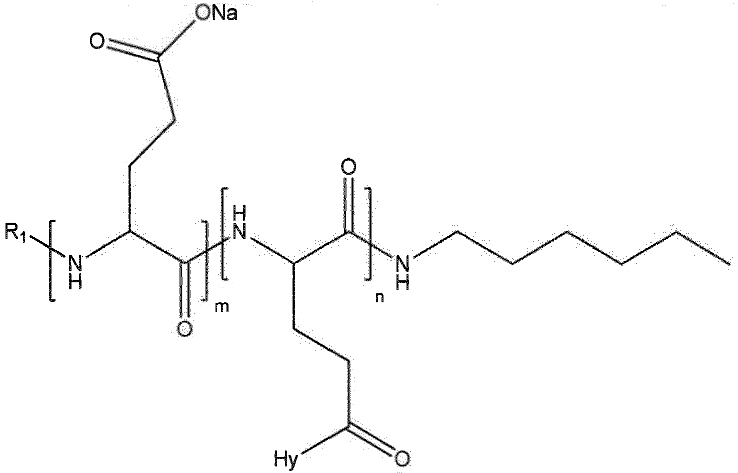 |
| | i = 0,032, DP (m + n) = 22 |
| | 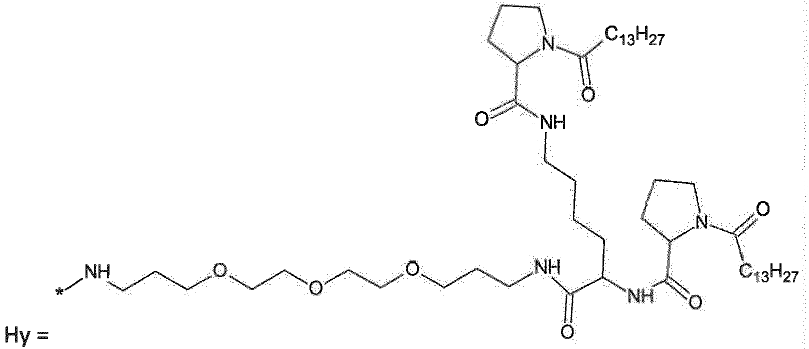 |
| | R1= CH₃-CO-, H ou pyroglutamate |
| **BB12** | 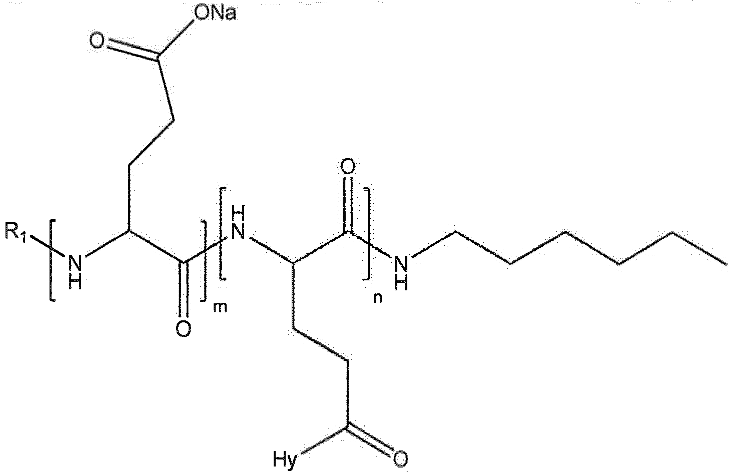 |
| | i = 0,03, DP (m + n) = 23 |
| | 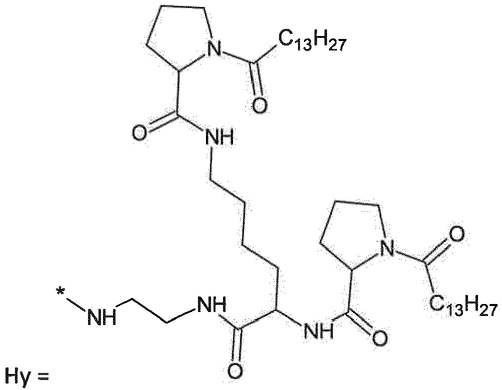 |
| | R1= CH₃-CO-, H ou pyroglutamate |
| **BB13** | 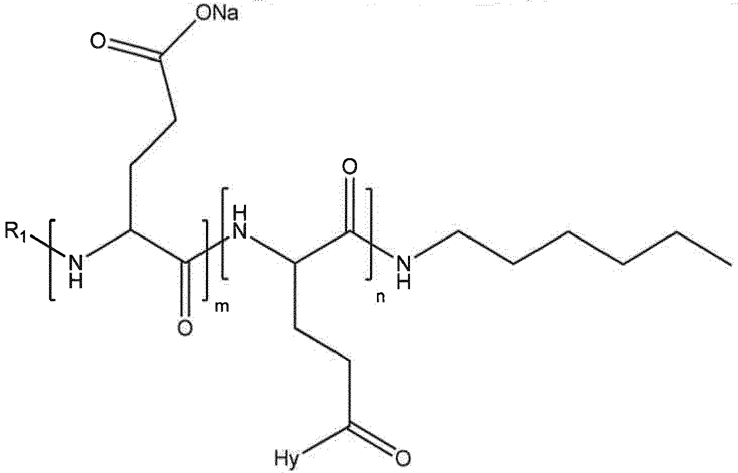 |
| | i = 0,08, DP = 25 |
| | 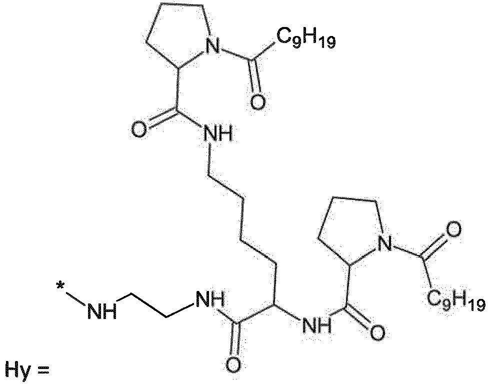 |
| | R₁ = H ou pyroglutamate |

### Part BB : synthèse des co-polyaminoacides

### Exemple BB1 : Co-polyaminoacide BB1 - poly-L-glutamate de sodium modifié par la molécule BA2 et ayant une masse molaire moyenne en nombre (Mn) de 2400 g/mol

### Co-polyaminoacide BB1-1 : acide poly-L-glutamique de masse molaire moyenne en nombre (Mn) relative 3860 g/mol issu de la polymérisation du γ-benzyl-L-glutamate N-carboxyanhydride initiée par l'hexylamine

Dans un ballon préalablement séché à l'étuve est placé sous vide du γ-benzyl-L-glutamate *N*-carboxyanhydride (90,0 g, 342 mmol) pendant 30 min, puis du DMF anhydre (465 mL) est introduit. Le mélange est alors agité sous argon jusqu'à complète dissolution, refroidi à 4°C, puis de l'hexylamine (1,8 mL 14 mmol) est introduite rapidement. Le mélange est agité entre 4°C et température ambiante pendant 2 jours. Le milieu réactionnel est ensuite chauffé à 65°C pendant 4 h, refroidi à température ambiante puis coulé goutte à goutte dans du diisopropyléther froid (6 L) sous agitation. Le précipité blanc est récupéré par filtration, lavé avec du diisopropyléther (500 mL puis 250 mL) puis séché sous vide à 30°C pour donner un acide poly(γ-benzyl-L-glutamique) (PBLG).

A une solution de PBLG (42,1 g) dans l'acide trifluoroacétique (TFA, 325 mL) à 4°C est ajoutée goutte à goutte une solution d'acide bromhydrique (HBr) à 33% dans l'acide acétique (135 mL, 0,77 mol). Le mélange est agité à température ambiante pendant 2 h, puis coulé goutte à goutte sur un mélange 1:1 (v/v) de diisopropyléther et d'eau sous agitation (1,6 L). Après 1 h 30 d'agitation, le mélange hétérogène est laissé au repos pendant une nuit. Le précipité blanc est récupéré par filtration, lavé avec un mélange 1:1 (v/v) de diisopropyléther et d'eau (200 mL).

Le solide obtenu est alors solubilisé dans de l'eau (1 L) en ajustant le pH à 7 par ajout d'une solution aqueuse de soude 10 N puis une solution aqueuse de soude 1 N. Après solubilisation, la concentration théorique est ajustée à 25 g/L théorique par addition d'eau pour obtenir un volume final de 1,5 L.

La solution est filtrée sur filtre 0,45 µm puis purifiée par ultrafiltration contre une solution de NaCl 0,9%, puis de l'eau jusqu'à ce que la conductimétrie du perméat soit inférieure à 50 µS/cm.

La solution aqueuse est alors acidifiée par ajout de solution d'acide chlorhydrique 37% jusqu'à atteindre un pH de 2. Après 4 h d'agitation, le précipité obtenu est filtré puis séché sous vide à 30°C pour donner un acide poly-L-glutamique de masse molaire moyenne en nombre (Mn) 3860 g/mol par rapport à un standard de polyoxyéthylène (PEG).

### Co-polyaminoacide BB1

Le co-polyaminoacide BB1-1 (10,0 g) est solubilisé dans le DMF (700 mL) à 30-40°C puis refroidi à 0°C. Le sel de chlorhydrate de la molécule BA2 (2,95 g, 3,8 mmol) est mis en suspension dans du DMF (45 mL) et de la triéthylamine (0,39 g, 3,8 mmol) est ensuite ajoutée à cette suspension puis le mélange est légèrement chauffé sous agitation jusqu'à complète dissolution. A la solution de co-polyaminoacide à 0°C, de la *N*-méthylmorpholine (NMM, 7,6 g, 75 mmol) dans le DMF (14 mL) et du chloroformate d'éthyle (ECF, 8,1 g, 75 mmol) sont ajoutés. Après 10 min à 0°C, la solution de molécule BA2 est ajoutée et le milieu maintenu à 30°C durant 1 h. Le milieu réactionnel est coulé goutte-à-goutte sur 6 L d'eau contenant du chlorure de sodium à 15% massique et du HCl (pH 2), puis laissé reposer une nuit. Le précipité est collecté par filtration, lavé par la solution de chlorure de sodium à pH 2 (1 L) et séché sous vide pendant environ 1 h. Le solide blanc obtenu est repris dans de l'eau (600 mL) et le pH est ajusté à 7 par ajout lent d'une solution aqueuse de NaOH 1 N. Le volume est ajusté à 700 mL par ajout d'eau. Après filtration sur filtre 0,45 µm, la solution limpide obtenue est purifiée par ultrafiltration contre une solution de NaCl 0,9% puis de l'eau, jusqu'à ce que la conductimétrie du perméat soit inférieure à 50 µS/cm. Après déchargement, la solution est filtrée sur filtre 0,2 µm et stockée à 2-8°C.
Extrait sec : 19,7 mg/g
DP (estimé d'après la RMN ¹H) : 23
D'après la RMN ¹H : i = 0,05
La masse molaire moyenne calculée du co-polyaminoacide BB1 est de 4350 g/mol. HPLC-SEC aqueuse (calibrant PEG) : Mn = 2400 g/mol.

### Exemple BB2 : co-polyaminoacide BB2 - poly-L-glutamate de sodium modifié par la molécule BA2 et ayant une mase molaire moyenne en nombre (Mn) de 4900 g/mol

Un acide poly-L-glutamique de masse molaire moyenne en nombre (Mn) 4100 g/mol (5,0 g) obtenu par un procédé similaire à celui utilisé pour la préparation du co-polyaminoacide BB1-1 est solubilisé dans le DMF (205 mL) à 30-40°C puis maintenu à cette température. En parallèle, le sel de chlorhydrate de la molécule BA2 (1,44 g, 1,84 mmol) est mis en suspension dans du DMF (10 mL) et de la triéthylamine (0,19 g, 1,84 mmol) est ajoutée, puis le mélange est légèrement chauffé sous agitation jusqu'à complète dissolution. A la solution de co-polyaminoacide dans le DMF, de la NMM (3,7 g, 36,7 mmol), la solution de molécule BA2 puis de la N-oxyde de 2-hydroxypyridine (HOPO, 0,31 g, 2,76 mmol) sont ajoutées successivement. Le milieu réactionnel est alors refroidi à 0°C, puis du EDC (0,53 g, 2,76 mmol) est ajouté et le milieu est remonté à température ambiante durant 3 h. Le milieu réactionnel est coulé au goutte-à-goutte sur 1,55 L d'eau contenant du NaCl à 15% massique et du HCl (pH 2) sous agitation. A la fin de l'ajout, le pH est réajusté à 2 avec une solution de HCl 1 N, et la suspension est laissée reposer une nuit. Le précipité est collecté par filtration, puis rincé par 100 mL d'eau. Le solide blanc obtenu est solubilisé dans 200 mL d'eau par ajout lent d'une solution aqueuse de NaOH 1 N jusqu'à pH 7 sous agitation, puis la solution est filtrée sur filtre 0,45 µm. La solution limpide obtenue est purifiée par ultrafiltration contre une solution de NaCl 0,9% puis de l'eau, jusqu'à ce que la conductimétrie du perméat soit inférieure à 50 µS/cm. La solution obtenue est filtrée sur filtre 0,2 µm et stockée à 2-8°C.
Extrait sec : 16,3 mg/g
DP (estimé d'après la RMN ¹H) : 21
D'après la RMN ¹H : i = 0,047
La masse molaire moyenne calculée du co-polyaminoacide BB2 est de 3932 g/mol. HPLC-SEC aqueuse (calibrant PEG) : Mn = 4900 g/mol.

### Exemple BB3 : Co-polyaminoacide BB3 - poly-L-glutamate de sodium modifié par la molécule BA2 et ayant une masse molaire moyenne en nombre (Mn) de 6400 g/mol

### Co-polyaminoacide BB3-1 : acide poly-L-glutamique de masse molaire moyenne en nombre (Mn) 17500 g/mol issu de la polymérisation du γ-méthyl-L-glutamate N-carboxyanhydride initiée par la L-leucinamide

Un acide poly-L-glutamique de masse moyenne en nombre (Mn) 17500 g/mol relative à un standard polymétacrylate de méthyle (PMMA) est obtenu par polymérisation du γ-méthyl N-carboxyanhydride d'acide glutamique en utilisant la L-leucinamide comme initiateur et en effectuant une déprotection des esters méthyliques par utilisation d'une solution d'acide chlorhydrique à 37% selon le procédé décrit dans la demande de brevet FR-A-2 801 226.

Par un procédé similaire à celui utilisé pour la préparation du co-polyaminoacide BB2 appliqué au sel chlorhydrate de la molécule BA2 (3,23 g, 4,1 mmol) et au co-polyaminoacie BB3-1 (11 g), un poly-L-glutamate de sodium modifié par la molécule BA2 est obtenu.
Extrait sec : 27,5 mg/g
DP (estimé d'après la RMN ¹H) : 34
D'après la RMN ¹H : li = 0,049
La masse molaire moyenne calculée du co-polyaminoacide BB3 est de 6405 g/mol. HPLC-SEC aqueuse (calibrant PEG) : Mn = 6400 g/mol.

### Exemple BB4 : co-polyaminoacide BB4 - poly-L-glutamate de sodium modifié par la molécule BA2 et ayant une masse molaire moyenne en nombre (Mn) de 10500 g/mol

Par un procédé similaire à celui utilisé pour la préparation du co-polyaminoacide BB2 appliqué au sel de chlorhydrate de la molécule BA2 (5 g, 6,35 mmol) et à un acide poly-L-glutamique de masse molaire moyenne en nombre Mn = 10800 g / mol (21,7 g) obtenu par un procédé similaire à celui utilisé pour la préparation du co-polyaminoacide BB1-1, un poly-L-glutamate de sodium modifié par la molécule BA2 est obtenu.
Extrait sec : 28,2 mg/g
DP (estimé d'après la RMN ¹H) : 65
D'après la RMN ¹H : i = 0,04
La masse molaire moyenne calculée du co-polyaminoacide BB4 est de 11721 g/mol. HPLC-SEC aqueuse (calibrant PEG) : Mn = 10500 g/mol.

### Exemple BB5 : Co-polyaminoacide BB5 - poly-L-glutamate de sodium cappé à une de ses extrémités par un groupement acétyle et modifié par la molécule BA2 et ayant une masse molaire moyenne en nombre (Mn) de 3600 g/mol

### Co-polyaminoacide BB5-1 : acide poly-L-glutamique de Mn 3700 g/mol issu de la polymérisation du γ-benzyl-L-glutamate N-carboxyanhydride initiée par l'hexylamine et cappé à une de ses extrémités par un groupement acétyle

Dans un ballon préalablement séché à l'étuve est placé sous vide du γ-benzyl-L-glutamate N-carboxyanhydride (100,0 g, 380 mmol) pendant 30 minutes puis du DMF anhydre (250 mL) est introduit. Le mélange est alors agité sous argon jusqu'à complète dissolution, refroidi à 4°C, puis de l'hexylamine (2,3 mL, 17 mmol) est introduite rapidement. Le mélange est agité entre 4°C et température ambiante pendant 2 jours puis précipité dans du diisopropyléther (3,4 L). Le précipité est récupéré par filtration, lavé deux fois avec du diisopropyléther (225 mL) puis séché pour donner un solide blanc qui est dissout dans 450 mL de THF. A cette solution sont ajoutés successivement de la *N,N*-diisopropyléthylamine (DIPEA, 31 mL, 176 mmol) puis de l'anhydride acétique (17 mL, 176 mmol). Après une nuit d'agitation à température ambiante, la solution est versée lentement dans du diisopropyléther (3 L) sur une durée de 30 min et sous agitation. Après 1 h d'agitation, le précipité est filtré, lavé deux fois avec du diisopropyléther (200 mL) puis séché sous vide à 30°C pour donner un acide poly(γ-benzyl-L-glutamique) cappé à une de ses extrémités par un groupement acétyle.

A une solution du co-polyaminoacide cappé (72 g) dans l'acide trifluoroacétique (TFA, 335 mL) à 4°C est ajoutée goutte à goutte une solution d'acide bromhydrique (HBr) à 33% dans l'acide acétique (235 mL, 1,34 mol). Le mélange est agité à température ambiante pendant 3 h 30, puis coulé goutte à goutte sur un mélange 1:1 (v/v) de diisopropyléther et d'eau sous agitation (4 L). Après 2 h d'agitation, le mélange hétérogène est laissé au repos pendant une nuit. Le précipité blanc est récupéré par filtration, lavé avec un mélange 1:1 (v/v) de diisopropyléther et d'eau (340 mL) puis avec de l'eau (340 mL). Le solide obtenu est alors solubilisé dans de l'eau (1,5 L) en ajustant le pH à 7 par ajout d'une solution aqueuse de soude 10 N puis une solution aqueuse de soude 1 N. Après solubilisation, la concentration théorique est ajustée à 20 g/L théorique par addition d'eau pour obtenir un volume final de 2,1 L. La solution est filtrée sur filtre 0,45 µm puis purifiée par ultrafiltration contre une solution de NaCl 0,9%, puis de l'eau jusqu'à ce que la conductimétrie du perméat soit inférieure à 50 µS/cm. La solution de co-polyaminoacide est ensuite concentrée jusqu'à obtenir un volume final de 1,8 L. La solution aqueuse est alors acidifiée par ajout de solution d'acide chlorhydrique 37% jusqu'à atteindre un pH de 2. Après 4 h d'agitation, le précipité obtenu est filtré, lavé avec de l'eau (330 mL) puis séché sous vide à 30°C pour donner un acide poly-L-glutamique de masse molaire moyenne en nombre (Mn) 3700 g/mol par rapport à un standard de polyoxyéthylène (PEG).

### Co-polyaminoacide BB5

Par un procédé similaire à celui utilisé pour la préparation du co-polyaminoacide BB2 appliqué au sel chlorhydrate de la molécule BA2 (6,92 g, 8,8 mmol) et au co-polyaminoacide BB5-1 (30,0 g), un poly-L-glutamate de sodium cappé à une de ses extrémités par un groupement acétyle et modifié par la molécule BA2 est obtenu.
Extrait sec : 29,4 mg/g
DP (estimé d'après la RMN ¹H) : 23
D'après la RMN ¹H : i = 0,042
La masse molaire moyenne calculée du co-polyaminoacide BB5 est de 4302 g/mol. HPLC-SEC aqueuse (calibrant PEG) : Mn = 3600 g/mol.

### Exemple BB6 : Co-polyaminoacide BB6 - poly-L-glutamate de sodium cappé à une de ses extrémités par un groupement acétyle et modifié par la molécule BA2 et ayant une masse molaire moyenne en nombre (Mn) de 4100 g/mol

Par un procédé similaire à celui utilisé pour la préparation du co-polyaminoacide BB2 appliqué au sel chlorhydrate de la molécule BA2 (5,8 g, 7,4 mmol) et à un acide poly-L-glutamique de masse molaire moyenne en nombre Mn = 3800 g/mol (25 g) obtenu par un procédé similaire à celui utilisé pour la préparation du co-polyaminoacide BB5-1 en utilisant l'ammoniac à la place de l'hexylamine, un poly-L-glutamate de sodium cappé à une de ses extrémités par un groupement acétyle et modifié par la molécule BA2 est obtenu.
Extrait sec : 27,6 mg/g
DP (estimé d'après la RMN ¹H) : 24
D'après la RMN ¹H : i = 0,04
La masse molaire moyenne calculée du co-polyaminoacide BB6 est de 4387 g/mol. HPLC-SEC aqueuse (calibrant PEG) : Mn = 4100 g/mol.

### Exemple BB7 : Co-polyaminoacide BB7 - poly-L-glutamate de sodium modifié par la molécule BA2 et ayant une masse molaire moyenne en nombre (Mn) de 4200 g/mol

Par un procédé similaire à celui utilisé pour la préparation du co-polyaminoacide BB2 appliqué au sel chlorhydrate de la molécule BA2 (7,07 g, 9,0 mmol) et à un acide poly-L-glutamique de masse molaire moyenne en nombre Mn = 3600 g/mol (30,0 g) obtenu par un procédé similaire à celui utilisé pour la préparation du co-polyaminoacide BB1-1, un poly-L-glutamate de sodium modifié par la molécule BA2 est obtenu.
Extrait sec : 28,3 mg/g
DP (estimé d'après la RMN ¹H) : 22
D'après la RMN ¹H : i = 0,042
La masse molaire moyenne calculée du co-polyaminoacide BB7 est de 4039 g/mol. HPLC-SEC aqueuse (calibrant PEG) : Mn = 4200 g/mol.

### Exemple BB8 : co-polyaminoacide BB8 - poly-L-glutamate de sodium modifié par la molécule BA2 et ayant une masse molaire moyenne en nombre (Mn) de 5200 g/mol

Par un procédé similaire à celui utilisé pour la préparation du co-polyaminoacide BB2 appliqué au sel chlorhydrate de la molécule BA2 (0,85 g, 1,1 mmol) et à un acide poly-L-glutamique de masse molaire moyenne en nombre Mn = 4100 g/mol (5,0 g) obtenu par un procédé similaire à celui utilisé pour la préparation du co-polyaminoacide BB1-1, un poly-L-glutamate de sodium modifié par la molécule BA2 est obtenu.
Extrait sec : 28,6 mg/g
DP (estimé d'après la RMN ¹H) : 21
D'après la RMN ¹H : i = 0,026
La masse molaire moyenne calculée du co-polyaminoacide BB8 est de 3620 g/mol. HPLC-SEC aqueuse (calibrant PEG) : Mn = 5200 g/mol.

### Exemple BB9 : co-polyaminoacide BB9 - poly-L-glutamate de sodium modifié par la molécule BA3 et ayant une masse molaire moyenne en nombre (Mn) de 4700 g/mol

Par un procédé similaire à celui utilisé pour la préparation du co-polyaminoacide BB2 appliqué au sel chlorhydrate de la molécule BA3 (3,05 g, 3,6 mmol) et à un acide poly-L-glutamique de masse molaire moyenne en nombre Mn = 4100 g/mol (10,0 g) obtenu par un procédé similaire à celui utilisé pour la préparation du co-polyaminoacide BB1-1, un poly-L-glutamate de sodium modifié par la molécule BA3 est obtenu.
Extrait sec : 28,6 mg/g
DP (estimé d'après la RMN ¹H) : 26
D'après la RMN ¹H : i = 0,05
La masse molaire moyenne calculée du co-polyaminoacide BB9 est de 4982 g/mol. HPLC-SEC aqueuse (calibrant PEG) : Mn = 4700 g/mol.

### Exemple BB10 : co-polyaminoacide BB10 - poly-L-glutamate de sodium modifié par la molécule BA3 et ayant une masse molaire moyenne en nombre (Mn) de 4200 g/mol

Par un procédé similaire à celui utilisé pour la préparation du co-polyaminoacide BB2 appliqué au sel chlorhydrate de la molécule BA3 (1,90 g, 2,3 mmol) et à un acide poly-L-glutamique de masse molaire moyenne en nombre Mn = 3500 g/mol (10,0 g) obtenu par un procédé similaire à celui utilisé pour la préparation du co-polyaminoacide BB1-1, un poly-L-glutamate de sodium modifié par la molécule BA3 est obtenu.
Extrait sec : 25,9 mg/g
DP (estimé d'après la RMN ¹H) : 22
D'après la RMN ¹H : i = 0,029
La masse molaire moyenne calculée du co-polyaminoacide BB10 est de 3872 g/mol. HPLC-SEC aqueuse (calibrant PEG) : Mn = 4200 g/mol.

### Exemple BB11 : co-polyaminoacide BB11 - poly-L-glutamate de sodium cappé à une de ses extrémités par un groupement acétyle et modifié par la molécule BA4 et ayant une masse molaire moyenne en nombre (Mn) de 3900 g/mol

Par un procédé similaire à celui utilisé pour la préparation du co-polyaminoacide BB2 appliqué au sel chlorhydrate de la molécule BA4 (2,21 g, 2,2 mmol) et à un acide poly-L-glutamique de masse moyenne en nombre Mn = 3700 g/mol (10 g) obtenu par un procédé similaire à celui utilisé pour la préparation du co-polyaminoacide BB5-1, un poly-L-glutamate de sodium cappé à une de ses extrémités par un groupement acétyle et modifié par la molécule BA4 est obtenu.
Extrait sec : 28,1 mg/g
DP (estimé d'après la RMN ¹H) : 22
D'après la RMN ¹H : i = 0,032
La masse molaire moyenne calculée du co-polyaminoacide BB11 est de 4118 g/mol. HPLC-SEC aqueuse (calibrant PEG) : Mn = 3900 g/mol.

### Exemple BB12 : co-polyaminoacide BB12 - poly-L-glutamate de sodium cappé à une de ses extrémités par un groupement acétyle et modifié par la molécule BA3 et ayant une masse molaire moyenne en nombre (Mn) de 3900 g/mol

Par un procédé similaire à celui utilisé pour la préparation du co-polyaminoacide BB2 appliqué au sel chlorhydrate de la molécule BA3 (1,9 g, 2,3 mmol) et à un acide poly-L-glutamique de masse moyenne en nombre Mn = 3600 g/mol (10 g) obtenu par un procédé similaire à celui utilisé pour la préparation du co-polyaminoacide BB5-1, un poly-L-glutamate de sodium cappé à une de ses extrémités par un groupement acétyle et modifié par la molécule BA3 est obtenu.
Extrait sec : 26,7 mg/g
DP (estimé d'après la RMN ¹H) : 23
D'après la RMN ¹H : i = 0,03
La masse molaire moyenne calculée du co-polyaminoacide BB12 est de 4145 g/mol. HPLC-SEC aqueuse (calibrant PEG) : Mn = 3900 g/mol.

### Exemple BB13 : co-polyaminoacide BB13 - poly-L-glutamate de sodium modifié par la molécule BA1 et ayant une masse molaire moyenne en nombre (Mn) de 2800 g/mol

Par un procédé similaire à celui utilisé pour la préparation du co-polyaminoacide BB1 appliqué au sel de chlorhydrate de la molécule BA1 (3,65 g, 5 mmol) et à un acide poly-L-glutamique de masse molaire moyenne en nombre Mn = 3600 g/mol (10 g) obtenu par un procédé similaire à celui utilisé pour la préparation du co-polyaminoacide BB1-1, un poly-L-glutamate de sodium modifié par la molécule BA1 est obtenu.
Extrait sec : 25,6 mg/g
DP (estimé d'après la RMN ¹H) : 25
D'après la RMN ¹H : i = 0,08
La masse molaire moyenne calculée du co-polyaminoacide BB13 est de 5253 g/mol. HPLC-SEC aqueuse (calibrant PEG) : Mn = 2800 g/mol.

### Partie C :

Le glucagon utilisé est du glucagon humain issu d'un processus de synthèse peptidique. Il provient de la société Bachem (référence 407473).

### Exemple C1 : Solution de Glucagon à 2 mg/ml

Du glucagon (80 mg) en poudre est introduit dans un tube falcon de 45 ml. Une solution aqueuse d'acide chlorhydrique à 0,003 N (40 ml) est ajoutée. La poudre de glucagon est mélangée par des inversions répétées du tube jusqu'à complète dissolution du glucagon. La solution de glucagon à 2 mg/ml est alors filtrée sur membrane (0,22 µm).

### Exemple C2 : Solution de Glucagon à 4 mg/ml

Du glucagon (160 mg) en poudre est introduit dans un tube falcon de 45 ml. Une solution aqueuse d'acide chlorhydrique à 0,006 N (40 ml) est ajoutée. La poudre de glucagon est mélangée par des inversions répétées du tube jusqu'à complète dissolution du glucagon. La solution de glucagon à 4 mg/ml est alors filtrée sur membrane (0,22 µm).

### Exemple C3 : Solution de Glucagon à 6 mg/ml

Du glucagon (240 mg) en poudre est introduit dans un tube falcon de 45 ml. Une solution aqueuse d'acide chlorhydrique à 0,01 N (40 ml) est ajoutée. La poudre de glucagon est mélangée par des inversions répétées du tube jusqu'à complète dissolution du glucagon. La solution de glucagon à 6 mg/ml est alors filtrée sur membrane (0,22 µm).

### Exemple C4 : Solution de Glucagon à 10 mg/ml

Du glucagon (400 mg) en poudre est introduit dans un tube falcon de 45 ml. Une solution aqueuse d'acide chlorhydrique à 0,01 N (40 ml) est ajoutée. La poudre de glucagon est mélangée par des inversions répétées du tube jusqu'à complète dissolution du glucagon. La solution de glucagon à 10 mg/ml est alors filtrée sur membrane (0,22 µm).

Des essais ont été effectués afin de vérifier si les co-polyaminoacides permettent de solubiliser le glucagon, et la concentration minimum de co-polyaminoacide nécessaire pour solubiliser le glucagon a été déterminée.

### Exemple CA2 : Compositions de co-polyaminoacide AB21 à concentrations variables et de glucagon à 1 mg/ml.

X' mg de co-polyaminoacide AB21 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée ci-dessus afin de conduire à une composition comprenant X mg/ml de co-polyaminoacide et 1 mg/ml de glucagon.

Une inspection visuelle est effectuée pour déterminer si l'on obtient ou non une solution limpide. Le résultat de la concentration minimum est présenté en Tableau 5.

### Exemple CA4 : Compositions de co-polyaminoacide BB2 à concentrations variables et de glucagon à 1 mg/ml.

De la même manière que décrite dans l'exemple CA2, des compositions comprenant X mg/ml de co-polyaminoacide BB2 et 1 mg/ml de glucagon sont préparées.

Une inspection visuelle est effectuée pour déterminer si l'on obtient ou non une solution limpide. Le résultat de la concentration minimum est présenté en Tableau 5.

### Exemple CA6 : Compositions de co-polyaminoacide BB9 à concentrations variables et de glucagon à 1 mg/ml.

De la même manière que décrite dans l'exemple CA2, des compositions comprenant X mg/ml de co-polyaminoacides BB9 et 1 mg/ml de glucagon sont préparées.
Une inspection visuelle est effectuée pour déterminer si l'on obtient ou non une solution limpide. Le résultat de la concentration minimum est présenté en Tableau 5.

**Tableau 5 : Concentration minimale en co-polyaminoacide (en mg/ml) pour la solubilisation du glucagon humain (1 mg/mL).**

| **Exemple** | **Co-polyaminoacide** | **Concentration minimale (x mg/ml) en co-polyaminoacide (en mg/ml) pour la solubilisation du glucagon humain (1 mg/mL)** |
|---|---|---|
| CA2 | AB21 | ≤ 1,25 |
| CA4 | BB2 | ≤ 0,82 |
| CA6 | BB9 | ≤ 1,25 |

Des gammes de concentration ont été effectuées avec des co-polyaminoacides selon l'invention et ont conduit à l'obtention des solutions stables suivantes.

### Exemple CB2 : Solution de co-polyaminoacide AB1 à 15 mg/ml et de glucagon à 1 mg/ml

60 mg de co-polyaminoacide AB1 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide AB1 telle que préparée ci-dessus. Puis trois échantillons d'1 ml chacun de cette solution sont préparés et placés en conditions statiques à 37°C.

### Exemple CB3 : Solution de co-polyaminoacide AB5 à 10 mg/ml et de glucagon à 1 mg/ml

40 mg de co-polyaminoacide AB5 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée ci-dessus. Puis trois échantillons d'1 ml chacun de cette solution sont préparés et placés en conditions statiques à 37°C.

### Exemple CB4 : Solution de co-polyaminoacide AB7 à 8,6 mg/ml et de glucagon à 1 mg/ml

34,4 mg de co-polyaminoacide AB7 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée ci-dessus. Puis trois échantillons d'1 ml chacun de cette solution sont préparés et placés en conditions statiques à 37°C.

### Exemple CB8 : Solution de co-polyaminoacide BB2 à 8 mg/ml et de glucagon à 1 mg/ml

32 mg de co-polyaminoacide BB2 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée ci-dessus. Puis trois échantillons d'1 ml chacun de cette solution sont préparés et placés en conditions statiques à 37°C.

### Exemple CB9 : Solution de co-polyaminoacide BB5 à 9 mg/ml et de glucagon à 1 mg/ml

36 mg de co-polyaminoacide BB5 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée ci-dessus. Puis trois échantillons d'1 ml chacun de cette solution sont préparés et placés en conditions statiques à 37°C.

### Exemple CB10 : Solution de co-polyaminoacide BB7 à 15,4 mg/ml et de glucagon à 1 mg/ml

61,6 mg de co-polyaminoacide BB7 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée dessus. Puis trois échantillons d'1 ml chacun de cette solution sont préparés et placés en conditions statiques à 37°C.

### Exemple CB11 : Solution de co-polyaminoacide BB8 à 7,6 mg/ml et de glucagon à 1 mg/ml

30,4 mg de co-polyaminoacide BB8 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée dessus. Puis trois échantillons d'1 ml chacun de cette solution sont préparés et placés en conditions statiques à 37°C.

### Exemple CB12 : Solution de co-polyaminoacide BB9 à 4,3 mg/ml et de glucagon à 1 mg/ml

17,2 mg de co-polyaminoacide BB9 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée ci-dessus. Puis trois échantillons d'1 ml chacun de cette solution sont préparés et placés en conditions statiques à 37°C.

### Exemple CB13 : Solution de co-polyaminoacide BB11 à 5,9 mg/ml et de glucagon à 1 mg/ml

23,6 mg de co-polyaminoacide BB11 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée dessus. Puis trois échantillons d'1 ml chacun de cette solution sont préparés et placés en conditions statiques à 37°C.

### Exemple CB14 : Solution de co-polyaminoacide BB11 à 8,6 mg/ml et de glucagon à 1 mg/ml

34,4 mg de co-polyaminoacide BB11 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée dessus. Puis trois échantillons d'1 ml chacun de cette solution sont préparés et placés en conditions statiques à 37°C.

### Exemple CB25 : Solution de co-polyaminoacide AB21 à 8,6 mg/ml et de glucagon à 1 mg/ml

34,4 mg de co-polyaminoacide AB21 sont pesés précisément, et ajouté à 2 ml d'une solution de tampon phosphate à 10 mM comprenant du m-crésol (46 mM), du glycérol (548 mM). La composition est agitée jusqu'à dissolution du co-polyaminoacide, puis la solution est filtrée sur membrane (0,22 µm).

2 ml d'une solution de glucagon telle que préparée à l'exemple C1 sont mélangés à 2 ml de la solution de co-polyaminoacide telle que préparée dessus. Puis trois échantillons d'1 ml chacun de cette solution sont préparés et placés en conditions statiques à 37°C.

### Partie C' - formulations contre-exemple

Le bromure de cétyltriméthylammonium (CTAB) provient de la société Sigma-Aldrich (ref A6909).

Le dodécylmaltoside (DDM) provient de la société Sigma-Aldrich (ref : D4641).

Le nom (mPEG-DSPE 2000) provient de la société Interchim (ref : KV5081).

Le myristoyl lysophosphatidyl choline (LMPC) provient de la société Combi-Block (ref : QE-2488).

De manière analogue à la composition décrite dans l'exemple CA2, les compositions CEC1 à CEC10 ont également été préparées de manière à obtenir les concentrations désirées (tableau 6d).

**Tableau 6d: compositions de glucagon à 1 mg/ml à pH 7,2 en présence de produits commerciaux à différentes concentrations.**

| **Composi tions** | **Glucag on (mg/ml )** | **Produits de l'art antérieur** | **concentratio n (mg/ml)** | **Tampon Phosphate (mM)** | **pH** |
|---|---|---|---|---|---|
| CEC1 | 1 | CTAB | 0,3 | 2 | 7,13 |
| CEC2 | 1 | CTAB | 15,7 | 2 | 7,14 |
| CEC3 | 1 | CTAB | 39,3 | 2 | 7,16 |
| CEC4 | 1 | DDM | 0,4 | 2 | ND |
| CEC5 | 1 | DDM | 3,2 | 2 | 7,15 |
| CEC6 | 1 | DDM | 10,7 | 2 | 7,34 |
| CEC7 | 1 | PEG-DSPE | 2,4 | 2 | 7,20 |
| CEC8 | 1 | PEG-DSPE | 10,9 | 2 | 7,12 |
| CEC9 | 1 | LMPC | 0,4 | 2 | ND |
| CEC10 | 1 | LMPC | 2,1 | 2 | 7,13 |

### Partie D : STABILITE

### Exemple D1 : Stabilité physique de compositions co-polyaminoacide/glucagon

L'inspection visuelle des échantillons placés en conditions statiques à 37°C est effectuée à 0, 7, 14 et 21 jours à 37°C afin de détecter l'apparition de particules visibles ou d'une turbidité. Cette inspection est réalisée selon les recommandations de la Pharmacopée Européenne (EP 2.9.20) : les échantillons sont soumis à un éclairage d'au moins 2000 Lux et sont observés face à un fond blanc et un fond noir. Quand des particules sont visibles dans au moins 2 des 3 échantillons la composition est estimée non stable. Stable signifie donc qu'au jour de l'inspection au moins 2 échantillons étaient dépourvus de particules.

Les résultats des inspections visuelles sont reportés dans le tableau 7 suivant.

L'étude des stabilités physiques des compositions des exemples CB2 à CEC9 décrites dans le tableau ci-dessous a été menée sur des volumes de 1 ml de composition dans des flacons de contenance de 3 ml (Adelphi - ref :VCDIN2RDLS1).

**Tableau 7 : Résultats des inspections visuelles de compositions comprenant un co-polyaminoacide et du glucagon**

| Exemple | Co-Polyaminoacide et produits de l'art antérieur (mg/ml) | Glucag on (mg/ml ) | Exenatide (mg/ml) | L-méthionine (mg/ml) | Stable à 7 j | Stable à 14 j | Stable à 21 j |
|---|---|---|---|---|---|---|---|
| CB2 | AB1 (15) | 1 | 0 | 0 | oui | non | non |
| CB3 | AB5 (10) | 1 | 0 | 0 | oui | oui | oui |
| CB4 | AB7 (8,6) | 1 | 0 | 0 | oui | oui | oui |
| CB8 | BB2 (8) | 1 | 0 | 0 | oui | oui | oui |
| CB9 | BB5 (9) | 1 | 0 | 0 | oui | oui | non |
| CB10 | BB7 (15,4) | 1 | 0 | 0 | oui | oui | oui- |
| CB11 | BB8 (7,6) | 1 | 0 | 0 | oui | non | non |
| CB12 | BB9 (4,3) | 1 | 0 | 0 | oui | oui | oui |
| CB13 | BB11 (5,9) | 1 | 0 | 0 | oui | oui | non |
| CB14 | BB11 (8,6) | 1 | 0 | 0 | oui | oui | oui |
| CB25 | AB21 (8,6) | 1 | 0 | 0 | oui | oui | oui |
| CB26 | AB24 (6,1) | 1 | 0 | 0 | oui | oui | oui |
| CB27 | AB24 (3,0) | 1 | 0 | 0 | oui | oui | oui |
| CB30 | AB25 (6,7) | 1 | 0 | 0 | oui | oui | oui |
| CB31 | AB28(6,9) | 1 | 0 | 0 | oui | oui | - |
| CEC1 | CTAB (0,3) | 1 | 0 | 0 | Non | - | - |
| CEC6 | DDM (10,7) | 1 | 0 | 0 | Non | - | - |
| CEC7 | mPEG-DSPE (2,4) | 1 | 0 | 0 | Non | - | - |
| CEC9 | LMPC (0,4) | 1 | 0 | 0 | Non | - | - |
| CEC10 | LPMC (2,1) | 1 | 0 | 0 | Non | - | - |

| | | | | | | | |
|---|---|---|---|---|---|---|---|
| « - » signifie non observé | | | | | | | |

### Exemple D2 : Stabilité chimique de compositions co-polyaminoacide/glucagon

Une méthode RP-HPLC adaptée à partir des directives USP a été utilisée pour déterminer la concentration de glucagon et de ses produits de dégradation. Cette méthode a été utilisée pour évaluer la stabilité chimique du glucagon des compositions. Les conditions HPLC sont les suivantes :
- Colonne : 4,6 x 150 mm, C-18
- Phase mobile A : Solution S/Acétonitrile 80/20 (v/v), la solution S étant une solution de dihydrogénophosphate de potassium 150 mM dans l'eau, ajustée à pH 2,7 avec une solution d'acide phosphorique à 85%
- Phase mobile B : eau/acétonitrile 60/40 (v/v)
- Phase mobile C : eau/acétonitrile 10/90 (v/v)
- Température de colonne : 45°C
- Détection : UV 210 nm
- Température de l'autoéchantillonneur : 4°C

La recouvrance a été mesurée sur des échantillons à 7, 14 et 21 jours à 37°C en conditions statiques. Les données de stabilités chimiques, c'est-à-dire de recouvrance en glucagon obtenues par RP-HPLC sont présentées dans le tableau 8 suivant.

L'étude des stabilités chimiques des compositions décrites dans le tableau ci-dessous a été menée sur des compositions dans des flacons (1 ml de composition dans flacon de contenance de 3 ml (Adelphi - ref :VCDIN2RDLS1)).

**Tableau 8 : Mesures de recouvrance de compositions comprenant un co-polyaminoacide et du glucagon**

| Exemple | Co-Polyaminoacide (mg/ml) | Glucagon (mg/ml) | Exenatide (mg/ml) | L-méthionine (mg/ml | Recouv rance à 7 j | Recouv rance à 14 j | Recouv rance à 21 j |
|---|---|---|---|---|---|---|---|
| CB4 | AB7 (8,6) | 1 | 0 | 0 | ≥ 95 | ≥ 90 | ≥ 85 |
| CB8 | BB2 (8) | 1 | 0 | 0 | - | ≥ 90 | ≥ 90 |
| CB10 | BB7 (15,4) | 1 | 0 | 0 | ≥ 95 | ≥ 90 | - |
| CB14 | BB11 (8,6) | 1 | 0 | 0 | ≥ 95 | ≥ 90 | ≥ 90 |
| CB25 | AB21 (8,6) | 1 | 0 | 0 | - | ≥ 90 | ≥ 85 |
| CB30 | AB25 (6,7) | 1 | 0 | 0 | - | ≥ 90 | - |
| CEC2 | CTAB | 1 | 0 | 0 | - | <50 | |

| | | | | | | | |
|---|---|---|---|---|---|---|---|
| « - » signifie non mesuré | | | | | | | |

Résultats des observations visuelles au mélange et des mesures de fibrillation par ThT

### Principe

La mauvaise stabilité d'un peptide peut conduire à la formation de fibrilles amyloïdes, définies comme des structures macromoléculaires ordonnées. Celles-ci peuvent éventuellement résulter à la formation de gel au sein de l'échantillon.

L'essai de suivi de la fluorescence de la thioflavine T (ThT) est utilisé pour analyser la stabilité physique des solutions. La Thioflavine est une petite molécule sonde ayant une signature de fluorescence caractéristique lorsque se lie à des fibrilles de type amyloïdes (Naiki et al. (1989) Anal. BioChem. 177, 244-249 ; LeVine (1999) Methods. Enzymol. 309, 274-284).

Cette méthode permet de suivre la formation de fibrilles pour de faibles concentrations de ThT au sein de solutions non diluées. Ce suivi est réalisé dans des conditions de stabilité accélérées : sous agitation et à 37°C.

### Conditions expérimentales

Les échantillons ont été préparés juste avant le début de la mesure. La préparation de chaque composition est décrite dans l'exemple associé. La Thioflavine T a été ajoutée dans la composition à partir d'une solution mère concentrée de manière à induire une dilution négligeable de la composition. La concentration de Thioflavine T dans la composition est de 40 µM.

Un volume de 150 µL de la composition a été introduit au sein d'un puit d'une plaque 96 puits puis 2.7 µL de solution concentrée de ThT a été introduite. Chaque composition a été analysée en en trois essais (triplicat) au sein d'une même plaque. La plaque a été scellée par du film transparent afin d'éviter l'évaporation de la composition.

Cette plaque a ensuite été placée dans l'enceinte d'un lecteur de plaques (EnVision 2104 Multilabel, Perkin Elmer). La température est réglée à 37°C, et une agitation latérale de 960 rpm avec 1 mm d'amplitude est imposée.

Une lecture de l'intensité de fluorescence dans chaque puit est réalisée avec une longueur d'onde d'excitation de 442 nm, et une longueur d'onde d'émission de 482 nm au cours du temps.

Le processus de fibrillation se manifeste par une forte augmentation de la fluorescence après un délai appelé temps de latence.

Le lag time est déterminé visuellement, en prenant en considération le temps où le signal de fluorescence commence à augmenter significativement au dessus de la ligne de base. La valeur de temps de latence reportée correspond à la moyenne des mesures de temps de latence faites sur trois puits.

Les résultats sont présentés dans le tableau 9 ci-dessous.

**Tableau 9: résultats des temps de latence obtenus avec les compositions de co-polyaminoacide ou produits commerciaux en présence de 1 mg/ml de glucagon.**

| **Compo sition** | **Glucago n mg/mL** | **BC** | **Concentrati on massique mg/mL** | **Temps de latence en heures** |
|---|---|---|---|---|
| CB26 | 1 | AB24 | 6,1 | >68 |
| CB30 | 1 | AB25 | 6,7 | >68 |
| CB31 | 1 | AB28 | 6,9 | >63 |
| CEC7 | 1 | mPEG-DSPE | 2,4 | 2,6 |
| CEC9 | 1 | LMPC | 0,4 | 3,7 |

Les temps de latence obtenus avec les compositions de co-polyaminoacide en présence de glucagon sont supérieurs à ceux des formulations des produits commerciaux en présence de glucagon.

## Revendications

1. Composition sous forme d'une solution aqueuse injectable, dont le pH est compris entre 6, 0 et 8,0, comprenant au moins :
a) du glucagon humain ;
b) un co-polyaminoacide porteur de charges carboxylates et de radicaux hydrophobes Hy, ledit co-polyaminoacide étant constitué d'unités glutamiques ou aspartiques et lesdits radicaux hydrophobes Hy étant de formule I suivante : 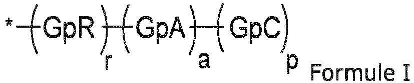 dans laquelle
- GpR est un radical de formules II: 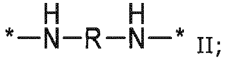
- GpA est un radical de formules III ou III' : 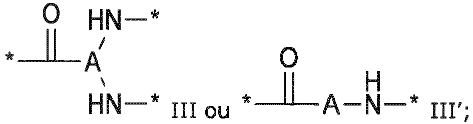
- GpC est un radical de formule IV : 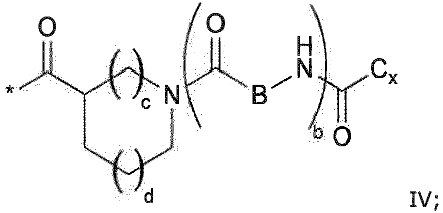
- les * indiquent les sites de rattachement des différents groupes liés par des fonctions amides ;
- a est un entier égal à 0 ou à 1 ;
- b est un entier égal à 0 ou à 1;
- p est un entier égal à 1 ou à 2 et
∘ si p est égal à 1 alors a est égal à 0 ou à 1 et GpA est un radical de formule III' et,
∘ si p est égal à 2 alors a est égal à 1, et GpA est un radical de formule III;
- c est un entier égal à 0 ou à 1, et si c est égal à 0 alors d est égal à 1 ou à 2;
- d est un entier égal à 0, à 1 ou à 2;
- r est un entier égal à 0 ou à 1, et
∘ si r est égal à 0 alors le radical hydrophobe de formule I est lié au co-polyaminoacide via une liaison covalente entre un carbonyl du radical hydrophobe et un atome d'azote en position N terminale du co-polyaminoacide, formant ainsi une fonction amide issue de la réaction d'une fonction amine en position N terminale du précurseur du co-polyaminoacide et une fonction acide portée par le précurseur du radical hydrophobe , et
∘ si r est égal à 1 alors le radical hydrophobe de formule I est lié au co-polyaminoacide :
▪ via une liaison covalente entre un atome d'azote du radical hydrophobe et un carbonyl du co-polyaminoacide, formant ainsi une fonction amide issue de la réaction d'une fonction amine du précurseur du radical hydrophobe et une fonction acide portée par le précurseur du co-polyaminoacide ou
▪ via une liaison covalente entre un carbonyl du radical hydrophobe et un atome d'azote en position N terminal du co-polyaminoacide, formant ainsi une fonction amide issue de la réaction d'une fonction acide du précurseur du radical hydrophobe et une fonction amine en position N terminale portée par le précurseur du co-polyaminoacide;
- R est un radical choisi dans le groupe constitué par :
∘ un radical alkyle divalent, linéaire ou ramifié, comprenant si GpR est un radical de formule II de 2 à 12 atomes de carbone;
∘ un radical alkyle divalent, linéaire ou ramifié, comprenant si GpR est un radical de formule II de 2 à 11 atomes de carbone, ledit radical alkyle portant une ou plusieurs fonctions -CONH₂, et
∘ un radical éther ou polyéther non substitué comprenant de 4 à 14 atomes de carbone et de 1 à 5 atomes d'oxygène ;
- A est un radical alkyle linéaire ou ramifié comprenant de 1 à 6 atomes de carbone;
- B est un radical alkyle linéaire ou ramifié, éventuellement comprenant un noyau aromatique, comprenant de 1 à 9 atomes de carbone;
- Cₓ est un radical alkyl monovalent linéaire ou ramifié, dans lequel x indique le nombre d'atomes de carbone et :
∘ si p est égal à 1, x est compris entre 11 et 25 (11 ≤ x ≤ 25) :
∘ si p est égal à 2, x est compris entre 9 et 15 (9 ≤ x ≤ 15),
- le ratio i entre le nombre de radicaux hydrophobes et le nombre d'unités glutamiques ou aspartiques étant compris entre entre 0 < i ≤ 0,5 ;
- lorsque plusieurs radicaux hydrophobes sont portés par un co-polyaminoacide alors ils sont identiques ou différents,
- le degré de polymérisation DP en unités glutamiques ou aspartiques est compris entre 10 et 250 ;
- les fonctions acides libres étant sous forme de sel de cation alkalin choisi dans le groupe constitué par Na⁺ et K⁺.

2. Composition selon la revendication 1, **caractérisée en ce que** lesdits radicaux hydrophobes sont choisis parmi les radicaux hydrophobes de formule I dans laquelle p = 1, représentée par la formule V suivante : 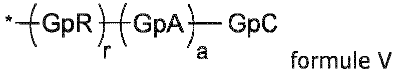 GpR, GpA, GpC, r et a tels que définis dans la revendication 1.

3. Composition selon la revendication 1, **caractérisée en ce que** lesdits radicaux hydrophobes sont choisis parmi les radicaux hydrophobes de formule I dans laquelle a = 1 et p = 2, représentée par la formule VI suivante : 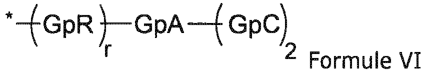 dans laquelle
GpR, GpA, GpC, r et a tels que définis dans la revendication 1.

4. Composition selon l'une quelconque des revendications précédentes, **caractérisée en ce que** le co-polyaminoacide porteur de charges carboxylates et de radicaux hydrophobes est choisi parmi les co-polyaminoacides de formule VIIa suivante : 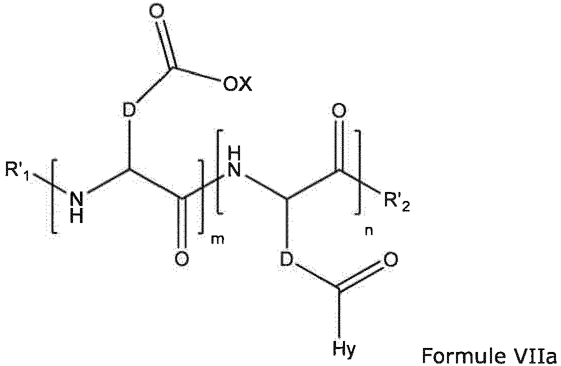 dans laquelle,
• D représente, indépendamment, soit un groupe -CH₂- (unité aspartique) soit un groupe -CH₂-CH₂- (unité glutamique),
• Hy est un radical hydrophobe choisi parmi les radicaux hydrophobes de formules I, V ou VI, dans lesquelles r = 1 et GpR est un radical de Formule II,
• X représente un H ou une entité cationique choisie dans le groupe comprenant les cations métalliques ;
• n + m représente le degré de polymérisation DP du co-polyaminoacide, c'est-à-dire le nombre moyen d'unités monomériques par chaîne de co-polyaminoacide et 5 ≤ n + m ≤ 250 ;R'1 est un radical choisi dans le groupe constitué par un H, un groupe acyle linéaire en C2 à C10, un groupe acyle ramifié en C4 à C10, un benzyle, une unité « acide aminé » terminale et un pyroglutamate,
• R'2 est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S.

5. Composition selon la revendication 4, **caractérisée en ce que** le co-polyaminoacide porteur de charges carboxylates et de radicaux hydrophobes est choisi parmi les co-polyaminoacides de formules VII, dans laquelle R₁ = R'₁ et R₂ = R'₂, de formule VIIa suivante : 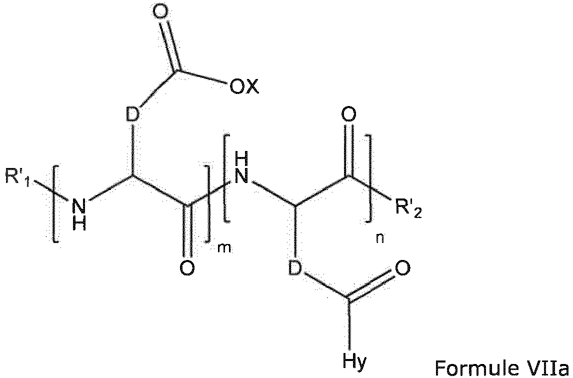 dans laquelle,
- m, n, X, D et Hy tels que définis dans la revendication 4,
- R'₁ est un radical choisi dans le groupe constitué par un H, un groupe acyle linéaire en C2 à C10, un groupe acyle ramifié en C4 à C10, un benzyle, une unité « acide aminé » terminale et un pyroglutamate,
- R'₂ est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S.

6. Composition selon l'une quelconque des revendications 4 et 5, **caractérisée en ce que** le co-polyaminoacide porteur de charges carboxylates et de radicaux hydrophobes est choisi parmi les co-polyaminoacides de formules VII ou VIIa dans lesquels le au moins un co-polyaminoacide est choisi parmi les co-polyaminoacides dans lesquels le groupe D est un groupe -CH₂-CH₂- (unité glutamique).

7. Composition selon l'une quelconque des revendications précédentes, **caractérisée en ce que** la concentration en co-polyaminoacide porteur de charges carboxylates et de radicaux hydrophobes est au plus de 40 mg/mL.

8. Composition selon l'une quelconque des revendications précédentes, **caractérisée en ce que** la concentration en glucagon humain est comprise entre 0,25 et 5 mg/mL.

9. Composition selon l'une quelconque des revendications précédentes, **caractérisée en ce que** le ratio molaire [radical hydrophobe]/[glucagon humain] est inférieur à 15.

10. Composition selon l'une quelconque des revendications précédentes, **caractérisée en ce qu'**elle comprend en outre un composé polyanionique.

11. Composition selon l'une quelconque des revendications précédentes, **caractérisée en ce qu'**elle comprend en outre un sel de zinc.

12. Composition selon l'une quelconque des revendications précédentes, **caractérisée en ce qu'**elle comprend en outre une hormone gastrointestinale.

13. Composition selon la revendication 12, **caractérisée en ce que** l'hormone gastrointestinale est choisie dans le groupe constitué par l'exenatide, le liraglutide, le lixisenatide, l'albiglutide et le dulaglutide et leurs sels pharmaceutiquement acceptables.

14. Composition selon l'une quelconque des revendications 12 et 13, **caractérisée en ce que** la concentration en hormone gastrointestinale est comprise dans un intervalle de 0,01 à 10 mg/mL.

15. Copolyaminoacide porteur de charges carboxylates et de radicaux hydrophobes Hy de formule I suivante : 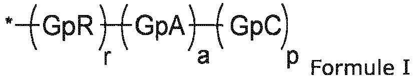 dans laquelle
- GpR est un radical de formules II: 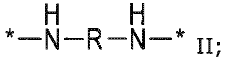
- GpA est un radical de formules III ou III' : 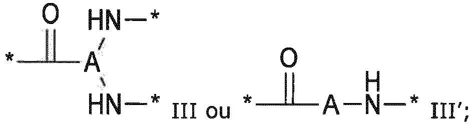
- GpC est un radical de formule IV : 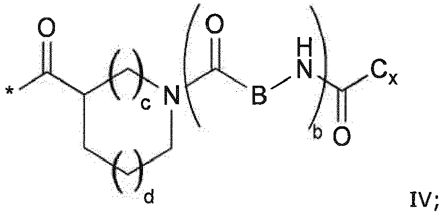
- les * indiquent les sites de rattachement des différents groupes liés par des fonctions amides ;
- a est un entier égal à 0 ou à 1 ;
- b est un entier égal à 0 ou à 1;
- p est un entier égal à 1 ou à 2 et
∘ si p est égal à 1 alors a est égal à 0 ou à 1 et GpA est un radical de formule III' et,
∘ si p est égal à 2 alors a est égal à 1, et GpA est un radical de formule III;
- c est un entier égal à 0 ou à 1, et si c est égal à 0 alors d est égal à 1 ou à 2;
- d est un entier égal à 0, à 1 ou à 2;
- r est un entier égal à 0 ou à 1, et
∘ si r est égal à 0 alors le radical hydrophobe de formule I est lié au co-polyaminoacide via une liaison covalente entre un carbonyl du radical hydrophobe et un atome d'azote en position N terminale du co-polyaminoacide, formant ainsi une fonction amide issue de la réaction d'une fonction amine en position N terminale du précurseur du co-polyaminoacide et une fonction acide portée par le précurseur du radical hydrophobe , et
∘ si r est égal à 1 alors le radical hydrophobe de formule I est lié au co-polyaminoacide :
▪ via une liaison covalente entre un atome d'azote du radical hydrophobe et un carbonyl du co-polyaminoacide, formant ainsi une fonction amide issue de la réaction d'une fonction amine du précurseur du radical hydrophobe et une fonction acide portée par le précurseur du co-polyaminoacide ou
▪ via une liaison covalente entre un carbonyl du radical hydrophobe et un atome d'azote en position N terminal du co-polyaminoacide, formant ainsi une fonction amide issue de la réaction d'une fonction acide du précurseur du radical hydrophobe et une fonction amine en position N terminale portée par le précurseur du co-polyaminoacide;
- R est un radical choisi dans le groupe constitué par :
∘ un radical alkyle divalent, linéaire ou ramifié, comprenant si GpR est un radical de formule II de 2 à 12 atomes de carbone;
∘ un radical alkyle divalent, linéaire ou ramifié, comprenant si GpR est un radical de formule II de 2 à 11 atomes de carbone, ledit radical alkyle portant une ou plusieurs fonctions -CONH₂, et
∘ un radical éther ou polyéther non substitué comprenant de 4 à 14 atomes de carbone et de 1 à 5 atomes d'oxygène ;
- A est un radical alkyle linéaire ou ramifié comprenant de 1 à 6 atomes de carbone;
- B est un radical alkyle linéaire ou ramifié, éventuellement comprenant un noyau aromatique, comprenant de 1 à 9 atomes de carbone;
- Cₓ est un radical alkyl monovalent linéaire ou ramifié, dans lequel x indique le nombre d'atomes de carbone et :
∘ si p est égal à 1, x est compris entre 11 et 25 (11 ≤ x ≤ 25) :
∘ si p est égal à 2, x est compris entre 9 et 15 (9 ≤ x ≤ 15),
- le ratio i entre le nombre de radicaux hydrophobes et le nombre d'unités glutamiques ou aspartiques étant compris entre entre 0 < i ≤ 0,5 ;
- lorsque plusieurs radicaux hydrophobes sont portés par un co-polyaminoacide alors ils sont identiques ou différents,
- le degré de polymérisation DP en unités glutamiques ou aspartiques est compris entre 10 et 250 ;
- les fonctions acides libres étant sous forme de sel de cation alkalin choisi dans le groupe constitué par Na⁺ et K⁺.

## Patentansprüche

1. Zusammensetzung in Form einer injizierbaren wässrigen Lösung, deren pH von 6,0 bis 8,0 beträgt, wenigstens umfassend:
a) menschliches Glucagon;
b) eine Co-Polyaminosäure, die Carboxylat-Ladungen und hydrophobe Radikale Hy trägt, wobei die Co-Polyaminosäure aus Glutamin- oder Asparagineinheiten besteht und die hydrophoben Radikale Hy die folgenden Formel I aufweisen: 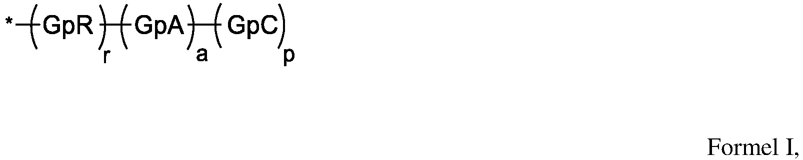 wobei
- GpR ein Radikal gemäß Formel II ist: 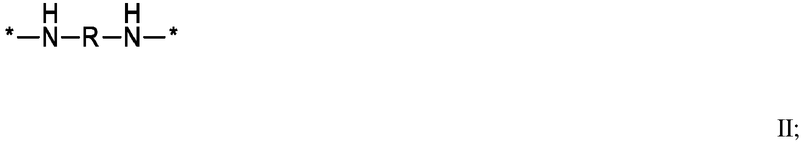
- GpA ein Radikal gemäß der Formel III oder III' ist: 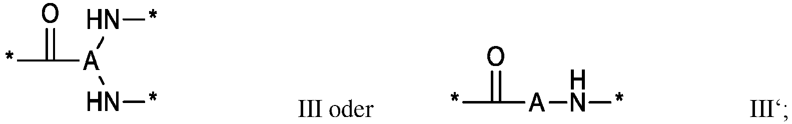
- GpC ein Radikal der Formel IV ist: 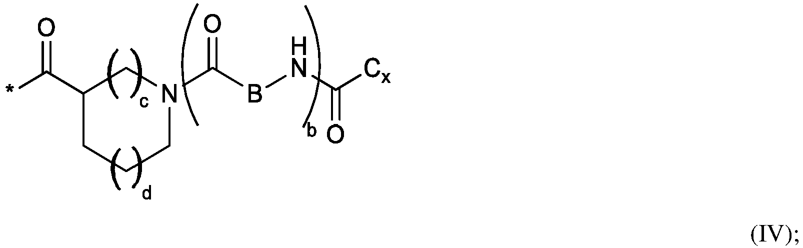
- die * die Bindungsstellen der verschiedenen durch Amidfunktionen gebundenen Gruppen anzeigt;
- a eine ganze Zahl von 0 oder 1 ist;
- b eine ganze Zahl von 0 oder 1 ist;
- p eine ganze Zahl von 1 oder 2 ist und
- wenn p gleich 1 ist, a gleich 0 oder 1 und GpA ein Radikal gemäß Formel III' ist, und
- wenn p gleich 2 ist, a gleich 1 und GpA ein Radikal gemäß Formel III ist;
- c eine ganze Zahl von 0 oder 1 ist und wenn c gleich 0 ist, d gleich 1 oder 2 ist;
- d eine ganze Zahl von 0, 1 oder 2 ist;
- r eine ganze Zahl von 0 oder 1 ist, und
- wenn r gleich 0 ist, das hydrophobe Radikal gemäß Formel I mit der Co-Polyaminosäure durch eine kovalente Bindung zwischen einem Carbonyl des hydrophoben Radikals und einem Stickstoffatom an N-terminaler Position der Co-Polyaminosäure verbunden ist, wodurch eine Amidfunktion gebildet ist als Ergebnis der Reaktion einer Aminfunktion an N-terminaler Position des Vorläufers der Co-Polyaminosäure und einer Säurefunktion, die von dem Vorläufer des hydrophoben Radikals getragen ist, und
- wenn r gleich 1 ist, das hydrophobe Radikal der Formel I an die Co-Polyaminosäure gebunden ist:
- mittels einer kovalenten Bindung zwischen einem Stickstoffatom des hydrophoben Radikals und einem Carbonyl der Co-Polyaminosäure, wodurch eine Amidfunktion gebildet ist als Ergebnis aus der Reaktion einer Aminfunktion des Vorläufers des hydrophoben Radikals und einer Säurefunktion, die von dem Vorläufer der Co-Polyaminosäure getragen ist, oder
- mittels einer kovalenten Bindung zwischen einem Carbonyl des hydrophoben Radikals und einem Stickstoffatom an N-terminaler Position der Co-Polyaminosäure, wodurch eine Amidfunktion gebildet ist als Ergebnis der Reaktion einer Säurefunktion des Vorläufers des hydrophoben Radikals und einer Aminfunktion an N-terminaler Position, die von dem Vorläufer der Co-Polyaminosäure getragen ist;
- R ein Radikal ist, das ausgewählt ist aus der Gruppe bestehend aus:
- einem linearen oder verzweigten divalenten Alkylradikal umfassend 2 bis 12 Kohlenstoffatome, wenn GpR ein Radikal gemäß Formel II ist;
- einem linearen oder verzweigten Alkylradikal umfassend 2 bis 11 Kohlenstoffatome, wenn GpR ein Radikal gemäß Formel II ist, wobei das Alkylradikal eine oder mehrere -CONH2 - Funktionen trägt, und
- einem nicht-substituierten Ether- oder Polyetherradikal umfassend 4 bis 14 Kohlenstoffatome und 1 bis 5 Sauerstoffatome;
- A ein lineares oder verzweigtes Alkylradikal ist umfassend 1 bis 6 Kohlenstoffatome ist;
- B ein lineares oder verzweigtes Alkylradikal ist, optional umfassend einen aromatischen Ring, umfassend 1 bis 9 Kohlenstoffatome;
- Cₓ ein lineares oder verzweigtes monovalentes Alkylradikal ist, in dem x die Anzahl von Kohlenstoffatomen angibt, und:
- wenn p gleich 1 ist, x zwischen 11 und 25 beträgt (11 ≤ x ≤ 25):
- wenn p gleich 2 ist, x zwischen 9 und 15 beträgt (9 ≤ x ≤ 15),
- das Verhältnis i aus der Anzahl hydrophober Radikale und der Anzahl an Glutamin-oder Asparagineinheiten zwischen 0 < i ≤ 0,5 beträgt;
- wenn mehrere hydrophobe Radikale von einer Co-Polyaminosäure getragen sind, sie identisch oder verschieden sind,
- der Polymerisationsgrad DP an Glutamin- oder Asparagineinheiten zwischen 10 und 250 beträgt;
- die freien Säuregruppen in Form eines Salzes eines Alkalikations vorliegen, das ausgewählt ist aus der Gruppe bestehend aus Na⁺ und K⁺.

2. Zusammensetzung nach Anspruch 1, **dadurch gekennzeichnet, dass** die hydrophoben Radikale ausgewählt sind aus den hydrophoben Radikalen gemäß Formel I, wobei p = 1 ist, dargestellt durch die folgende Formel V: 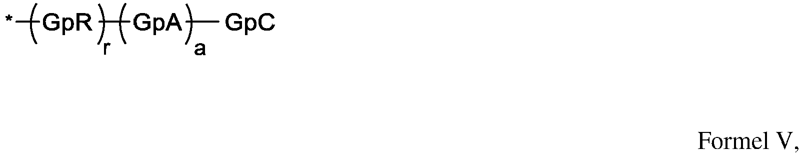 wobei GpR, GpA, GpC, r und a wie in Anspruch 1 definiert sind.

3. Zusammensetzung nach Anspruch 1, **dadurch gekennzeichnet, dass** die hydrophoben Radikale ausgewählt sind aus den hydrophoben Radikalen gemäß Formel I, wobei a = 1 und p = 2 ist, dargestellt durch die folgende Formel VI: 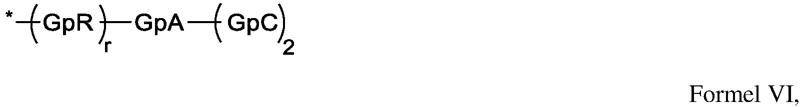 wobei GpR, GpA, GpC, r und a wie in Anspruch 1 definiert sind.

4. Zusammensetzung nach einem der vorangehenden Ansprüche, **dadurch gekennzeichnet, dass** die Carboxylatladungen und hydrophobe Radikale tragende Co-Polyaminosäure ausgewählt ist aus den Co-Polyaminosäuren gemäß der folgenden Formel VIIa: 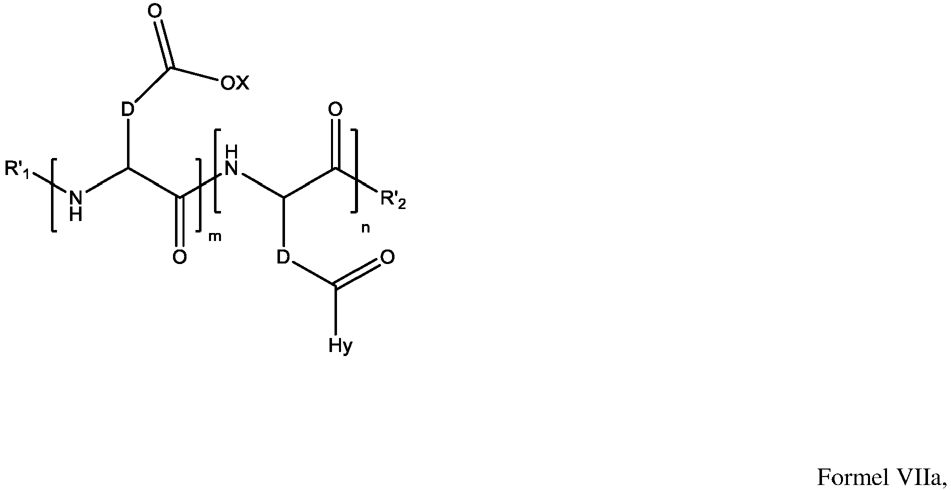 wobei
- D, unabhängig, entweder eine Gruppe -CH2- (Asparagineinheit) oder eine Gruppe - CH₂-CH₂- (Glutamineinheit) darstellt,
- Hy ein hydrophobes Radikal ist, das ausgewählt ist aus den hydrophoben Radikalen gemäß Formeln I, V oder VI, wobei r = 1 und GpR ein Radikal gemäß Formel II ist,
- X H oder eine kationische Einheit darstellt, die ausgewählt ist aus der Gruppe umfassend Metallkationen;
- n + m den Polymerisationsgrad DP der Co-Polyaminosäure darstellt, d.h. die mittlere Anzahl monomerer Einheiten pro Co-Polyaminosäurekette und 5 ≤ n + m ≤ 250; R'1 ein Radikal ist, das ausgewählt ist aus der Gruppe bestehend aus H, einer linearen Acylgruppe aus C2 bis C10, einer verzweigten Acylgruppe aus C4 bis C10, einem Benzyl, einer terminalen "Aminosäure"-Einheit und Pyroglutamat,
- R'2 ein Radikal -NR'R" ist, wobei R' und R" identisch oder verschieden sind, ausgewählt aus der Gruppe bestehend aus H, linearen oder verzweigten oder zyklischen Alkylen aus C2 bis C10, wobei das Benzyl und die R'- und R"-Alkyle zusammen einen oder mehrere gesättigte, ungesättigte und/oder aromatische Kohlenstoffzyklen bilden und/oder Heteroatome tragen können, die ausgewählt sind aus der Gruppe bestehend aus O, N und S.

5. Zusammensetzung nach Anspruch 4, **dadurch gekennzeichnet, dass** die Carboxylatladungen und hydrophobe Radikale tragende Co-Polyaminosäure ausgewählt ist aus den Co-Polyaminosäuren gemäß Formeln VII, wobei R₁ = R'₁ und R₂ = R'₂ der folgenden Formel VIIa: 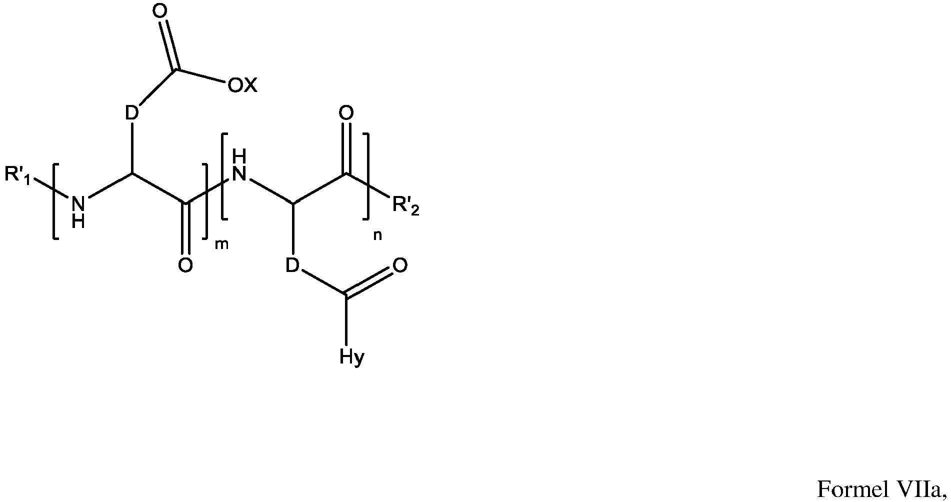 wobei
- m, n X, D und Hy wie in Anspruch 4 definiert sind,
- R'₁ ein Radikal ist, das ausgewählt ist aus der Gruppe bestehend aus H, einer linearen Acylgrupp aus C2 bis C10, einer verzweigten Acylgruppe aus C4 bis C10, einem Benzyl, einer terminalen "Aminosäure" und Pyroglutamat,
- R'₂ ein Radikal -NR'R" ist, wobei R' und R" identisch oder verschieden sind, ausgewählt aus der Gruppe bestehend aus H, linearen oder verzweigten oder zyklischen Alkylen aus C2 bis C10, wobei das Benzyl und die R' und R"-Alkyle zusammen einen oder mehrere gesättigte, ungesättigte und/oder aromatische Kohlenstoffzyklen bilden und/oder Heteroatome tragen können, die ausgewählt sind aus der Gruppe bestehend aus O, N und D.

6. Zusammensetzung nach einem der Ansprüche 4 und 5, **dadurch gekennzeichnet, dass** die Carboxylatladungen und hydrophobe Radikale tragende Co-Polyaminosäure ausgewählt ist aus den Co-Polyaminosäuren der Formeln VII oder VIIa, wobei die wenigstens eine Co-Polyaminosäure ausgewählt ist aus den Co-Polyaminosäuren, bei denen die Gruppe D eine - CH2-CH2- Gruppe (Glutamineinheit) ist.

7. Zusammensetzung nach einem der vorangehenden Ansprüche, **dadurch gekennzeichnet, dass** die Konzentration an Carboxylatladungen und hydrophobe Radikale tragender Co-Polyaminosäure höchstens 40 mg/ml beträgt.

8. Zusammensetzung nach einem der vorangehenden Ansprüche, **dadurch gekennzeichnet, dass** die Konzentration an humanem Glucagon zwischen 0,25 und 5 mg/ml beträgt.

9. Zusammensetzung nach einem der vorangehenden Ansprüche, **dadurch gekennzeichnet, dass** das Molverhältnis [hydrophobes Radikal]/[menschliches Glucagon] kleiner als 15 ist.

10. Zusammensetzung nach einem der vorangehenden Ansprüche, **dadurch gekennzeichnet, dass** sie außerdem eine polyanionische Verbindung umfasst.

11. Zusammensetzung nach einem der vorangehenden Ansprüche, **dadurch gekennzeichnet, dass** sie außerdem ein Zinksalz umfasst.

12. Zusammensetzung nach einem der vorangehenden Ansprüche, **dadurch gekennzeichnet, dass** sie ein gastrointestinales Hormon umfasst.

13. Zusammensetzung nach Anspruch 12, **dadurch gekennzeichnet, dass** das gastrointestinale Hormon ausgewählt ist aus der Gruppe bestehend aus Exenatid, Liraglutid, Lixisenatid, Albiglutide und Dulaglutid und ihren pharmazeutisch akzeptablen Salzen.

14. Zusammensetzung nach einem der Ansprüche 12 und 13, **dadurch gekennzeichnet, dass** die Konzentration an gastrointestinalem Hormon in einem Bereich von 0,01 bis 10 mg/ml liegt.

15. Co-Polyaminosäure tragend Carboxylatladungen und hydrophobe Radikale Hy gemäß der folgenden Formel I: 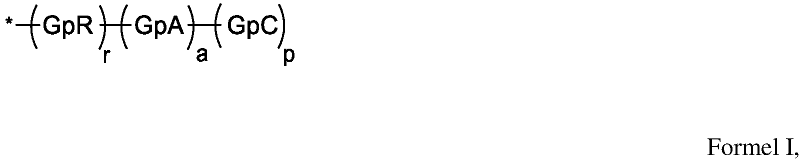 wobei
- GpR ein Radikal gemäß Formel II ist: 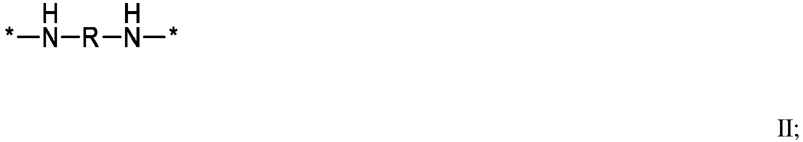
- GpA ein Radikal gemäß der Formel III oder III' ist: 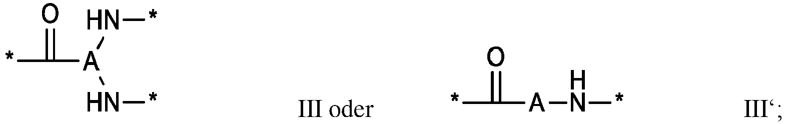
- GpC ein Radikal der Formel IV ist: 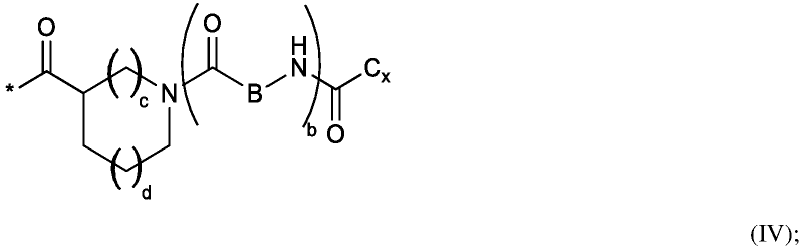
- die * die Bindungsstellen der verschiedenen durch Amidfunktionen gebundenen Gruppen anzeigt;
- a eine ganze Zahl von 0 oder 1 ist;
- b eine ganze Zahl von 0 oder 1 ist;
- p eine ganze Zahl von 1 oder 2 ist und
- wenn p gleich 1 ist, a gleich 0 oder 1 und GpA ein Radikal gemäß Formel III' ist, und
- wenn p gleich 2 ist, a gleich 1 und GpA ein Radikal gemäß Formel III ist;
- c eine ganze Zahl von 0 oder 1 ist und wenn c gleich 0 ist, d gleich 1 oder 2 ist;
- d eine ganze Zahl von 0, 1 oder 2 ist;
- r eine ganze Zahl von 0 oder 1 ist, und
- wenn r gleich 0 ist, das hydrophobe Radikal gemäß Formel I mit der Co-Polyaminosäure durch eine kovalente Bindung zwischen einem Carbonyl des hydrophoben Radikals und einem Stickstoffatom an N-terminaler Position der Co-Polyaminosäure verbunden ist, wodurch eine Amidfunktion gebildet ist als Ergebnis der Reaktion einer Aminfunktion an N-terminaler Position des Vorläufers der Co-Polyaminosäure und einer Säurefunktion, die von dem Vorläufer des hydrophoben Radikals getragen ist, und
- wenn r gleich 1 ist, das hydrophobe Radikal der Formel I an die Co-Polyaminosäure gebunden ist:
- mittels einer kovalenten Bindung zwischen einem Stickstoffatom des hydrophoben Radikals und einem Carbonyl der Co-Polyaminosäure, wodurch eine Amidfunktion gebildet ist als Ergebnis der Reaktion einer Aminfunktion des Vorläufers des hydrophoben Radikals und einer Säurefunktion, die von dem Vorläufer der Co-Polyaminosäure getragen ist, oder
- mittels einer kovalenten Bindung zwischen einem Carbonyl des hydrophoben Radikals und einem Stickstoffatom an N-terminaler Position der Co-Polyaminosäure, wodurch eine Amidfunktion gebildet ist als Ergebnis der Reaktion einer Säurefunktion des Vorläufers des hydrophoben Radikals und einer Aminfunktion an N-terminaler Position, die von dem Vorläufer der Co-Polyaminosäure getragen ist;
- R ein Radikal ist, das ausgewählt ist aus der Gruppe bestehend aus:
- einem linearen oder verzweigten divalenten Alkylradikal umfassend 2 bis 12 Kohlenstoffatome, wenn GpR ein Radikal gemäß Formel II ist;
- einem linearen oder verzweigten Alkylradikal umfassend 2 bis 11 Kohlenstoffatome, wenn GpR ein Radikal gemäß Formel II ist, wobei das Alkylradikal eine oder mehrere -CONH2 - Funktionen trägt, und
- einem nicht-substituierten Ether- oder Polyetherradikal umfassend 4 bis 14 Kohlenstoffatome und 1 bis 5 Sauerstoffatome;
- A ein lineares oder verzweigtes Alkylradikal ist umfassend 1 bis 6 Kohlenstoffatome ist;
- B ein lineares oder verzweigtes Alkylradikal ist, optional umfassend einen aromatischen Ring, umfassend 1 bis 9 Kohlenstoffatome;
- Cₓ ein lineares oder verzweigtes monovalentes Alkylradikal ist, in dem x die Anzahl von Kohlenstoffatomen angibt, und:
- wenn p gleich 1 ist, x zwischen 11 und 25 beträgt (11 ≤ x ≤ 25):
- wenn p gleich 2 ist, x zwischen 9 und 15 beträgt (9 ≤ x ≤ 15),
- das Verhältnis i aus der Anzahl hydrophober Radikale und der Anzahl an Glutamin-oder Asparagineinheiten zwischen 0 < i ≤ 0,5 beträgt;
- wenn mehrere hydrophobe Radikale von einer Co-Polyaminosäure getragen sind, sie identisch oder verschieden sind,
- der Polymerisationsgrad DP an Glutamin- oder Asparagineinheiten zwischen 10 und 250 beträgt;
- die freien Säuregruppen in Form eines Salzes eines Alkalikations vorliegen, das ausgewählt ist aus der Gruppe bestehend aus Na⁺ und K⁺.

## Claims

1. A composition in the form of an injectable aqueous solution, the pH of which is from 6.0 to 8.0, comprising at least:
a) human glucagon;
b) a co-polyamino acid bearing carboxylate charges and hydrophobic radicals Hy, said co-polyamino acid consisting of glutamic or aspartic units, and said hydrophobic radicals Hy being radicals of the following formula I: 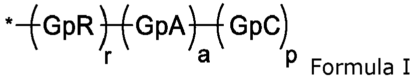 in which
- GpR is a radical of formula II: 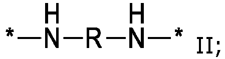
- GpA is a radical of formula III or III' : 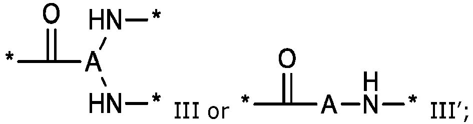
- GpC is a radical of formula IV : 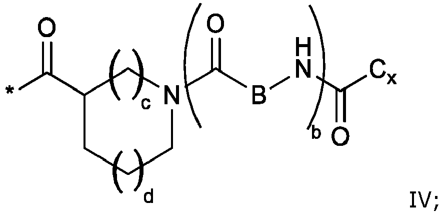
- the * indicate the sites of attachment of the different groups bound by amide functions;
- a is a whole number equal to 0 or 1;
- b is a whole number equal to 0 or 1;
- p is a whole number equal to 1 or 2, and
∘ if p is equal to 1, then a is equal to 0 or 1 and GpA is a radical of formula III', and,
∘ if p is equal to 2, then a is equal to 1 and GpA is a radical of formula III;
- c is a whole number equal to 0 or 1, and, if c is equal to 0, then d is equal to 1 or 2;
- d is a whole number equal to 0, to 1 or 2;
- r is a whole number equal to 0 or 1, and
∘ if r is equal to 0, then the hydrophobic radical of formula I is bound to the co-polyamino acid via a covalent bond between a carbonyl of the hydrophobic radical and a nitrogen atom in the N-terminal position of the co-polyamino acid, thus forming an amide function originating from the reaction of an amine function in N-terminal position of the precursor of the co-polyamino acid and an acid function borne by the precursor of the hydrophobic radical, and
∘ if r is equal to 1, then the hydrophobic radical of formula I is bound to the co-polyamino acid:
▪ via a covalent bond between a nitrogen atom of the hydrophobic radical and a carbonyl of the co-polyamino acid therefore forming an amide function originating from the reaction between an amine function of the precursor of the hydrophobic radical and an acid function borne by the precursor of the co-polyamino acid, or
▪ via a covalent bond between a carbonyl of the hydrophobic radical and a nitrogen atom in N-terminal position of the co-polyamino acid therefore forming an amide function originating from the reaction of an acid function of the precursor of the hydrophobic radical and an amine function in N terminal position borne by the precursor of the co-polyamino acid;
- R is a radical selected from the group consisting of:
∘ a linear or branched divalent alkyl radical comprising, if GpR is a radical of formula II, from 2 to 12 carbon atoms;
∘ a linear or branched divalent alkyl radical comprising, if GpR is a radical of formula II, from 2 to 11 carbon atoms, said alkyl radical bearing one or more -CONH₂ functions, and
∘ an unsubstituted ether or polyether radical comprising from 4 to 14 carbon atoms and from 1 to 5 oxygen atoms;
- A is a linear or branched alkyl radical comprising from 1 to 6 carbon atoms;
- B is a linear or branched alkyl radical, optionally comprising an aromatic ring, comprising from 1 to 9 carbon atoms;
- Cₓ is a linear or branched monovalent alkyl radical, in which x indicates the number of carbon atoms, and:
∘ if p is equal to 1, x is from 11 to 25 (11 ≤ x ≤ 25);
∘ if p is equal to 2, x is from 9 to 15 (9 ≤ x ≤ 15),
- the ratio i between the number of hydrophobic radicals and the number of glutamic or aspartic units being between 0 < i ≤ 0.5;
- when several hydrophobic radicals are borne by a co-polyamino acid, then they are identical or different,
- the degree of polymerization DP in glutamic or aspartic units is from 10 to 250;
- the free acid functions being in the form of a salt of an alkaline cation selected from the group consisting of Na⁺ and K⁺.

2. The composition according to Claim 1, **characterized in that** said hydrophobic radicals are selected from the hydrophobic radicals of formula I in which p = 1, represented by the following formula V: 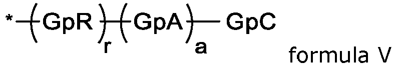 GpR, GpA, GpC, r and a as defined in claim 1.

3. The composition according to Claim 1, **characterized in that** said hydrophobic radicals are selected from the hydrophobic radicals of formula I in which a = 1 and p = 2, represented by the following formula VI: 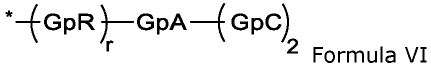 In which
GpR, GpA, GpC, r and a as defined in claim 1.

4. The composition according to any one of the preceding claims, **characterized in that** the co-polyamino acid bearing carboxylate charges and hydrophobic radicals is selected from the co-polyamino acids of the following formula VIIa: 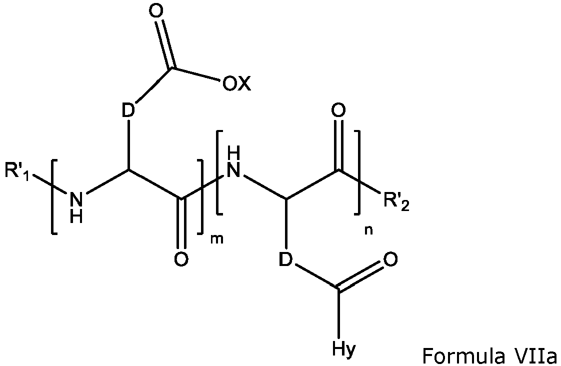 in which,
• D represents, independently, either a -CH₂- group (aspartic unit) or a - CH2-CH2- group (glutamic unit),
• Hy is a hydrophobic radical selected from the hydrophobic radicals of formula I, V or VI, in which r = 1 and GpR is a radical of Formula II,
• X represents an H or a cationic entity selected from the group comprising the metal cations;
• n + m represents the degree of polymerization DP of the co-polyamino acid, that is to say the average number of monomer units per chain and 5 ≤ n + m ≤ 250;R'1 is a radical selected from the group consisting of H, a C2 to C10 linear acyl group, a C4 to C10 branched acyl group, benzyl, a terminal "amino acid" unit and a pyroglutamate,
• R'2 is a -NR'R", radical, R' and R", which are identical or different, being selected from the group consisting of H, the C2 to C10 linear or branched or cyclic alkyls, benzyl, and said alkyl R' and R" together optionally forming one or more saturated, unsaturated and/or aromatic carbon rings and/or optionally comprising heteroatoms, selected from the group consisting of O, N and S.

5. The composition according to Claim 4, **characterized in that** co-polyamino acid bearing carboxylate charges and hydrophobic charges is selected from the co-polyamino acids of formulas VII, in which R₁ = R'₁ and R₂ = R'₂, of the following formula VIIa: 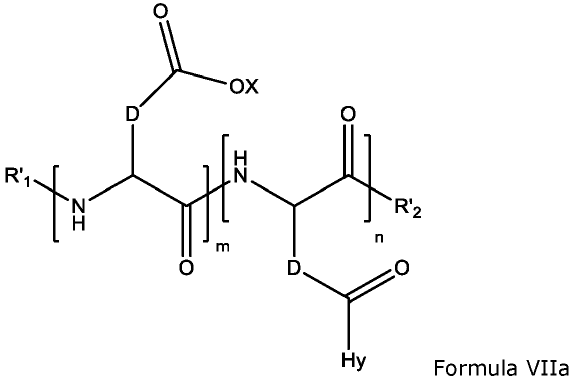 in which,
- m, n, X, D and Hy as defined in claim 4,
- R'₁ is a radical selected from the group consisting of H, a C2 to C10 linear acyl group, a C4 to C10 branched acyl group, benzyl, a terminal "amino acid" unit and a pyroglutamate,
- R'₂ is a -NR'R" radical, R' and R", which are identical or different, being selected from the group consisting of H, the C2 to C10 linear or branched or cyclic alkyls, benzyl, and said alkyl R' and R" together optionally forming one or more saturated, unsaturated and/or aromatic rings and/or optionally comprising heteroatoms, selected from the group consisting of O, N and S.

6. The composition according to any one of claims 4 and 5, **characterized in that** the co-polyamino acid bearing carboxylate charges and hydrophobic radicals is selected from the co-polyamino acids of formulas VII or VIIa in which the at least one co-polyamino acid is selected from the co-polyamino acids in which group D is a group - CH2-CH2- (glutamic unit).

7. The composition according to any one of the preceding claims, **characterized in that** the concentration of co-polyamino acid bearing carboxylate charges and hydrophobic radicals is at most 40 mg/mL.

8. The composition according to any one of the preceding claims, **characterized in that** the concentration of human glucagon is from 0,25 and 5 mg/mL.

9. The composition according to any one of the preceding claims, **characterized in that** the molar ratio [hydrophobic radical]/[humain glucagon] is less than 15.

10. The composition according to any one of the preceding claims, **characterized in that** it comprises, in addition, a polyanionic compound.

11. The composition according to any one of the preceding claims, **characterized in that** it comprises, in addition, a zinc salt.

12. The composition according to any one of the preceding claims, **characterized in that** it comprises, in addition, a gastrointestinal hormone.

13. The composition according to claim 12, **characterized in that** the gastrointestinal hormone is selected from the group consisting of exenatide, liraglutide, lixisenatide, albiglutide and dulaglutide.

14. The composition according to any one of Claims 12 and 13, **characterized in that** the concentration of gastrointestinal hormone is within an interval from 0,01 to 10 mg/mL.

15. A co-polyamino acid bearing carboxylate charges and hydrophobic radicals Hy of the following formula I: 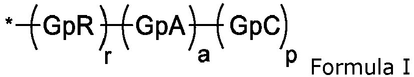 in which
- GpR is a radical of formula II: 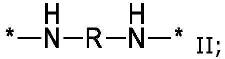
- GpA is a radical of formula III or III' : 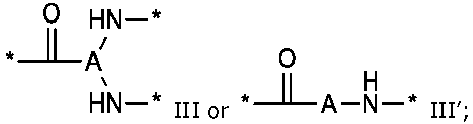
- GpC is a radical of formula IV : 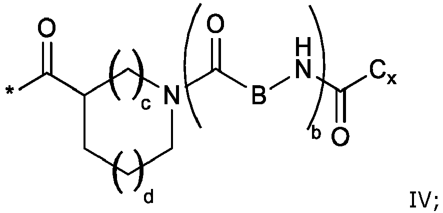
- the * indicate the sites of attachment of the different groups bound by amide functions;
- a is a whole number equal to 0 or 1;
- b is a whole number equal to 0 or 1;
- p is a whole number equal to 1 or 2, and
∘ if p is equal to 1, then a is equal to 0 or 1 and GpA is a radical of formula III', and,
∘ if p is equal to 2, then a is equal to 1 and GpA is a radical of formula III;
- c is a whole number equal to 0 or 1, and, if c is equal to 0, then d is equal to 1 or 2;
- d is a whole number equal to 0, to 1 or 2;
- r is a whole number equal to 0 or 1, and
∘ if r is equal to 0, then the hydrophobic radical of formula I is bound to the co-polyamino acid via a covalent bond between a carbonyl of the hydrophobic radical and a nitrogen atom in the N-terminal position of the co-polyamino acid, thus forming an amide function originating from the reaction of an amine function in N-terminal position of the precursor of the co-polyamino acid and an acid function borne by the precursor of the hydrophobic radical, and
∘ if r is equal to 1, then the hydrophobic radical of formula I is bound to the co-polyamino acid:
▪ via a covalent bond between a nitrogen atom of the hydrophobic radical and a carbonyl of the co-polyamino acid therefore forming an amide function originating from the reaction between an amine function of the precursor of the hydrophobic radical and an acid function borne by the precursor of the co-polyamino acid, or
▪ via a covalent bond between a carbonyl of the hydrophobic radical and a nitrogen atom in N-terminal position of the co-polyamino acid therefore forming an amide function originating from the reaction of an acid function of the precursor of the hydrophobic radical and an amine function in N terminal position borne by the precursor of the co-polyamino acid;
- R is a radical selected from the group consisting of:
∘ a linear or branched divalent alkyl radical comprising, if GpR is a radical of formula II, from 2 to 12 carbon atoms;
∘ a linear or branched divalent alkyl radical comprising, if GpR is a radical of formula II, from 2 to 11 carbon atoms, said alkyl radical bearing one or more -CONH₂ functions, and
∘ an unsubstituted ether or polyether radical comprising from 4 to 14 carbon atoms and from 1 to 5 oxygen atoms;
- A is a linear or branched alkyl radical comprising from 1 to 6 carbon atoms;
- B is a linear or branched alkyl radical, optionally comprising an aromatic ring, comprising from 1 to 9 carbon atoms;
- Cₓ is a linear or branched monovalent alkyl radical, in which x indicates the number of carbon atoms, and:
∘ if p is equal to 1, x is from 11 to 25 (11 ≤ x ≤ 25);
∘ if p is equal to 2, x is from 9 to 15 (9 ≤ x ≤ 15),
- the ratio i between the number of hydrophobic radicals and the number of glutamic or aspartic units being between 0 < i ≤ 0.5;
- when several hydrophobic radicals are borne by a co-polyamino acid, then they are identical or different,
- the degree of polymerization DP in glutamic or aspartic units is from 10 to 250;
- the free acid functions being in the form of a salt of an alkaline cation selected from the group consisting of Na⁺ and K⁺.
